# EUROPEAN PATENT APPLICATION

(11) **EP 4 813 403 A1**
(43) Date of publication of application: **30.09.2026**
(21) Application number: 24893536.3
(22) Date of filing: 21.11.2024
(51) Int. Cl.: A61K 38/16, C07K 14/00, A61P 3/10, A61P 3/04, A61P 9/10, A61P 9/00

(54) **USE OF GLP-1R/GIPR DOUBLE-TARGET AGONIST IN PREPARING ANIMAL DRUG**

(30) Priority: 22.11.2023 CN 202311565075
(71) Applicant: Hangzhou Zhongmeihuadong Pharmaceutical Co., Ltd., GongShu District HangZhou, Zhejiang 310011 (CN)
(72) Inventor: ZHANG, Nan, Hangzhou, Zhejiang 310011 (CN); JIANG, Chunhua, Hangzhou, Zhejiang 310011 (CN); LIU, Dongzhou, Hangzhou, Zhejiang 310011 (CN); TENG, Li, Hangzhou, Zhejiang 310011 (CN)
(74) Representative: Nederlandsch Octrooibureau
(86) International application number: PCT/CN2024/133548
(87) International publication number: WO 2025/108381

(57) **Abstract**

Use of a GLP-1R/GIPR double-target agonist compound in preparing a drug for preventing or treating diseases in non-primate and non-rodent animals.

## Description

### TECHNICAL FIELD

The present invention relates to the field of medicine for treating an animal, and in particular relates to use of a glucagon-like peptide-1 receptor/glucose-dependent insulinotropic polypeptide receptor (GLP-1R/GIPR) dual agonist in the manufacture of a medicament for preventing or treating a disease in a non-primate and non-rodent animal.

### BACKGROUND

With the development of social economy, more and more people start to keep pets. Keeping pets becomes an indispensable part of their lives. The number of pets, in particular dogs and cats, in China is growing rapidly, which has led to a rapid increase in the demand for pet supplies and animal supplies.

There are very few pet-specific medicines produced in China. Until 2017, among the veterinary medicines approved by the Ministry of Agriculture of the People's Republic of China, there were only 187 pet-specific medicines, consisting of 180 pet-specific chemical medicines and 7 pet-specific biological medicines. As stipulated in the Regulations on the Administration of Veterinary Drugs, the medicines for human are prohibited to be used in pets. However, due to the scarcity of pet-specific medicines, many pets in China are still treated with medicines for human. Moreover, some medicines for human are not effective in pets. Therefore, many problems in connection with veterinary clinics continuously arise, and the most notable issue is associated with the misuse of human medicines on animals in the clinics. The mixed use of drugs for humans and animals may lead to serious harm. Since humans and animals have different physiological mechanisms and metabolic characteristics and different species of animals are significantly different in drug tolerance, some human medicines may be ineffective or not fully effective when used in animals, and some may cause unexpected side effects, or even lead to death of pets. For example, the use of human antibiotics in animals can lead to the emergence of drug-resistant bacteria, thereby posing immeasurable risks and losses to public health.

Obesity is a common problem in pets. Obesity leads to metabolic disorders in pets, and increases the risk of diabetes mellitus, cardiovascular diseases, arthropathy, dermatosis and the like, which severely affects the pet's health. Currently, pet weight loss prescription diet is one of the options for managing overweight in pets. In terms of drug treatment, Slentrol (Dirlotapide) and Yarvitan (Mitratapide) have been approved. Yarvitan is the first weight loss medicine specifically developed for pet dogs, and can achieve an effect of an average weight loss of about 6.4 kilograms per month. Both Slentrol and Yarvitan are selective microsomal triglyceride transfer protein (MTP) inhibitors, both of which act on intestinal epithelial cells to block lipid absorption and have a local effect on satiety.

Diabetes mellitus is also a endocrine disorder commonly found in pets such as cats and dogs. At present, in veterinary classifications, pet diabetes mellitus is also classified into four types by referring to the mechanisms of human diabetes mellitus and pancreatic β-cell failure: Type I, Type II, gestational, and other specific types. Type I diabetes mellitus is characterized by immune-mediated β-cell destruction, which leads to absolute insulin deficiency. This is relatively rare in pets. Type II diabetes mellitus remains the most common type of diabetes mellitus in pets. Due to insufficient insulin secretion, the utilization of glucose in the body is reduced, thereby clinically exhibiting a positive result in a urine glucose test. In one aspect, the increase in blood glucose concentration stimulates the hypothalamic feeding center, leading to increased appetite. In another aspect, due to the increase in blood glucose concentration, plasma osmotic pressure is raised, which enhances vascular permeability and leads to clinical osmotic diuresis. Meanwhile, due to the decreased ability to metabolize glucose, the body cannot utilize enough glucose as an energy source, which increases the relative energy consumption. Glucose is excreted in the urine, which in turn carries away more water, and ultimately leads to an increase in the body's desire to drink, emaciatation and weight loss. The clinical symptoms are primarily characterized by polyuria (PU), polydipsia (PD), polyphagia (PP), and weight loss. In severe cases, a fruity odor may appear in the breath. Dogs may sometimes develop cataracts, hepatomegaly, and liver fatty deposition. The therapeutic objective for pet diabetes mellitus is to minimize the symptoms while minimizing the risk of hypoglycemia. The current treatment for pet diabetes mellitus generally uses insulin products, such as porcine insulin zinc suspension (Vetsulin^{®}, Intervet, Netherlands; Vetsulin, Merck), and protamine zinc recombinant human insulin (ProZinc^{®}, Boehringer Ingelheim), which can be rationally administrated based on the onset time and duration of the insulin.

Incretin hormones can affect pancreatic β-cells and play an important role in glucose homeostasis. The incretin hormones GIP and GLP-1, upon a nutritient-induced intestinal secretion, are important in increasing insulin secretion and regulating glucagon secretion. GIP is secreted predominantly by K-cells at the duodenum and the upper jejunum, and GLP-1 is secreted by intestinal L-cells. GLP-1R is expressed in multiple brain nuclei involved in appetite regulation, and can suppress appetite and energy intake in both normal-weight and obese individuals. In obese animal models (including miniature pigs and rodents), GLP-1 analogs can reduce feeding frequency and meal size. GLP-1 can also enhance satiety and reduce appetite by slowing gastric emptying. GIP and GLP-1 in active forms can be degraded into their inactive forms by dipeptidyl peptidase-4 (DPP-4, also known as CD26) and neutral endopeptidase 24.11 (NEP-24.11). DPP-4 and NEP are widely found in tissues, and DPP-4 is also present in blood in a soluble form. Therefore, upon intravenous administration, the GLP-1 and GIP in active forms are rapidly degraded to their inactive forms, and rapidly cleared by the kidneys, thus GLP-1 (1-2 min) and GIP (5 min) in active forms have shorter half-lives. Although the half-lives of GLP-1 and GIP in cats and dogs are unknown, the activity of DPP-4 in healthy cats has been indirectly demonstrated: after healthy cats are stimulated with an oral administration of a DPP-4 inhibitor in combination with intravenous injection of glucose, the increase in insulin concentration is associated with the decrease in glucagon concentration. In another study, the administration of a DPP-4 inhibitor was associated with the increase in GLP-1 concentration.

The distribution of L cells and K cells in the intestine varies from species to species. Such distribution to some extent determines the importance of different stimulants for the secretion of GLP-1 and GIP. Quantitative immunohistochemical studies on the intestines of dogs, rats, pigs, and humans have shown that the density gradient of L cells is opposite to that of K cells, that is, L cells are scarce (if any) in the distal duodenum, and their density gradually increases along the jejunum and reaches a maximum in the ileum and colon. In contrast, K cells have the highest density in the duodenum, and their density gradually decreases along the jejunum. Dogs, rats, pigs, and humans have no K cells in the ileum and colon. In addition, the stimulation degree of different nutrients to GIP and GLP-1 varies from species to species (New approaches to feline diabetes mellitus Glucagon-like peptide-1 analogs). Fats and carbohydrates are effective stimulants for GIP secretion in humans, dogs, pigs, and rodents. In humans and dogs, fats are more potent stimulators for GIP secretion as compared to carbohydrates, while the opposite is true for rodents and pigs. In cats, fats can stimulate GIP secretion more effectively than amino acids, but the orally-administrated glucose has no effect on GIP secretion. Postprandial GLP-1 secretion is generally in a biphasic pattern, and stimulated by ingested lipids, carbohydrates, and proteins. In a study on healthy cats, the overall effects of lipids, carbohydrates, and amino acids on total GLP-1 secretion were similar, although the temporal profile are different.

At present, there is no GLP-1R agonist medicine for obesity and/or hyperglycemia in animals, let along GLP-1R/GIPR dual agonist pet medicines. Therefore, it is necessary to develop GLP-1R/GIPR dual agonist animal medicines so as to provide multifaceted options for safe, effective, and long-lasting medicines for animals, especially for overweight, obesity, and/or diabetes mellitus in pets.

### SUMMARY OF INVENTION

An object of the present invention is to provide a use of some long-acting GLP-1R/GIPR dual agonist compounds in the manufacture of veterinary medicines, particularly in the manufacture of a medicament for preventing or treating a disease in a non-primate and non-rodent animal. The compounds according to the present invention have excellent GIPR/GLP-1R dual agonist activity in various species of animals, including but not limited to, dogs, cats, rabbits, pigs, alpacas, horses, sheep, and bovines. As compared with known GIPR/GLP-1R dual agonist compounds, the compounds according to the present invention have a longer half-life and/or a longer duration of action. The compounds according to the present invention have a stronger hypoglycemic effect and more significant weight loss effect. The compounds according to the present invention have less side effects, including but not limited to gastrointestinal reactions and heart safety risks and the like. The compounds according to the present invention could adopt shorter titration strategies. The compounds according to the present invention have a wider safety window. In addition, the compounds according to the present invention have a protective effect on the liver.

As a part of the present invention, the applicant has found that the selections in terms of the length, composition and bonding position of the fatty acid chains, and the linker between the peptide and the fatty acid chain and the like, have an unexpected effect on prolonging the half-life of the polypeptide.

An embodiment of the present invention provides a use of a compound of Formula (AI) or a pharmaceutically acceptable salt thereof in the manufacture of a medicament for preventing or treating a disease in a non-primate and non-rodent animal:

Y-X₁-E-G-T-X₂-T-S-D-Y-A11-A12-A13-L-D-K-A17-A-Q-A20-E-F-V-K-W-L-L-K-A29-G-P-S- S-G-A-P-P-P-S-K Formula (AI);

wherein X₁ is Aib; X₂ is αMePhe;
A11 is Aib or Ala;
A12 is Ala, Ile, Lys, Phe or Pya(4);
A13 is Aib, Cha, Leu, αMePhe or αMeTyr;
A17 is Gln or Ile;
A20 is Ala or Ser;
A29 is Gln or Gly;
1, 2, 3 or 4 Ks, which are selected from the group consisting of K at position 16, K at position 24, K at position 28 and K at position 40, are chemically modified through conjugation to the ε-aminos of the K side-chains with ([2-(2-amino-ethoxy)-ethoxy]-acetyl)ₐ-(γ-Glu)_{b}-CO-(CH₂)_{c}-Z, wherein each a is independently an integer from 0 to 5, each b is independently an integer from 0 to 5, and each c is independently an integer from 10 to 24, wherein Z is independently selected from the group consisting of -CH₃, carboxylic acids or carboxylic acid bioisosteres, phosphonates or sulfonates.

In another embodiment, the present invention provides a use of a compound of Formula (AI) or a pharmaceutically acceptable salt thereof in the manufacture of a medicament for preventing or treating a disease in a non-primate and non-rodent animal, wherein,
X₁ is Aib; X₂ is αMePhe;
A11 is Aib or Ala;
A12 is Ala, Ile, Lys, Phe or Pya(4);
A13 is Aib, Cha, Leu, αMePhe or αMeTyr;
A17 is Gln or Ile;
A20 is Ala or Ser;
A29 is Gln or Gly,
1, 2, 3 or 4 Ks, which are selected from the group consisting of K at position 16, K at position 24, K at position 28 and K at position 40, are chemically modified through conjugation to the ε-aminos of the K side-chains with ([2-(2-amino-ethoxy)-ethoxy]-acetyl)ₐ-(γ-Glu)_{b}-CO-(CH₂)_{c}-Z, wherein a is independently selected from an integer of 1, 2, 3, 4 or 5, b is independently selected from an integer of 1, 2, 3, 4 or 5, and c is independently selected from an integer of 12 to 22; wherein Z is independently selected from the group consisting of -CH₃, carboxylic acids or carboxylic acid bioisosteres, phosphonates or sulfonates; in addition, a compound is provided wherein c is independently selected from 14, 16, 18 or 20.

In some embodiments, in the peptide of the above Formula (AI) or a pharmaceutically acceptable salt thereof, A11 is Aib; and/or
in the peptide of the above Formula (AI) or a pharmaceutically acceptable salt thereof, A12 is Ile; and/or
in the peptide of the above Formula (AI) or a pharmaceutically acceptable salt thereof, A13 is Aib; and/or
in the peptide of the above Formula (AI) or a pharmaceutically acceptable salt thereof, A17 is Gln; and/or
in the peptide of the above Formula (AI) or a pharmaceutically acceptable salt thereof, A20 is Ala; and/or
in the peptide of the above Formula (AI) or a pharmaceutically acceptable salt thereof, A29 is Gly.

An embodiment of the present invention provides a use of a compound of Formula (I) or a pharmaceutically acceptable salt thereof in the manufacture of a medicament for preventing or treating a disease in a non-primate and non-rodent animal:

Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K Formula (I);

wherein X₁ is Aib; X₂ is αMePhe; X₃ is Aib; X₄ is Aib; 1, 2, 3 or 4 Ks, which are selected from the group consisting of K at position 16, K at position 24, K at position 28 and K at position 40, are chemically modified through conjugation to the ε-aminos of the K side-chains with ([2-(2-amino-ethoxy)-ethoxy]-acetyl)ₐ-(γ-Glu)_{b}-CO-(CH₂)_{c}-Z, wherein each a is independently an integer from 0 to 5, each b is independently an integer from 0 to 5, and each c is independently an integer from 10 to 24, wherein Z is independently selected from the group consisting of -CH₃, carboxylic acids or carboxylic acid bioisosteres, phosphonates or sulfonates; and the C-terminal amino acid is amidated as a C-terminal primary amide.

In some embodiments, in the compounds of Formula (I) or a pharmaceutically acceptable salt thereof, 1 K, which is selected from the group consisting of K at position 16, K at position 24, K at position 28 and K at position 40, is chemically modified through conjugation to the ε-aminos of the K side-chains with ([2-(2-amino-ethoxy)-ethoxy]-acetyl)ₐ-(γ-Glu)_{b}-CO-(CH₂)_{c}-Z. For example, K at position 16 is chemically modified through conjugation to the ε-amino of the K side-chain with ([2-(2-amino-ethoxy)-ethoxy]-acetyl)ₐ-(γ-Glu)_{b}-CO-(CH₂)_{c}-Z; for example, K at position 24 is chemically modified through conjugation to the ε-amino of the K side-chain with ([2-(2-amino-ethoxy)-ethoxy]-acetyl)ₐ-(γ-Glu)_{b}-CO-(CH₂)_{c}-Z; for example, K at position 28 is chemically modified through conjugation to the ε-amino of the K side-chain with ([2-(2-amino-ethoxy)-ethoxy]-acetyl)ₐ-(γ-Glu)_{b}-CO-(CH₂)_{c}-Z; or for example K at position 40 is chemically modified through conjugation to the ε-amino of the K side-chain with ([2-(2-amino-ethoxy)-ethoxy]-acetyl)ₐ-(γ-Glu)_{b}-CO-(CH₂)_{c}-Z.

In some embodiments, in the compoud of Formula (I) or a pharmaceutically acceptable salt thereof, 2 Ks, which are selected from the group consisting of K at position 16, K at position 24, K at position 28 and K at position 40, are chemically modified with two modifying chains through conjugation to the ε-aminos of the K side-chains with ([2-(2-amino-ethoxy)-ethoxy]-acetyl)ₐ-(γ-Glu)_{b}-CO-(CH₂)_{c}-Z. For example, K at position 16 and K at position 24 are chemically modified with two modifying chains through conjugation to the ε-aminos of the K side-chains with ([2-(2-amino-ethoxy)-ethoxy]-acetyl)ₐ-(γ-Glu)_{b}-CO-(CH₂)_{c}-Z; for example, K at position 16 and K at position 28 are chemically modified with two modifying chains through conjugation to the ε-aminos of the K side-chains with ([2-(2-amino-ethoxy)-ethoxy]-acetyl)ₐ-(γ-Glu)_{b}-CO-(CH₂)_{c}-Z; for example, K at position 16 and K at position 40 are chemically modified with two modifying chains through conjugation to the ε-aminos of the K side-chains with ([2-(2-amino-ethoxy)-ethoxy]-acetyl)ₐ-(γ-Glu)_{b}-CO-(CH₂)_{c}-Z; for example, K at position 24 and K at position 28 are chemically modified with two modifying chains through conjugation to the ε-aminos of the K side-chains with ([2-(2-amino-ethoxy)-ethoxy]-acetyl)ₐ-(γ-Glu)_{b}-CO-(CH₂)_{c}-Z; for example, K at position 24 and K at position 40 are chemically modified with two modifying chains through conjugation to the ε-aminos of the K side-chains with ([2-(2-amino-ethoxy)-ethoxy]-acetyl)ₐ-(γ-Glu)_{b}-CO-(CH₂)_{c}-Z; for example, K at position 28 and K at position 40 are chemically modified with two modifying chains through conjugation to the ε-aminos of the K side-chains with ([2-(2-amino-ethoxy)-ethoxy]-acetyl)ₐ-(γ-Glu)_{b}-CO-(CH₂)_{c}-Z.

In some embodiments, in the compound of Formula (I) or a pharmaceutically acceptable salt thereof, 3 Ks, which are selected from the group consisting of K at position 16, K at position 24, K at position 28 and K at position 40, are chemically modified with three modifying chains through conjugation to the ε-aminos of the K side-chains with ([2-(2-amino-ethoxy)-ethoxy]-acetyl)ₐ-(γ-Glu)_{b}-CO-(CH₂)_{c}-Z. For example, K at position 16, K at position 24 and K at position 28 are chemically modified with three modifying chains through conjugation to the ε-aminos of the K side-chains with ([2-(2-amino-ethoxy)-ethoxy]-acetyl)ₐ-(γ-Glu)_{b}-CO-(CH₂)_{c}-Z; for example, K at position 16, K at position 24 and K at position 40 are chemically modified with three modifying chains through conjugation to the ε-aminos of the K side-chains with ([2-(2-amino-ethoxy)-ethoxy]-acetyl)ₐ-(γ-Glu)_{b}-CO-(CH₂)_{c}-Z; for example, K at position 16, K at position 28 and K at position 40 are chemically modified with three modifying chains through conjugation to the ε-aminos of the K side-chains with ([2-(2-amino-ethoxy)-ethoxy]-acetyl)ₐ-(γ-Glu)_{b}-CO-(CH₂)_{c}-Z; for example, K at position 24, K at position 28 and K at position 40 are chemically modified with three modifying chains through conjugation to the ε-aminos of the K side-chains with ([2-(2-amino-ethoxy)-ethoxy]-acetyl)ₐ-(γ-Glu)_{b}-CO-(CH₂)_{c}-Z.

In some embodiments, in the compound of Formula (I) or a pharmaceutically acceptable salt thereof, 4 Ks, which are selected from the group consisting of K at position 16, K at position 24, K at position 28 and K at position 40, are chemically modified with four modifying chains through conjugation to the ε-aminos of the K side-chains with ([2-(2-amino-ethoxy)-ethoxy]-acetyl)ₐ-(γ-Glu)_{b}-CO-(CH₂)_{c}-Z.

In some embodiments, in the compound of Formula (I) or a pharmaceutically acceptable salt thereof, Z is independently selected from the group consisting of -CH₃, carboxylic acids or carboxylic acid bioisosteres, phosphonates or sulfonates, for example, but not limited to, the Z can comprise carboxylic acids (-CO₂H) or carboxylic acid bioisosteres (such as 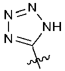 ), phosphonates (-P(O)(OH)₂) or sulfonates (-SO₂OH) groups, preferably -CO₂H.

Suitable carboxylic acid bioisosteres are known in the art. Preferably, the bioisosteres have protons with a pKₐ similar to that of the corresponding carboxylic acids. Examples of suitable bioisosteres may include, but are not limited to, tetrazoles, acylsulfonamides, acylhydroxylamines and squaric acid derivatives as shown below: 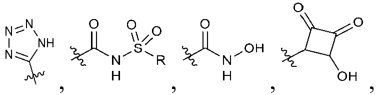 R is Me or CF₃.

In some embodiments, in the compound of Formula (I) or a pharmaceutically acceptable salt thereof, a is independently selected from an integer of 1, 2, 3, 4 or 5, preferably 1, 2 or 3, b is independently selected from an integer of 1, 2, 3, 4 or 5, preferably 1, 2 or 3, and c is independently selected from an integer of 12 to 22; in addition, the present invention provides a compound wherein c is 14, 16, 18 or 20.

An embodiment of the present invention provides a use of a compound of Formula (I) or a pharmaceutically acceptable salt thereof in the manufacture of a medicament for preventing or treating a disease in a non-primate and non-rodent animal:

Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K Formula (I);

wherein X₁ is Aib; X₂ is αMePhe; X₃ is Aib; X₄ is Aib; 1 or 2 Ks, which are selected from the group consisting of K at position 16, K at position 24, K at position 28 and K at position 40, are chemically modified through conjugation to the ε-aminos of the K side-chains with ([2-(2-amino-ethoxy)-ethoxy]-acetyl)ₐ-(γ-Glu)_{b}-CO-(CH₂)_{c}-Z, wherein Z is independently selected from the group consisting of -CH₃, carboxylic acids or carboxylic acid bioisosteres, phosphonates or sulfonates, preferably -CO₂H, each a is independently an integer from 0 to 5, each b is independently an integer from 0 to 5, and each c is independently an integer from 10 to 24; and the C-terminal amino acid is amidated as a C-terminal primary amide.

In another embodiment, the present invention provides a use of a compound of Formula (I) or a pharmaceutically acceptable salt thereof in the manufacture of a medicament for preventing or treating a disease in a non-primate and non-rodent animal, wherein, X₁ is Aib; X₂ is αMePhe; X₃ is Aib; X₄ is Aib; 1 or 2 Ks, which are selected from the group consisting of K at position 16, K at position 24, K at position 28 and K at position 40, are chemically modified through conjugation to the ε-aminos of the K side-chains with ([2-(2-amino-ethoxy)-ethoxy]-acetyl)ₐ-(γ-Glu)_{b}-CO-(CH₂)_{c}-Z, wherein Z is independently selected from the group consisting of -CH₃, carboxylic acids or carboxylic acid bioisosteres, phosphonates or sulfonates, preferably -CO₂H, a is independently selected from an integer of 1, 2, 3, 4 or 5, b is independently selected from an integer of 1, 2, 3, 4 or 5, and c is independently selected from an integer of 12 to 22; preferably, wherein c 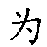 14, 16, 18 or 20.

An embodiment of the present invention provides a use of a compound of Formula (I) or a pharmaceutically acceptable salt thereof in the manufacture of a medicament for preventing or treating a disease in a non-primate and non-rodent animal:

Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K Formula (I);

wherein X₁ is Aib; X₂ is αMePhe; X₃ is Aib; X₄ is Aib; K at position 24 is chemically modified through conjugation to the ε-amino of the K side-chain with ([2-(2-amino-ethoxy)-ethoxy]-acetyl)ₐ-(γ-Glu)_{b}-CO-(CH₂)_{c}-CO₂H, wherein a is independently an integer from 0 to 5, b is independently an integer from 0 to 5, and c is independently an integer from 10 to 24; and the C-terminal amino acid is amidated as a C-terminal primary amide.

In another embodiment, the present invention provides a use of a compound of Formula (I) or a pharmaceutically acceptable salt thereof in the manufacture of a medicament for preventing or treating a disease in a non-primate and non-rodent animal, wherein, X₁ is Aib; X₂ is αMePhe; X₃ is Aib; X₄ is Aib; K at position 24 is chemically modified through conjugation to the ε-amino of the K side-chain with ([2-(2-amino-ethoxy)-ethoxy]-acetyl)ₐ-(γ-Glu)_{b}-CO-(CH₂)_{c}-CO₂H, wherein a is independently selected from an integer of 1, 2, 3, 4 or 5, b is independently selected from an integer of 1, 2, 3, 4 or 5, and c is independently selected from an integer of 12 to 22; preferably, c is 14, 16, 18 or 20.

In another embodiment, the present invention provides a use of a compound of Formula (I) or a pharmaceutically acceptable salt thereof in the manufacture of a medicament for preventing or treating a disease in a non-primate and non-rodent animal, wherein, X₁ is Aib; X₂ is αMePhe; X₃ is Aib; X₄ is Aib; K at position 24 is chemically modified through conjugation to the ε-amino of the K side-chain with ([2-(2-amino-ethoxy)-ethoxy]-acetyl)ₐ-(γ-Glu)_{b}-CO-(CH₂)_{c}-CO₂H, wherein each a is independently selected from an integer of 2 and 3, each b is independently 1, and each c is independently selected from an integer of 16, 18 and 20, wherein Z is independently selected from carboxylic acids.

Another embodiment of the present invention provides a use of a compound of Formula (I) or a pharmaceutically acceptable salt thereof in the manufacture of a medicament for preventing or treating a disease in a non-primate and non-rodent animal:

Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K Formula (I);

wherein X₁ is Aib; X₂ is αMePhe; X₃ is Aib; X₄ is Aib; K at position 28 is chemically modified through conjugation to the ε-amino of the K side-chain with ([2-(2-amino-ethoxy)-ethoxy]-acetyl)ₐ-(γ-Glu)_{b}-CO-(CH₂)_{c}-CO₂H, wherein a is independently selected from an integer from 0 to 5, b is independently selected from an integer from 0 to 5, and c is independently selected from an integer from 10 to 24; and the C-terminal amino acid is amidated as a C-terminal primary amide.

In another embodiment, the present invention provides a use of a compound of Formula (I) or a pharmaceutically acceptable salt thereof in the manufacture of a medicament for preventing or treating a disease in a non-primate and non-rodent animal, wherein, X₁ is Aib; X₂ is αMePhe; X₃ is Aib; X₄ is Aib; K at position 28 is chemically modified through conjugation to the ε-amino of the K side-chain with ([2-(2-amino-ethoxy)-ethoxy]-acetyl)ₐ-(γ-Glu)_{b}-CO-(CH₂)_{c}-CO₂H, wherein a is independently selected from an integer of 1, 2, 3, 4 or 5, b is independently selected from an integer of 1, 2, 3, 4 or 5, and c is independently selected from an integer of 12 to 22; preferably, c is 14, 16, 18 or 20.

In another embodiment, the present invention provides a use of a compound of Formula (I) or a pharmaceutically acceptable salt thereof in the manufacture of a medicament for preventing or treating a disease in a non-primate and non-rodent animal, wherein, X₁ is Aib; X₂ is αMePhe; X₃ is Aib; X₄ is Aib; K at position 28 is chemically modified through conjugation to the ε-amino of the K side-chain with ([2-(2-amino-ethoxy)-ethoxy]-acetyl)ₐ-(γ-Glu)_{b}-CO-(CH₂)_{c}-CO₂H, wherein each a is independently selected from an integer of 2 and 3, each b is independently selected from an integer of 1 and 3, and each c is independently selected from an integer of 16 and 20, wherein Z is independently selected from carboxylic acids.

In another embodiment, the present invention provides a use of a compound of Formula (I) or a pharmaceutically acceptable salt thereof in the manufacture of a medicament for preventing or treating a disease in a non-primate and non-rodent animal, wherein, X₁ is Aib; X₂ is αMePhe; X₃ is Aib; X₄ is Aib; K at position 28 is chemically modified through conjugation to the ε-amino of the K side-chain with ([2-(2-amino-ethoxy)-ethoxy]-acetyl)ₐ-(γ-Glu)_{b}-CO-(CH₂)_{c}-CO₂H, wherein each a is independently selected from an integer of 2 and 3, each b is independently selected from an integer of 1 and 3, and each c is independently selected from an integer of 16 and 20, wherein Z is independently selected from carboxylic acids.

Another embodiment of the present invention provides a use of a compound of Formula (I) or a pharmaceutically acceptable salt thereof in the manufacture of a medicament for preventing or treating a disease in a non-primate and non-rodent animal:

Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K Formula (I);

wherein X₁ is Aib; X₂ is αMePhe; X₃ is Aib; X₄ is Aib; K at position 16 is chemically modified through conjugation to the ε-amino of the K side-chain with ([2-(2-amino-ethoxy)-ethoxy]-acetyl)ₐ-(γ-Glu)_{b}-CO-(CH₂)_{c}-CO₂H, wherein a is independently selected from an integer from 0 to 5, b is independently selected from an integer from 0 to 5, and c is independently selected from an integer from 10 to 24; and the C-terminal amino acid is amidated as a C-terminal primary amide.

In another embodiment, the present invention provides a use of a compound of Formula (I) or a pharmaceutically acceptable salt thereof in the manufacture of a medicament for preventing or treating a disease in a non-primate and non-rodent animal, wherein, X₁ is Aib; X₂ is αMePhe; X₃ is Aib; X₄ is Aib; K at position 16 is chemically modified through conjugation to the ε-amino of the K side-chain with ([2-(2-amino-ethoxy)-ethoxy]-acetyl)ₐ-(γ-Glu)_{b}-CO-(CH₂)_{c}-CO₂H, wherein a is independently selected from an integer of 1, 2, 3, 4 or 5, b is independently selected from an integer of 1, 2, 3, 4 or 5, and c is independently selected from an integer of 12 to 22; preferably, c is 14, 16, 18 or 20.

Another embodiment of the present invention provides a use of a compound of Formula (I) or a pharmaceutically acceptable salt thereof in the manufacture of a medicament for preventing or treating a disease in a non-primate and non-rodent animal:

Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K Formula (I);

wherein X₁ is Aib; X₂ is αMePhe; X₃ is Aib; X₄ is Aib; K at position 40 is chemically modified through conjugation to the ε-amino of the K side-chain with ([2-(2-amino-ethoxy)-ethoxy]-acetyl)ₐ-(γ-Glu)_{b}-CO-(CH₂)_{c}-CO₂H, wherein a is independently selected from an integer from 0 to 5, b is independently selected from an integer from 0 to 5, and c is independently selected from an integer from 10 to 24; and the C-terminal amino acid is amidated as a C-terminal primary amide.

In another embodiment, the present invention provides a use of a compound of Formula (I) or a pharmaceutically acceptable salt thereof in the manufacture of a medicament for preventing or treating a disease in a non-primate and non-rodent animal, wherein, X₁ is Aib; X₂ is αMePhe; X₃ is Aib; X₄ is Aib; K at position 40 is chemically modified through conjugation to the ε-amino of the K side-chain with ([2-(2-amino-ethoxy)-ethoxy]-acetyl)ₐ-(γ-Glu)_{b}-CO-(CH₂)_{c}-CO₂H, wherein a is independently selected from an integer of 1, 2, 3, 4 or 5, b is independently selected from an integer of 1, 2, 3, 4 or 5, and c is independently selected from an integer of 12 to 22; preferably, c is 14, 16, 18 or 20.

Another embodiment of the present invention provides a use of a compound of Formula (I) or a pharmaceutically acceptable salt thereof in the manufacture of a medicament for preventing or treating a disease in a non-primate and non-rodent animal:

Y-X₁-E-G-T-X₂-T-S-D-Y-X₃-I-X₄-L-D-K-Q-A-Q-A-E-F-V-K-W-L-L-K-G-G-P-S-S-G-A-P-P-P S-K Formula (I);

wherein X₁ is Aib; X₂ is αMePhe; X₃ is Aib; X₄ is Aib; K at position 24 and K at position 28 are chemically modified with two modifying chains through conjugation to the ε-aminos of the K side-chains with ([2-(2-amino-ethoxy)-ethoxy]-acetyl)ₐ-(γ-Glu)_{b}-CO-(CH₂)_{c}-CO₂H, wherein each a is independently an integer from 0 to 5, each b is independently an integer from 0 to 5, and each c is independently an integer from 10 to 22; and the C-terminal amino acid is amidated as a C-terminal primary amide.

In another embodiment, the present invention provides a use of a compound of Formula (I) or a pharmaceutically acceptable salt thereof in the manufacture of a medicament for preventing or treating a disease in a non-primate and non-rodent animal, wherein, X₁ is Aib; X₂ is αMePhe; X₃ is Aib; X₄ is Aib; K at position 24 and K at position 28 are chemically modified with two modifying chains through conjugation to the ε-aminos of the K side-chains with ([2-(2-amino-ethoxy)-ethoxy]-acetyl)ₐ-(γ-Glu)_{b}-CO-(CH₂)_{c}-CO₂H, wherein a is independently selected from an integer of 1, 2, 3, 4 or 5, b is independently selected from an integer of 1, 2, 3, 4 or 5, and c is independently selected from an integer of 12 to 22; preferably, c is 14, 16, 18 or 20.

Another embodiment of the present invention provides a use of a compound of Formula (I) or a pharmaceutically acceptable salt thereof in the manufacture of a medicament for preventing or treating a disease in a non-primate and non-rodent animal:

Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K Formula (I);

wherein X₁ is Aib; X₂ is αMePhe; X₃ is Aib; X₄ is Aib; K at position 16 and K at position 24 are chemically modified with two modifying chains through conjugation to the ε-aminos of the K side-chains with ([2-(2-amino-ethoxy)-ethoxy]-acetyl)ₐ-(γ-Glu)_{b}-CO-(CH₂)_{c}-CO₂H, wherein each a is independently an integer from 0 to 5, each b is independently an integer from 0 to 5, and each c is independently an integer from 10 to 22; and the C-terminal amino acid is amidated as a C-terminal primary amide.

In another embodiment, the present invention provides a use of a compound of Formula (I) or a pharmaceutically acceptable salt thereof in the manufacture of a medicament for preventing or treating a disease in a non-primate and non-rodent animal, wherein, X₁ is Aib; X₂ is αMePhe; X₃ is Aib; X₄ is Aib; K at position 16 and K at position 24 are chemically modified with two modifying chains through conjugation to the ε-aminos of the K side-chains with ([2-(2-amino-ethoxy)-ethoxy]-acetyl)ₐ-(γ-Glu)_{b}-CO-(CH₂)_{c}-CO₂H, wherein a is independently selected from an integer of 1, 2, 3, 4 or 5, b is independently selected from an integer of 1, 2, 3, 4 or 5, and c is independently selected from an integer of 12 to 22; preferably, c is 14, 16, 18 or 20.

Another embodiment of the present invention provides a use of a compound of Formula (I) or a pharmaceutically acceptable salt thereof in the manufacture of a medicament for preventing or treating a disease in a non-primate and non-rodent animal:

Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K Formula (I);

wherein X₁ is Aib; X₂ is αMePhe; X₃ is Aib; X₄ is Aib; K at position 16 and K at position 28 are chemically modified with two modifying chains through conjugation to the ε-aminos of the K side-chains with ([2-(2-amino-ethoxy)-ethoxy]-acetyl)ₐ-(γ-Glu)_{b}-CO-(CH₂)_{c}-CO₂H, wherein each a is independently an integer from 0 to 5, each b is independently an integer from 0 to 5, and each c is independently an integer from 10 to 22; and the C-terminal amino acid is amidated as a C-terminal primary amide.

In another embodiment, the present invention provides a use of a compound of Formula (I) or a pharmaceutically acceptable salt thereof in the manufacture of a medicament for preventing or treating a disease in a non-primate and non-rodent animal, wherein, X₁ is Aib; X₂ is αMePhe; X₃ is Aib; X₄ is Aib; K at position 16 and K at position 28 are chemically modified with two modifying chains through conjugation to the ε-aminos of the K side-chains with ([2-(2-amino-ethoxy)-ethoxy]-acetyl)ₐ-(γ-Glu)_{b}-CO-(CH₂)_{c}-CO₂H, wherein a is independently selected from an integer of 1, 2, 3, 4 or 5, b is independently selected from an integer of 1, 2, 3, 4 or 5, and c is independently selected from an integer of 12 to 22; preferably, c is 14, 16, 18 or 20.

Another embodiment of the present invention provides a use of a compound of Formula (I) or a pharmaceutically acceptable salt thereof in the manufacture of a medicament for preventing or treating a disease in a non-primate and non-rodent animal:

Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K Formula (I);

wherein X₁ is Aib; X₂ is αMePhe; X₃ is Aib; X₄ is Aib; K at position 16 and K at position 40 are chemically modified with two modifying chains through conjugation to the ε-aminos of the K side-chains with ([2-(2-amino-ethoxy)-ethoxy]-acetyl)ₐ-(γ-Glu)_{b}-CO-(CH₂)_{c}-CO₂H, wherein each a is independently an integer from 0 to 5, each b is independently an integer from 0 to 5, and each c is independently an integer from 10 to 22; and the C-terminal amino acid is amidated as a C-terminal primary amide.

In another embodiment, the present invention provides a use of a compound of Formula (I) or a pharmaceutically acceptable salt thereof in the manufacture of a medicament for preventing or treating a disease in a non-primate and non-rodent animal, wherein, X₁ is Aib; X₂ is αMePhe; X₃ is Aib; X₄ is Aib; K at position 16 and K at position 40 are chemically modified with two modifying chains through conjugation to the ε-aminos of the K side-chains with ([2-(2-amino-ethoxy)-ethoxy]-acetyl)ₐ-(γ-Glu)_{b}-CO-(CH₂)_{c}-CO₂H, wherein a is independently selected from an integer of 1, 2, 3, 4 or 5, b is independently selected from an integer of 1, 2, 3, 4 or 5, and c is independently selected from an integer of 12 to 22; preferably, c is 14, 16, 18 or 20.

Another embodiment of the present invention provides a use of a compound of Formula (I) or a pharmaceutically acceptable salt thereof in the manufacture of a medicament for preventing or treating a disease in a non-primate and non-rodent animal:

Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K Formula (I);

wherein X₁ is Aib; X₂ is αMePhe; X₃ is Aib; X₄ is Aib; K at position 24 and K at position 40 are chemically modified with two modifying chains through conjugation to the ε-aminos of the K side-chains with ([2-(2-amino-ethoxy)-ethoxy]-acetyl)ₐ-(γ-Glu)_{b}-CO-(CH₂)_{c}-CO₂H, wherein each a is independently an integer from 0 to 5, each b is independently an integer from 0 to 5, and each c is independently an integer from 10 to 22; and the C-terminal amino acid is amidated as a C-terminal primary amide.

In another embodiment, the present invention provides a use of a compound of Formula (I) or a pharmaceutically acceptable salt thereof in the manufacture of a medicament for preventing or treating a disease in a non-primate and non-rodent animal, wherein, X₁ is Aib; X₂ is αMePhe; X₃ is Aib; X₄ is Aib; K at position 24 and K at position 40 are chemically modified with two modifying chains through conjugation to the ε-aminos of the K side-chains with ([2-(2-amino-ethoxy)-ethoxy]-acetyl)ₐ-(γ-Glu)_{b}-CO-(CH₂)_{c}-CO₂H, wherein a is independently selected from an integer of 1, 2, 3, 4 or 5, b is independently selected from an integer of 1, 2, 3, 4 or 5, and c is independently selected from an integer of 12 to 22; preferably, c is 14, 16, 18 or 20.

Another embodiment of the present invention provides a use of a compound of Formula (I) or a pharmaceutically acceptable salt thereof in the manufacture of a medicament for preventing or treating a disease in a non-primate and non-rodent animal:

Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K Formula (I);

wherein X₁ is Aib; X₂ is αMePhe; X₃ is Aib; X₄ is Aib; K at position 28 and K at position 40 are chemically modified with two modifying chains through conjugation to the ε-aminos of the K side-chains with ([2-(2-amino-ethoxy)-ethoxy]-acetyl)ₐ-(γ-Glu)_{b}-CO-(CH₂)_{c}-CO₂H, wherein each a is independently an integer from 0 to 5, each b is independently an integer from 0 to 5, and each c is independently an integer from 10 to 22; and the C-terminal amino acid is amidated as a C-terminal primary amide.

In another embodiment, the present invention provides a use of a compound of Formula (I) or a pharmaceutically acceptable salt thereof in the manufacture of a medicament for preventing or treating a disease in a non-primate and non-rodent animal, wherein, X₁ is Aib; X₂ is αMePhe; X₃ is Aib; X₄ is Aib; K at position 28 and K at position 40 are chemically modified with two modifying chains through conjugation to the ε-aminos of the K side-chains with ([2-(2-amino-ethoxy)-ethoxy]-acetyl)ₐ-(γ-Glu)_{b}-CO-(CH₂)_{c}-CO₂H, wherein a is independently selected from an integer of 1, 2, 3, 4 or 5, b is independently selected from an integer of 1, 2, 3, 4 or 5, and c is independently selected from an integer of 12 to 22; preferably, c is 14, 16, 18 or 20.

In all embodiments as described above, each a is independently an integer from 1 to 3, each b is independently an integer from 1 to 3, and each c is independently an integer from 12 to 22; in addition, the present invention provides a use of a compound of Formula (AI) or Formula (I), wherein c is 14, 16, 18 or 20, in the manufacture of a medicament for preventing or treating a disease in a non-primate and non-rodent animal. Among all embodiments as described above, preferred are embodiments wherein K at position 24 or K at position 28 is chemically modified with one modifying chain.

In an embodiment, the present invention provides a use of a compound of the following Formula or a pharmaceutically acceptable salt thereof in the manufacture of a medicament for preventing or treating a disease in a non-primate and non-rodent animal:

Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K;

wherein X₁ is Aib; X₂ is αMePhe; X₃ is Aib; X₄ is Aib; K at position 24 is chemically modified through conjugation to the ε-amino of the K side-chain with ([2-(2-amino-ethoxy)-ethoxy]-acetyl)₂-(γ-Glu)₁-CO-(CH₂)₁₆-CO₂H; and the C-terminal amino acid is amidated as a C-terminal primary amide (SEQ ID NO:7).

In an embodiment, the present invention provides a use of a compound of the following Formula or a pharmaceutically acceptable salt thereof in the manufacture of a medicament for preventing or treating a disease in a non-primate and non-rodent animal:

Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K;

wherein X₁ is Aib; X₂ is αMePhe; X₃ is Aib; X₄ is Aib; K at position 24 is chemically modified through conjugation to the ε-amino of the K side-chain with ([2-(2-amino-ethoxy)-ethoxy]-acetyl)₂-(γ-Glu)₁-CO-(CH₂)₁₈-CO₂H; and the C-terminal amino acid is amidated as a C-terminal primary amide (SEQ ID NO:13).

In an embodiment, the present invention provides a use of a compound of the following Formula or a pharmaceutically acceptable salt thereof in the manufacture of a medicament for preventing or treating a disease in a non-primate and non-rodent animal:

Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K;

wherein X₁ is Aib; X₂ is αMePhe; X₃ is Aib; X₄ is Aib; K at position 24 is chemically modified through conjugation to the ε-amino of the K side-chain with ([2-(2-amino-ethoxy)-ethoxy]-acetyl)₂-(γ-Glu)₁-CO-(CH₂)₂₀-CO₂H; and the C-terminal amino acid is amidated as a C-terminal primary amide (SEQ ID NO:14).

In an embodiment, the present invention provides a use of a compound of the following Formula or a pharmaceutically acceptable salt thereof in the manufacture of a medicament for preventing or treating a disease in a non-primate and non-rodent animal:

Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K;

wherein X₁ is Aib; X₂ is αMePhe; X₃ is Aib; X₄ is Aib; K at position 24 is chemically modified through conjugation to the ε-amino of the K side-chain with ([2-(2-amino-ethoxy)-ethoxy]-acetyl)₂-(γ-Glu)₃-CO-(CH₂)₁₆-CO₂H; and the C-terminal amino acid is amidated as a C-terminal primary amide (SEQ ID NO:15).

In an embodiment, the present invention provides a use of a compound of the following Formula or a pharmaceutically acceptable salt thereof in the manufacture of a medicament for preventing or treating a disease in a non-primate and non-rodent animal:

Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K;

wherein X₁ is Aib; X₂ is αMePhe; X₃ is Aib; X₄ is Aib; K at position 24 is chemically modified through conjugation to the ε-amino of the K side-chain with ([2-(2-amino-ethoxy)-ethoxy]-acetyl)₃-(γ-Glu)₁-CO-(CH₂)₂₀-CO₂H; and the C-terminal amino acid is amidated as a C-terminal primary amide (SEQ ID NO:16).

In an embodiment, the present invention provides a use of a compound of the following Formula or a pharmaceutically acceptable salt thereof in the manufacture of a medicament for preventing or treating a disease in a non-primate and non-rodent animal:

Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K;

wherein X₁ is Aib; X₂ is αMePhe; X₃ is Aib; X₄ is Aib; K at position 28 is chemically modified through conjugation to the ε-aminos of the K side-chains with ([2-(2-amino-ethoxy)-ethoxy]-acetyl)₂-(γ-Glu)₁-CO-(CH₂)₁₆-CO₂H; and the C-terminal amino acid is amidated as a C-terminal primary amide (SEQ ID NO:8).

In an embodiment, the present invention provides a use of a compound of the following Formula or a pharmaceutically acceptable salt thereof in the manufacture of a medicament for preventing or treating a disease in a non-primate and non-rodent animal:

Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K ;

wherein X₁ is Aib; X₂ is αMePhe; X₃ is Aib; X₄ is Aib; K at position 28 is chemically modified through conjugation to the ε-aminos of the K side-chains with ([2-(2-amino-ethoxy)-ethoxy]-acetyl)₂-(γ-Glu)₁-CO-(CH₂)₂₀-CO₂H; and the C-terminal amino acid is amidated as a C-terminal primary amide (SEQ ID NO:17).

In an embodiment, the present invention provides a use of a compound of the following Formula or a pharmaceutically acceptable salt thereof in the manufacture of a medicament for preventing or treating a disease in a non-primate and non-rodent animal:

Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K ;

wherein X₁ is Aib; X₂ is αMePhe; X₃ is Aib; X₄ is Aib; K at position 28 is chemically modified through conjugation to the ε-aminos of the K side-chains with ([2-(2-amino-ethoxy)-ethoxy]-acetyl)₂-(γ-Glu)₃-CO-(CH₂)₁₆-CO₂H; and the C-terminal amino acid is amidated as a C-terminal primary amide (SEQ ID NO:18).

In an embodiment, the present invention provides a use of a compound of the following Formula or a pharmaceutically acceptable salt thereof in the manufacture of a medicament for preventing or treating a disease in a non-primate and non-rodent animal:

Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K ;

wherein X₁ is Aib; X₂ is αMePhe; X₃ is Aib; X₄ is Aib; K at position 28 is chemically modified through conjugation to the ε-aminos of the K side-chains with ([2-(2-amino-ethoxy)-ethoxy]-acetyl)₃-(γ-Glu)₁-CO-(CH₂)₁₆-CO₂H; and the C-terminal amino acid is amidated as a C-terminal primary amide (SEQ ID NO:19).

In an embodiment, the present invention provides a use of a compound of the following Formula or a pharmaceutically acceptable salt thereof in the manufacture of a medicament for preventing or treating a disease in a non-primate and non-rodent animal:

Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K ;

wherein X₁ is Aib; X₂ is αMePhe; X₃ is Aib; X₄ is Aib; K at position 28 is chemically modified through conjugation to the ε-aminos of the K side-chains with ([2-(2-amino-ethoxy)-ethoxy]-acetyl)₃-(γ-Glu)₁-CO-(CH₂)₂₀-CO₂H; and the C-terminal amino acid is amidated as a C-terminal primary amide (SEQ ID NO:20).

Another object of the present invention is to provide some GLP-1R/GIPR dual agonist compounds for preventing or treating a disease in a non-primate and non-rodent animal. The compounds described in any one of the above embodiments or pharmaceutically acceptable salts thereof are suitable for the purpose of the present invention.

Another object of the present invention is to provide some methods for preventing or treating a disease in a non-primate and non-rodent animal, comprising administering to the animal in need thereof the GLP-1R/GIPR dual agonist compound according to the present invention. The compounds described in any one of the above embodiments or pharmaceutically acceptable salts thereof are suitable for the purpose of the present invention.

The present invention further provides a method for increasing the action duration of a GLP-1R/GIPR dual agonist in a non-primate and non-rodent animal, characterized in that the GLP-1R/GIPR dual agonist provided by the present invention as described above is used.

In an embodiment, the present invention provides a composition comprising a compound according to the present invention or a pharmaceutically acceptable salt thereof, and a pharmaceutically acceptable carrier, diluent or excipient.

An embodiment provides a use of the compound according to any one of the above embodiments or a pharmaceutically acceptable salt thereof in the manufacture of a medicament for preventing or treating the following diseases in a non-primate and non-rodent animal: animal hyperglycemia, impaired glucose tolerance, diabetes mellitus (including Type I diabetes mellitus, Type II diabetes mellitus), obesity, hypertension, dyslipidemia, cognitive impairment, atherosclerosis, myocardial infarction, coronary heart disease and other cardiovascular diseases, stroke, inflammatory bowel syndrome, dyspepsia and gastric ulcers.

An embodiment provides a use of the compound according to any one of the above embodiments, the compound or a pharmaceutically acceptable salt thereof is used to manufacture a medicament for preventing, delaying or treating Type II diabetes mellitus in a non-primate and non-rodent animal.

An embodiment provides a use of the compound according to any one of the above embodiments or a pharmaceutically acceptable salt thereof for the preparation of a medicament for reducing food intake, reducing β-cell apoptosis, increasing β-cell function and β-cell volume and/or restoring glucose sensitivity of β-cells in a non-primate and non-rodent animal.

In an embodiment, the present invention provides a method for preventing or treating Type II diabetes mellitus in a non-primate and non-rodent animal, comprising administering to an animal in need thereof, an effective amount of the compound according to the present invention or a pharmaceutically acceptable salt thereof.

In an embodiment, the present invention provides a method for improving blood glucose control in a non-primate and non-rodent animal suffering from Type II diabetes mellitus, comprising administering to an animal in need thereof, an effective amount of the compound according to the present invention or a pharmaceutically acceptable salt thereof as an adjunct to diet and exercise.

In an embodiment, the present invention provides a method for chronic weight management in an overweight or obese animal, comprising administering to an animal in need thereof, an effective amount of the compound according to the present invention or a pharmaceutically acceptable salt thereof as an adjunct to a reduced-calorie diet and increased physical activity.

In an embodiment, the present invention provides a method for treating metabolic syndrome, comprising administering to a non-primate and non-rodent animal in need thereof, an effective amount of the compound according to the present invention or a pharmaceutically acceptable salt thereof. In another embodiment, the present invention provides a method for preventing or treating dyslipidaemia, obesity and/or hepatic steatosis associated with insulin resistance and diabetes mellitus in a non-primate and non-rodent animal, comprising administering to an animal in need thereof, an effective amount of the compound according to the present invention or a pharmaceutically acceptable salt thereof. In addition, the present invention provides a method for preventing or treating frailty or increasing bone strength in a non-primate and non-rodent animal, comprising administering to an animal in need thereof, an effective amount of the compound according to the present invention or a pharmaceutically acceptable salt thereof. In another embodiment, the present invention provides a method for treating osteopenia and bone/joint diseases, such as for treating knee osteoarthritis, hip osteoarthritis, spondylitis deformans and lumbar pain in a non-primate and non-rodent animal, comprising administering to an animal in need thereof, an effective amount of the compound according to the present invention or a pharmaceutically acceptable salt thereof.

In an embodiment, the present invention provides the compound according to the present invention or a pharmaceutically acceptable salt thereof as an adjunct to diet and exercise for blood glucose control in a non-primate and non-rodent animal suffering from Type II diabetes mellitus. In an embodiment, the present invention provides the compound according to the present invention or a pharmaceutically acceptable salt thereof as an adjunct to a reduced-calorie diet and increased physical activity in an overweight or obese non-primate and a non-rodent animal for chronic weight management.

An embodiment provides the use according to any one of the above embodiments for the manufacture of a medicament for treating or preventing the above diseases in cats, dogs, rabbits, pigs, alpacas, horses, sheep, and bovines.

The compound according to the present invention has activation effect as disclosed above on GLP-1 receptors and GIP receptors, which indicates that the compounds according to the present invention or pharmaceutically acceptable salts thereof can be used as a medicament for preventing or treating diseases, for example, symptomatic obesity, obesity based on simple obesity, obesity-related conditions or diseases, eating disorders, diabetes mellitus (for example, Type I diabetes mellitus, Type II diabetes mellitus, gestational diabetes mellitus, obesity diabetes mellitus), hyperlipidemia (for example, hypertriglyceridemia, hypercholesterolemia, hyper-LDL-cholesterolemia, low HDL-cholesterolemia, postprandial hyperlipidemia), hypertension, heart failure, complications of diabetes mellitus (for example, neuropathy, nephropathy, retinopathy, diabetic cardiomyopathy, cataract, macroangiopathy, osteopenia, hyperosmolar diabetic coma), infectious disease (for example, respiratory tract infection, urinary tract infection, gastrointestinal infection, superficial soft tissue infection, lower extremity infection), diabetic gangrene, xerostomia, hypacusia, cerebrovascular disorder, peripheral blood circulation disorder, metabolic syndrome (with three or more conditions selected from the group consisting of hypertriglyceridemia (TG), low HDL-cholesterolemia , hypertension, abdominal obesity, and impaired glucose tolerance), and/or sarcopenia, and the like.

Examples of symptomatic obesity include: endocrine obesity (e.g., Cushing's syndrome, hypothyroidism, insulinoma, obese Type II diabetes mellitus, pseudohypoparathyroidism, hypogonadism), central obesity (e.g. hypothalamic obesity, frontal lobe syndrome, Klein-Lewis syndrome), genetic obesity (e.g., Prader-Willi syndrome, Laurence-Moon-Biedl's syndrome), drug-induced obesity (e.g., steroids, phenothiazines, insulin, sulfonylurea drugs, β-blocker induced obesity), and the like.

Examples of conditions or diseases associated with obesity include: glucose tolerance disorder, diabetes mellitus (especially Type II diabetes mellitus, obesity diabetes mellitus), abnormal lipid metabolism (synonymous with hyperlipidemia above), hypertension, heart failure, hyperuricemia, fatty liver (including non-alcoholic hepatitis), coronary heart disease (myocardial infarction, angina pectoris), cerebral infarction (cerebral thrombosis, transient ischemic attack), bone/joint disease (knee osteoarthritis, hip osteoarthritis, deformed spondylitis, low back pain), sleep apnea syndrome/Pickwick syndrome, menstrual disorders (abnormal menstrual cycle, amenorrhea, abnormal menstrual symptoms), metabolic syndrome, and the like.

The compounds provided by the present invention have GLP-1/GIP dual agonist activity, a long half-life, and show a longer duration of drug effect.

The compounds according to the present invention can react with any of a variety of inorganic and organic acids to form pharmaceutically acceptable acid addition salts. The pharmaceutically acceptable salts and the methods commonly used for their preparation are well-known in the art.

In an embodiment, the compounds according to the present invention are administered in the form of formulations. In addition to the compounds according to the present invention, the formulations further comprise pharmaceutically acceptable carriers, diluents, fillers, disintegrants, binders, lubricants, surfactants, hydrophobic vehicles, water-soluble vehicles, emulsifiers, buffers, wetting agents, moisturizers, solubilizers and preservatives, etc. The formulations described herein may be in the form of solutions, suspensions, emulsions, tablets, pills, granules, capsules, liquid-containing capsules, powders, sustained release preparations, suppositories, aerosols, sprays, or any other forms suitable for use. Saline solutions, aqueous glucose solutions, and aqueous glycerol solutions can be used as liquid carriers, especially for injectable solutions.

The compounds according to the present invention may be administered in any conventional manner by any route in which they are active, for example, they may be administered systemically or topically. For example, administration may be performed by inhalation, by depot injection, or by implantation. For example, administration may be, but is not limited to, parenteral, subcutaneous, intravenous, intramuscular, intraperitoneal, transdermal, oral, buccal, sublingual or ocular administration.

The compounds according to the present invention may be used with at least one other therapeutic agent for combination therapy. In some embodiments, the compounds according to the present invention may be administered in combination with additional diabetes drugs, anti-obesity drugs, blood pressure drugs, and/or cholesterol drugs. The compounds according to the present invention may act additively or synergistically with other therapeutic agent(s). The other therapeutic agent(s) and the compounds according to the present invention may be comprised in the same composition, or may be separately comprised in different compositions. The compositions containing a compound according to the invention and another therapeutic agent(s) can be administered simultaneously or sequentially. In another embodiment, the composition comprising a compound of the invention is administered before or after the other therapeutic agent(s).

In some embodiments, the compounds according to the present invention are used to treat or prevent Type II diabetes mellitus, overweight and/or obesity in cats or dogs.

In some embodiments, the compounds according to the present invention are administered in the form of solutions or suspensions.

In some embodiments, the compounds according to the present invention are administered by subcutaneous injection.

In some embodiments, the compounds according to the present invention are administered to cats or dogs at a dose of 0.003 to 2.5 mg/kg.

In some embodiments, the compounds according to the present invention are administered to cats or dogs at a dose of 0.003 to 0.6 mg/kg.

In some embodiments, the compounds according to the present invention or pharmaceutically acceptable salts thereof are administered to cats or dogs at a dose of about 0.005 to about 0.1mg/kg, about 0.1 to about 0.125mg/kg, about 0.1 to about 0.15mg/kg, about 0.15 to about 0.25mg/kg, about 0.125 to about 0.25mg/kg, about 0.005 to about 2 mg/kg, about 0.005 to about 1.5 mg/kg, about 0.005 to about 1 mg/kg, about 0.005 to about 0.5 mg/kg, about 0.1 to about 2 mg/kg, about 0.1 to about 1.5 mg/kg, about 0.1 to about 1 mg/kg, about 0.1 to about 0.5 mg/kg, about 0.15 to about 2.5 mg/kg, about 0.15 to about 2 mg/kg, about 0.15 to about 1.5 mg/kg, about 0.15 to about 1 mg/kg, about 0.15 to about 0.5 mg/kg, about 0.2 to about 2.5 mg/kg, about 0.2 to about 2 mg/kg, about 0.2 to about 1.5 mg/kg, about 0.2 to about 1 mg/kg, about 0.2 to about 0.5 mg/kg, about 0.25 to about 2.5 mg/kg, about 0.25 to about 2 mg/kg, about 0.25 to about 1.5 mg/kg, about 0.25 to about 1 mg/kg, about 0.25 to about 0.5 mg/kg, about 0.3 to about 2.5 mg/kg, about 0.3 to about 2 mg/kg, about 0.3 to about 1.5 mg/kg, about 0.3 to about 1 mg/kg, about 0.3 to about 0.5 mg/kg, about 0.301 to 2.001 mg/kg, about 0.35 to about 2.5 mg/kg, about 0.35 to about 2 mg/kg, about 0.35 to about 1.5 mg/kg, about 0.35 to about 1 mg/kg, about 0.35 to about 0.5 mg/kg, about 0.4 to about 2.5 mg/kg, about 0.4 to about 2 mg/kg, about 0.4 to about 1.5 mg/kg, about 0.4 to about 1 mg/kg, about 0.4 to about 0.5 mg/kg, about 0.5 to about 2.5 mg/kg, about 0.5 to about 2 mg/kg, about 0.5 to about 1.5 mg/kg, about 0.5 to about 1 mg/kg, about 2.5 mg/kg, about 2.0 mg/kg, about 1.95 mg/kg, about 1.9 mg/kg, about 1.85 mg/kg, about 1.8 mg/kg, about 1.75 mg/kg, about 1.7 mg/kg, about 1.65 mg/kg, about 1.6 mg/kg, about 1.55 mg/kg, about 1.5 mg/kg, about 1.45 mg/kg, about 1.4 mg/kg, about 1.35 mg/kg, about 1.3 mg/kg, about 1.25 mg/kg, about 1.2 mg/kg, about 1.15 mg/kg, about 1.1 mg/kg, about 1.05 mg/kg, about 1.0 mg/kg, about 0.95 mg/kg, about 0.9 mg/kg, about 0.85 mg/kg, about 0.8 mg/kg, about 0.75 mg/kg, about 0.7 mg/kg, about 0.65 mg/kg, about 0.6 mg/kg, about 0.55 mg/kg, about 0.5 mg/kg, about 0.45 mg/kg, about 0.4 mg/kg, about 0.35 mg/kg, about 0.3 mg/kg, about 0.25 mg/kg, about 0.2 mg/kg, about 0.15 mg/kg, or about 0.1 mg/kg.

In some embodiments, the compounds according to the present invention are administered to cats or dogs at a frequency of once per week (QW).

In some embodiments, the compounds according to the present invention or pharmaceutically acceptable salts thereof are administered to cats or dogs at a dose of about 0.003 to about 2.5 mg/kg QW, preferably about 0.01 to about 2mg/kg QW, more preferably about 0.25 to about 2.0 mg/kg QW, and/or, about 0.1 to about 2.0 mg/kg QW, further more preferably about 0.5 to about 2.0 mg/kg QW. For example, the compounds according to the present invention or pharmaceutically acceptable salts thereof are administered to cats or dogs at a dose of about 0.01 to about 2 mg/kg QW, about 0.01 to about 1.5 mg/kg QW, about 0.01 to about 1 mg/kg QW, about 0.01 to about 0.5 mg/kg QW, about 0.02 to about 2.5 mg/kg QW, about 0.02 to about 2 mg/kg QW, about 0.02 to about 1.5 mg/kg QW, about 0.02 to about 1 mg/kg QW, about 0.02 to about 0.5 mg/kg QW, about 0.021 to about 0.499 mg/kg QW, about 0.03 to about 2.5 mg/kg QW, about 0.03 to about 2 mg/kg QW, about 0.03 to about 1.5 mg/kg QW, about 0.03 to about 1 mg/kg QW, about 0.03 to about 0.5 mg/kg QW, about 0.04 to about 2.5 mg/kg QW, about 0.04 to about 2 mg/kg QW, about 0.04 to about 1.5 mg/kg QW, about 0.04 to about 1 mg/kg QW, about 0.04 to about 0.5 mg/kg QW, about 0.05 to about 2.5 mg/kg QW, about 0.05 to about 2 mg/kg QW, about 0.05 to about 1.5 mg/kg QW, about 0.05 to about 1 mg/kg QW, about 0.05 to about 0.5 mg/kg QW, about 0.06 to about 2.5 mg/kg QW, about 0.06 to about 2 mg/kg QW, about 0.06 to about 1.5 mg/kg QW, about 0.06 to about 1 mg/kg QW, about 0.06 to about 0.5 mg/kg QW, about 0.062 to2.001 mg/kg QW, about 0.07 to about 2.5 mg/kg QW, about 0.07 to about 2 mg/kg QW, about 0.07 to about 1.5 mg/kg QW, about 0.07 to about 1 mg/kg QW, about 0.07 to about 0.5 mg/kg QW, about 0.073 to 0.998 mg/kg QW, about 0.08 to about 2.5 mg/kg QW, about 0.08 to about 2 mg/kg QW, about 0.08 to about 1.5 mg/kg QW, about 0.08 to about 1 mg/kg QW, about 0.08 to about 0.5 mg/kg QW, about 0.09 to about 2.5 mg/kg QW, about 0.09 to about 2 mg/kg QW, about 0.09 to about 1.5 mg/kg QW, about 0.09 to about 1 mg/kg QW, about 0.09 to about 0.5 mg/kg QW, about 0.1 to about 2.5 mg/kg QW, about 0.1 to about 2 mg/kg QW, about 0.1 to about 1.5 mg/kg QW, about 0.1 to about 1 mg/kg QW, about 0.1 to about 0.5 mg/kg QW, about 0.15 to about 2.5 mg/kg QW, about 0.15 to about 2 mg/kg QW, about 0.15 to about 1.5 mg/kg QW, about 0.15 to about 1 mg/kg QW, about 0.15 to about 0.5 mg/kg QW, about 0.2 to about 2.5 mg/kg QW, about 0.2 to about 2 mg/kg QW, about 0.2 to about 1.5 mg/kg QW, about 0.2 to about 1 mg/kg QW, about 0.2 to about 0.5 mg/kg QW, about 0.25 to about 2.5 mg/kg QW, about 0.25 to about 2 mg/kg QW, about 0.25 to about 1.5 mg/kg QW, about 0.25 to about 1 mg/kg QW, about 0.25 to about 0.5 mg/kg QW, about 0.3 to about 2.5 mg/kg QW, about 0.3 to about 2 mg/kg QW, about 0.3 to about 1.5 mg/kg QW, about 0.3 to about 1 mg/kg QW, about 0.3 to about 0.5 mg/kg QW, about 0.301 to 2.001 mg/kg QW, about 0.35 to about 2.5 mg/kg QW, about 0.35 to about 2 mg/kg QW, about 0.35 to about 1.5 mg/kg QW, about 0.35 to about 1 mg/kg QW, about 0.35 to about 0.5 mg/kg QW, about 0.4 to about 2.5 mg/kg QW, about 0.4 to about 2 mg/kg QW, about 0.4 to about 1.5 mg/kg QW, about 0.4 to about 1 mg/kg QW, about 0.4 to about 0.5 mg/kg QW, about 0.5 to about 2 mg/kg QW, about 0.5 to about 1.5 mg/kg QW, about 0.5 to about 1 mg/kg QW, about 2.5 mg/kg QW, about 2.0 mg/kg QW, about 1.95 mg/kg QW, about 1.9 mg/kg QW, about 1.85 mg/kg QW, about 1.8 mg/kg QW, about 1.75 mg/kg QW, about 1.7 mg/kg QW, about 1.65 mg/kg QW, about 1.6 mg/kg QW, about 1.55 mg/kg QW, about 1.5 mg/kg QW, about 1.45 mg/kg QW, about 1.4 mg/kg QW, about 1.35 mg/kg QW, about 1.3 mg/kg QW, about 1.25 mg/kg QW, about 1.2 mg/kg QW, about 1.15 mg/kg QW, about 1.1 mg/kg QW, about 1.05 mg/kg QW, about 1.0 mg/kg QW, about 0.95 mg/kg QW, about 0.9 mg/kg QW, about 0.85 mg/kg QW, about 0.8 mg/kg QW, about 0.75 mg/kg QW, about 0.7 mg/kg QW, about 0.65 mg/kg QW, about 0.6 mg/kg QW, about 0.55 mg/kg QW, about 0.5 mg/kg QW, about 0.45 mg/kg QW, about 0.4 mg/kg QW, about 0.35 mg/kg QW, about 0.3 mg/kg QW, about 0.25 mg/kg QW, or about 0.2 mg/kg QW.

In some embodiments, the compounds according to the present invention or pharmaceutically acceptable salts thereof are continuously administered to cats or dogs at a frequency of once a week. The administration period is determined according to the needs of the subject, and the administration period may be 4-8 weeks, or 8-20 weeks, such as 4, 5, 6, 7 or 8 weeks. The dose for each administration may be the same or different.

In some embodiments, the compounds according to the invention or pharmaceutically acceptable salts thereof are continuously administered to dogs at a frequency of once a week for 6 weeks. The dose for each administration may be the same or different.

In some embodiments, the compounds according to the invention or pharmaceutically acceptable salts thereof are continuously administered to cats at a frequency of once a week for 5 weeks. The dose for each administration may be the same or different.

In some embodiments, the compounds according to the invention or pharmaceutically acceptable salts thereof are administered to cats or dogs at escalating doses.

In some embodiments, the compounds according to the present invention or pharmaceutically acceptable salts thereof are administered to cats at a dose that:
the first administration and the second administration are identical and at the first dose of about 0.02 to about 0.35 mg/kg QW,
the third adminisiation is at a second dose of about 0.2 to about 1 mg/kg QW, wherein the second dose is from about 1.2 to about 50 times of the first dose, preferably from about 10 to about 15 times, such as about 12 times,
the fourth administration and the fifth administration are identical and at the third dosge of from about 0.49 to about 2.5 mg/kg QW, wherein the third dose is from about 1.75 to about 2.5 times of the second dose, preferably from about 1.95 to about 2.05 times, more preferably about 2 times.

Preferably, the compounds according to the present invention or pharmaceutically acceptable salts thereof are administered to cats at a dose that:
the first dose is about 0.019 mg/kg QW,
the second dose is about 0.25 mg/kg QW, and
the third dose is about 0.5 mg/kg QW.

Preferably, the compounds according to the present invention or pharmaceutically acceptable salts thereof are administered to cats at a dose that
the first dose is about 0.075 mg/kg QW,
the second dose is about 0.5 mg/kg QW, and
the third dose is about 1.00 mg/kg QW.

Preferably, the compounds according to the present invention or pharmaceutically acceptable salts thereof are administered to cats at a dose that:
the first dose is about 0.30 mg/kg QW,
the second dose is about 1.00 mg/kg QW, and
the third dose is about 2.00 mg/kg QW.

In some embodiments, the compounds according to the present invention or pharmaceutically acceptable salts thereof are administered to cats at a dosage regimen that:
the dose is about 0.25 to about 2.5 mg/kg;
the administration frequency is QW; and
the administration period is 5 weeks.

In some embodiments, the compounds according to the present invention or pharmaceutically acceptable salts thereof are administered to cats at a dosage regimen that:
the dose is about 0.25 to about 2.5 mg per injection;
the administration frequency is QW; and
the administration period is 5 weeks.

In some embodiments, the compounds according to the present invention or pharmaceutically acceptable salts thereof are administered to dogs at a dose that:
the first administration and the second administration are identical and at the first dose of about 0.015 to about 0.07 mg/kg QW,
the third adminisiation is at a second dose of about 0.02 to about 0.14 mg/kg QW, preferably about 0.03 to about 0.1 mg/kg QW, wherein the second dose is from about 1.4 to 2 times of the first dose, preferably from about 1.45 to 1.6 times, more preferably about 1.5 times,
the fourth administration to the six administration are identical and at the third dosge of from about 0.49 to about 2.5 mg/kg QW, preferably about 0.495 to about 2.1 mg/kg QW, wherein the third dose is from about 13 to about 25 times of the second dose, preferably from about 16 to 21 times.

Preferably, the compounds according to the present invention or pharmaceutically acceptable salts thereof are administered to dogs at a dose that:
the first dose is about 0.02 mg/kg QW,
the second dose is about 0.03 mg/kg QW, and
the third dose is about 0.50 mg/kg QW.

Preferably, the compounds according to the present invention or pharmaceutically acceptable salts thereof are administered to dogs at a dose that:
the first dose is about 0.06 mg/kg QW,
the second dose is about 0.10mg/kg QW, and
the third dose is about 2.00 mg/kg QW.

Preferably, the compounds according to the present invention or pharmaceutically acceptable salts thereof are administered to dogs at a dosage regimen that:
the dose is about 0.1 to about 2.5 mg/kg;
the administration frequency is QW; and
the administration period is 6 weeks.

In some embodiments, the compounds according to the present invention or pharmaceutically acceptable salts thereof are administered to dogs at a dosage regimen that:
the dose is about 0.1 to about 2.5 mg per injection;
the administration frequency is QW; and
the administration period is 6 weeks.

In some embodiments, the above doses in the unit of mg/kg may be replaced with flat doses in the unit of mg.

In some embodimetns, the compounds according to the present invention or pharmaceutically acceptable salts thereof are administered to cats at a flat dose of from about 0.25 to about 2.5 mg per injection QW. Preferably, the compounds according to the present invention or pharmaceutically acceptable salts thereof are administered to cats at a flat dose as below per injection: about 0.25 to about 2.5 mg QW, about 0.25 to about 2 mg QW, about 0.25 to about 1.5 mg QW, about 0.25 to about 1 mg QW, about 0.25 to about 0.5 mg QW, about 0.3 to about 2.5 mg QW, about 0.3 to about 2 mg QW, about 0.3 to about 1.5 mg QW, about 0.3 to about 1 mg QW, about 0.3 to about 0.5 mg QW, about 0.301 to 2.001 mg QW, about 0.35 to about 2.5 mg QW, about 0.35 to about 2 mg QW, about 0.35 to about 1.5 mg QW, about 0.35 to about 1 mg QW, about 0.35 to about 0.5 mg QW, about 0.4 to about 2.5 mg QW, about 0.4 to about 2 mg QW, about 0.4 to about 1.5 mg QW, about 0.4 to about 1 mg QW, about 0.4 to about 0.5 mg QW, about 0.5 to about 2 mg QW, about 0.5 to about 1.5 mg QW, about 0.5 to about 1 mg QW, about 2.5 mg QW, about 2.0 mg QW, about 1.95 mg QW, about 1.9 mg QW, about 1.85 mg QW, about 1.8 mg QW, about 1.75 mg QW, about 1.7 mg QW, about 1.65 mg QW, about 1.6 mg QW, about 1.55 mg QW, about 1.5 mg QW, about 1.45 mg QW, about 1.4 mg QW, about 1.35 mg QW, about 1.3 mg QW, about 1.25 mg QW, about 1.2 mg QW, about 1.15 mg QW, about 1.1 mg QW, about 1.05 mg QW, about 1.0 mg QW, about 0.95 mg QW, about 0.9 mg QW, about 0.85 mg QW, about 0.8 mg QW, about 0.75 mg QW, about 0.7 mg QW, about 0.65 mg QW, about 0.6 mg QW, about 0.55 mg QW, about 0.5 mg QW, about 0.45 mg QW, about 0.4 mg QW, about 0.35 mg QW, about 0.3 mg QW, or about 0.25 mg QW.

In some embodimetns, the compounds according to the present invention or pharmaceutically acceptable salts thereof are administered to dogs at a flat dose of from about 0.1 to about 2.5 mg per injection QW. Preferably, the compounds according to the present invention or pharmaceutically acceptable salts thereof are administered to dogs at a flat dose as below per injection: about 0.1 to about 2 mg QW, about 0.1 to about 1.5 mg QW, about 0.1 to about 1 mg QW, about 0.1 to about 0.5 mg QW, about 0.15 to about 2.5 mg QW, about 0.15 to about 2 mg QW, about 0.15 to about 1.5 mg QW, about 0.15 to about 1 mg QW, about 0.15 to about 0.5 mg QW, about 0.2 to about 2.5 mg QW, about 0.2 to about 2 mg QW, about 0.2 to about 1.5 mg QW, about 0.2 to about 1 mg QW, about 0.2 to about 0.5 mg QW, about 0.25 to about 2.5 mg QW, about 0.25 to about 2 mg QW, about 0.25 to about 1.5 mg QW, about 0.25 to about 1 mg QW, about 0.25 to about 0.5 mg QW, about 0.3 to about 2.5 mg QW, about 0.3 to about 2 mg QW, about 0.3 to about 1.5 mg QW, about 0.3 to about 1 mg QW, about 0.3 to about 0.5 mg QW, about 0.301 to2.001 mg QW, about 0.35 to about 2.5 mg QW, about 0.35 to about 2 mg QW, about 0.35 to about 1.5 mg QW, about 0.35 to about 1 mg QW, about 0.35 to about 0.5 mg QW, about 0.4 to about 2.5 mg QW, about 0.4 to about 2 mg QW, about 0.4 to about 1.5 mg QW, about 0.4 to about 1 mg QW, about 0.4 to about 0.5 mg QW, about 0.5 to about 2 mg QW, about 0.5 to about 1.5 mg QW, about 0.5 to about 1 mg QW, about 2.5 mg QW, about 2.0 mg QW, about 1.95 mg QW, about 1.9 mg QW, about 1.85 mg QW, about 1.8 mg QW, about 1.75 mg QW, about 1.7 mg QW, about 1.65 mg QW, about 1.6 mg QW, about 1.55 mg QW, about 1.5 mg QW, about 1.45 mg QW, about 1.4 mg QW, about 1.35 mg QW, about 1.3 mg QW, about 1.25 mg QW, about 1.2 mg QW, about 1.15 mg QW, about 1.1 mg QW, about 1.05 mg QW, about 1.0 mg QW, about 0.95 mg QW, about 0.9 mg QW, about 0.85 mg QW, about 0.8 mg QW, about 0.75 mg QW, about 0.7 mg QW, about 0.65 mg QW, about 0.6 mg QW, about 0.55 mg QW, about 0.5 mg QW, about 0.45 mg QW, about 0.4 mg QW, about 0.35 mg QW, about 0.3 mg QW, or about 0.25 mg QW.

The dosage regimen according to the present invention significantly reduces body weights, reduces food taking, and reduces BMI, SFV, TFV, AC and BCS, etc, of dogs, cats, rabbits, pigs, alpacas, horses, sheep, and bovines, especially of dogs and cats.

In the context, "dog ( 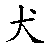 )" and "dog ( 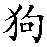 )" are synonyms and are used exchangeably.

The amino acid sequences of the present invention contain the standard single letter or three letter codes for twenty natural amino acids. In addition, "Aib" is α-aminoisobutyric acid, "αMePhe" is α-methylphenylalanine, "Pya(4)" is 4-pyridylalanine; "Cha" is cyclohexylalanine, "αMeTyr" is α-methyltyrosine.

"AEEA" as disclosed in the present invention is an abbreviation for 2-(2-amino-ethoxy)-ethoxy]-acetyl. "Ste" as disclosed in the present invention represents octadecanedioyl -C(O)-C₁₆H₃₂-C(O)-derived from octadecanedioic acid.

"Effective amount" or "therapeutically effective amount" as disclosed in the present invention refers to the amount or dose of the compound or a pharmaceutically acceptable salt thereof according to the present invention which, upon single or multiple dose administration to the subject, provides the desired effect in the animal under diagnosis or treatment.

"Minimum effective dose" as disclosed in the present invention refers to the minimum administration dose at which the drug effect is produced. Specifically, as the drug effect experiment of multiple administrations on db/db mice in the present invention is concerned, it refers to the minimum dose at which the blood glucose AUC of the group taken the test compound is significantly different (p<0.05) from that of the Vehicle group.

"Efficacy dose" as disclosed in the present invention refers to the administration dose at which the drug effect is produced. Specifically, as the drug effect experiment of multiple administrations on db/db mice in the present invention is concerned, it refers to the dose at which the blood glucose AUC of the group taken the test compound is significantly different (p<0.05) from that of the Vehicle group.

"Vehicle group" as disclosed in the present invention refers to the vehicle control group.

The "AUC" as disclosed in the present invention refers to the area under the concentration-time curve, that is, the area surrounded by the drug concentration-time curve and the time axis. This parameter is an important indicator to evaluate the degree of drug absorption, which reflects the exposure characteristics of a drug in the body.

The "MRT" as disclosed in the present invention refers to the mean residence time, and is the average value that a drug molecule resides in the body and means the time required for 63.2% of the drug to be eliminated from the body.

The "MTD" as disclosed in the present invention refers to the maximum tolerated dose, which is the highest dose that does not cause the death of the test animals.

The "internal standard" as disclosed in the present invention refers to a specified amount of a certain pure compound added to the sample for correcting the errors due to signal fluctuations of instruments, personnel operations and the like.

The "inhibition of blood glucose AUC" as disclosed in the present invention is the reduction percentage of blood glucose AUC in the group taken the test compound as compared with the group taken the Vehicle. Inhibition of blood glucose AUC=(blood glucose AUC_{Vehicle}-blood glucose AUC_{test compound})/blood glucose AUC_{Vehicle}.

The "body weight change rate" as disclosed in the present invention is the change percentage of body weight in the group taken the test compound after administration as compared with the body weight thereof before administration, and reflects effect of the test compound on the body weight. For example, body weight change rate on day 28 (D28)=(body weight_{D28 after administration}-body weight_{before administration})/body weight_{before administration} ×100%.

The "body weight reduction rate" as disclosed in the present invention is the reduction percentage of body weight in the group taken the test compound after administration as compared with the body weight thereof before administration, and reflects the reduction effect of the test compound. For example, weight reduction rate on day 28 (D28)=(body weight_{before administration}-body weight_{D28 after administration})/body weight_{before administration} ×100%.

The "titration strategy" as disclosed in the present invention is a dose escalation method, that is, after the initial dose, the frequency for dose escalation and the dosage are adjusted to obtain the optimal administration dosage.

"Treatment" as disclosed in the present invention includes attenuating, inhibiting, reversing, slowing, delaying or halting the progression or severity of an existing condition, disease, disorder or symptom. "Prevention" as disclosed in the present invention includes reducing the risk of acquiring a particular disease, disease condition or disorder.

Unless otherwise specified or clearly contradictory, the "animal" as disclosed in the present invention means a non-primate and non-rodent animal, that is, humans, non-human primates and rodents are excluded. The "animal" as disclosed in the present invention includes pets. Unless otherwise specified or apparently contradictory, the "pet" as disclosed in the present invention means non-primate and non-rodent pets, that is, primates and rodents are excluded. The "primates" as disclosed in the present invention include monkeys, such as cynomolgus monkeys. The "rodents" as disclosed in the present invention include rats and mice. Unless otherwise specified or apparently contradictory, the "animals", "non-primate and non-rodent animals", "pets", "non-primate and non-rodent pets" as disclosed in the present invention are used interchangeably and all include dogs, cats, rabbits, pigs, alpacas, horses, sheep and bovines.

The "modifying chain" as disclosed in the present invention is ([2-(2-amino-ethoxy)-ethoxy]-acetyl)ₐ-(γ-Glu)_{b}-CO-(CH₂)_{c}-Z, wherein each a is independently an integer from 0 to 5, each b is independently an integer from 0 to 5, and each c is independently an integer from 10 to 22; preferably, wherein a is independently selected from an integer of 1, 2, 3, 4 or 5, b is independently selected from an integer of 1, 2, 3, 4 or 5, and c is independently selected from an integer of 12 to 22; in addition, the present invention further provides compounds wherein c is 14, 16, 18 or 20, wherein Z is independently selected from the group consisting of -CH₃, carboxylic acids or carboxylic acid bioisosteres, phosphonates or sulfonates, for example, but not limited to the cases where said Z may comprise a carboxy acid (-CO₂H) or carboxylic acid bioisosteres (for example 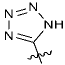 ), a phosphonic acid (-P(O)(OH)₂) or a sulfonic acid (-SO₂OH) group, preferably -CO₂H.

As for carboxylic acid bioisosteres, suitable carboxylic acid bioisosteres are well-known in the art. Preferably, the bioisosteres have protons with a pKa similar to that of the corresponding carboxylic acid. Examples of suitable bioisosteres may include, but are not limited to, tetrazoles, acylsulfonamides, acylhydroxylamines and squaric acid derivatives, as shown below: 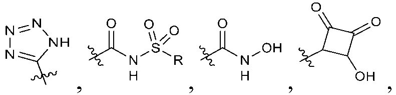 R is Me or CF₃.

As for the peptides mentioned herein, according to conventional peptide designation, the left end is the N-terminus (amino-terminus) and the right end is the C-terminus (carboxyl-terminus). The C-terminus of the peptide can be any one of amide (-CONH₂), carboxyl (-COOH), carboxylate (-COO⁻), alkylamide (-CONHR') and ester (-COOR'), and R' is C₁₋₈ alkyl. In particular, amides (-CONH₂) are preferred.

The "coupling" or "incorporating" in the preparation process of the present invention refers to the process of adding a new amino acid to the combined amino acids or peptide.

In the context, the value modified by "about" can have a deviation of ±10%. For example, "about 10" indicates a fluctuation within the range of from 9 to 11.

In the context, "escalating doses" means that in two adjacent doses, the subsequent dose is equal to or greater than the preceding dose. "Escalating" is used in contrast to "decreasing," and "escalating" includes the situation where two or more consecutive administrations are at at the same dose.

In the context, "overweight" refers to the body weight of a non-primate and non-rodent animals with a BCS (Body Condition Score) of ≥6.

### DETAILED DESCRIPTION OF EMBODIMENTS

1. Use of a compound of the following Formula (AI) or a pharmaceutically acceptable salt thereof in the manufacture of a medicament for preventing or treating a disease in a non-primate and non-rodent animal:

   Y-X₁-E-G-T-X₂-T-S-D-Y-A11-A12-A13-L-D-K-A17-A-Q-A20-E-F-V-K-W-L-L-K-A29-G-P-S- S-G-A-P-P-P-S-K Formula (AI);

   wherein X₁ is Aib; X₂ is αMePhe;
   A11 is Aib or Ala;
   A12 is Ala, Ile, Lys, Phe or Pya(4);
   A13 is Aib, Cha, Leu, αMePhe or αMeTyr;
   A17 is Gln or Ile;
   A20 is Ala or Ser;
   A29 is Gln or Gly,
   1, 2, 3 or 4 Ks, which are selected from the group consisting of K at position 16, K at position 24, K at position 28 and K at position 40, are chemically modified through conjugation to the ε-aminos of the K side-chains with ([2-(2-amino-ethoxy)-ethoxy]-acetyl)ₐ-(γ-Glu)_{b}-CO-(CH₂)_{c}-Z, wherein each a is independently an integer from 0 to 5, each b is independently an integer from 0 to 5, and each c is independently an integer from 10 to 24, wherein Z is independently selected from the group consisting of -CH₃, carboxylic acids or carboxylic acid bioisosteres, phosphonates or sulfonates.
2. The use according to Embodiment 1, wherein
   A11 is Aib; or/and
   A12 is Ile; or/and
   A13 is Aib; or/and
   A17 is Gln; or/and
   A20 is Ala; or/and
   A29 is Gly; or/and
   a is independently selected from an integer of 1, 2, 3, 4 or 5, b is independently selected from an integer of 1, 2, 3, 4 or 5, and c is independently selected from an integer of 12 to 22; preferably, c is independently selected from 14, 16, 18 or 20.
3. Use of a compound of the following Formula (I) or a pharmaceutically acceptable salt thereof in the manufacture of a medicament for preventing or treating a disease in a non-primate and non-rodent animal:

   Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K Formula (I);

   wherein X₁ is Aib; X₂ is αMePhe; X₃ is Aib; X₄ is Aib; 1, 2, 3 or 4 Ks, which are selected from the group consisting of K at position 16, K at position 24, K at position 28 and K at position 40, are chemically modified through conjugation to the ε-aminos of the K side-chains with ([2-(2-amino-ethoxy)-ethoxy]-acetyl)ₐ-(γ-Glu)_{b}-CO-(CH₂)_{c}-Z, wherein each a is independently an integer from 0 to 5, each b is independently an integer from 0 to 5, and each c is independently an integer from 10 to 24, wherein Z is independently selected from the group consisting of -CH₃, carboxylic acids or carboxylic acid bioisosteres, phosphonates or sulfonates; and the C-terminal amino acid is amidated as a C-terminal primary amide.
4. The use according to Embodiment 3, wherein the compound is:

   Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K Formula (I),

   or a pharmaceutically acceptable salt thereof, wherein X₁ is Aib; X₂ is αMePhe; X₃ is Aib; X₄ is Aib; 1 or 2 Ks, which are selected from the group consisting of K at position 16, K at position 24, K at position 28 and K at position 40, are chemically modified through conjugation to the ε-aminos of the K side-chains with ([2-(2-amino-ethoxy)-ethoxy]-acetyl)ₐ-(γ-Glu)_{b}-CO-(CH₂)_{c}-Z, wherein Z is independently selected from the group consisting of -CH₃, carboxylic acids or carboxylic acid bioisosteres, phosphonates or sulfonates, preferably -CO₂H, each a is independently an integer from 0 to 5, each b is independently an integer from 0 to 5, and each c is independently an integer from 10 to 22; and the C-terminal amino acid is amidated as a C-terminal primary amide.
5. The use according to Embodiment 4, wherein
   K at position 16 is chemically modified through conjugation to the ε-amino of the K side-chain with ([2-(2-amino-ethoxy)-ethoxy]-acetyl)ₐ-(γ-Glu)_{b}-CO-(CH₂)_{c}-CO₂H; or
   K at position 24 is chemically modified through conjugation to the ε-amino of the K side-chain with ([2-(2-amino-ethoxy)-ethoxy]-acetyl)ₐ-(γ-Glu)_{b}-CO-(CH₂)_{c}-CO₂H; or
   K at position 28 is chemically modified through conjugation to the ε-amino of the K side-chain with ([2-(2-amino-ethoxy)-ethoxy]-acetyl)ₐ-(γ-Glu)_{b}-CO-(CH₂)_{c}-CO₂H; or
   K at position 40 is chemically modified through conjugation to the ε-amino of the K side-chain with ([2-(2-amino-ethoxy)-ethoxy]-acetyl)ₐ-(γ-Glu)_{b}-CO-(CH₂)_{c}-CO₂H.
6. The use according to Embodiment 4, wherein
   K at position 24 and K at position 28 are chemically modified with two modifying chains through conjugation to the ε-aminos of the K side-chains with([2-(2-amino-ethoxy)-ethoxy]-acetyl)ₐ-(γ-Glu)_{b}-CO-(CH₂)_{c}-CO₂H; or
   K at position 16 and K at position 24 are chemically modified with two modifying chains through conjugation to the ε-aminos of the K side-chains with ([2-(2-amino-ethoxy)-ethoxy]-acetyl)ₐ-(γ-Glu)_{b}-CO-(CH₂)_{c}-CO₂H; or
   K at position 16 and K at position 28 are chemically modified with two modifying chains through conjugation to the ε-aminos of the K side-chains with ([2-(2-amino-ethoxy)-ethoxy]-acetyl)ₐ-(γ-Glu)_{b}-CO-(CH₂)_{c}-CO₂H; or
   K at position 16 and K at position 40 are chemically modified with two modifying chains through conjugation to the ε-aminos of the K side-chains with ([2-(2-amino-ethoxy)-ethoxy]-acetyl)ₐ-(γ-Glu)_{b}-CO-(CH₂)_{c}-CO₂H; or
   K at position 24 and K at position 40 are chemically modified with two modifying chains through conjugation to the ε-aminos of the K side-chains with ([2-(2-amino-ethoxy)-ethoxy]-acetyl)ₐ-(γ-Glu)_{b}-CO-(CH₂)_{c}-CO₂H; or
   K at position 28 and K at position 40 are chemically modified with two modifying chains through conjugation to the ε-aminos of the K side-chains with ([2-(2-amino-ethoxy)-ethoxy]-acetyl)ₐ-(γ-Glu)_{b}-CO-(CH₂)_{c}-CO₂H.
7. The use according to any one of Embodiments 3-6, wherein
   a is independently selected from an integer of 1, 2, 3, 4 or 5, b is independently selected from an integer of 1, 2, 3, 4 or 5, and c is independently selected from an integer of 12 to 22; preferably, c is independently selected from 14, 16, 18 or 20; and/or
   Z is -CO₂H.
8. The use according to Embodiment 4, wherein the compound is

   Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K Formula (I);

   wherein X₁ is Aib; X₂ is αMePhe; X₃ is Aib; X₄ is Aib; K at position 24 is chemically modified through conjugation to the ε-amino of the K side-chain with ([2-(2-amino-ethoxy)-ethoxy]-acetyl)ₐ-(γ-Glu)_{b}-CO-(CH₂)_{c}-CO₂H, wherein a is independently selected from an integer from 0 to 5, b is independently selected from an integer from 0 to 5, and c is independently selected from an integer from 10 to 24; and the C-terminal amino acid is amidated as a C-terminal primary amide;
   preferably, wherein, X₁ is Aib; X₂ is αMePhe; X₃ is Aib; X₄ is Aib; K at position 24 is chemically modified through conjugation to the ε-amino of the K side-chain with ([2-(2-amino-ethoxy)-ethoxy]-acetyl)ₐ-(γ-Glu)_{b}-CO-(CH₂)_{c}-CO₂H, wherein each a is independently selected from an integer of 2 and 3, each b is independently 1, and each c is independently selected from an integer of 16, 18 and 20, wherein Z is independently selected from carboxylic acids.
9. The use according to Embodiment 4, the compound is

   Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K Formula (I);

   wherein X₁ is Aib; X₂ is αMePhe; X₃ is Aib; X₄ is Aib; K at position 28 is chemically modified through conjugation to the ε-amino of the K side-chain with ([2-(2-amino-ethoxy)-ethoxy]-acetyl)ₐ-(γ-Glu)_{b}-CO-(CH₂)_{c}-CO₂H, wherein a is independently selected from an integer from 0 to 5, b is independently selected from an integer from 0 to 5, and c is independently selected from an integer from 10 to 24; and the C-terminal amino acid is amidated as a C-terminal primary amide;
   preferably, wherein, X₁ is Aib; X₂ is αMePhe; X₃ is Aib; X₄ is Aib; K at position 28 is chemically modified through conjugation to the ε-amino of the K side-chain with ([2-(2-amino-ethoxy)-ethoxy]-acetyl)ₐ-(γ-Glu)_{b}-CO-(CH₂)_{c}-CO₂H, wherein each a is independently selected from an integer of 2 and 3, each b is independently selected from an integer of 1 and 3, and each c is independently selected from an integer of 16 and 20, wherein Z is independently selected from carboxylic acids.
10. The use according to any one of Embodiments 8-9, wherein
   a is independently selected from an integer of 1, 2, 3, 4 or 5, b is independently selected from an integer of 1, 2, 3, 4 or 5, and c is independently selected from an integer of 12 to 22; preferably, c is independently selected from 14, 16, 18 or 20.
11. The use according to Embodiments 4, the compound is

   Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K;

   wherein X₁ is Aib; X₂ is αMePhe; X₃ is Aib; X₄ is Aib; K at position 24 is chemically modified through conjugation to the ε-amino of the K side-chain with ([2-(2-amino-ethoxy)-ethoxy]-acetyl)₂-(γ-Glu)₁-CO-(CH₂)₁₆-CO₂H; and the C-terminal amino acid is amidated as a C-terminal primary amide (SEQ ID NO:7); or

   Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K;

   wherein X₁ is Aib; X₂ is αMePhe; X₃ is Aib; X₄ is Aib; K at position 24 is chemically modified through conjugation to the ε-amino of the K side-chain with ([2-(2-amino-ethoxy)-ethoxy]-acetyl)₂-(γ-Glu)₁-CO-(CH₂)₁₈-CO₂H; and the C-terminal amino acid is amidated as a C-terminal primary amide (SEQ ID NO:13); or

   Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K;

   wherein X₁ is Aib; X₂ is αMePhe; X₃ is Aib; X₄ is Aib; K at position 24 is chemically modified through conjugation to the ε-amino of the K side-chain with ([2-(2-amino-ethoxy)-ethoxy]-acetyl)₂-(γ-Glu)₁-CO-(CH₂)₂₀-CO₂H; and the C-terminal amino acid is amidated as a C-terminal primary amide (SEQ ID NO:14); or

   Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K;

   wherein X₁ is Aib; X₂ is αMePhe; X₃ is Aib; X₄ is Aib; K at position 24 is chemically modified through conjugation to the ε-amino of the K side-chain with ([2-(2-amino-ethoxy)-ethoxy]-acetyl)₂-(γ-Glu)₃-CO-(CH₂)₁₆-CO₂H; and the C-terminal amino acid is amidated as a C-terminal primary amide (SEQ ID NO:15); or

   Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K;

   wherein X₁ is Aib; X₂ is αMePhe; X₃ is Aib; X₄ is Aib; K at position 24 is chemically modified through conjugation to the ε-amino of the K side-chain with ([2-(2-amino-ethoxy)-ethoxy]-acetyl)₃-(γ-Glu)₁-CO-(CH₂)₂₀-CO₂H; and the C-terminal amino acid is amidated as a C-terminal primary amide (SEQ ID NO:16); or

   Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K;

   wherein X₁ is Aib; X₂ is αMePhe; X₃ is Aib; X₄ is Aib; K at position 28 is chemically modified through conjugation to the ε-amino of the K side-chain with ([2-(2-amino-ethoxy)-ethoxy]-acetyl)₂-(γ-Glu)₁-CO-(CH₂)₁₆-CO₂H; and the C-terminal amino acid is amidated as a C-terminal primary amide (SEQ ID NO:8); or

   Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K;

   wherein X₁ is Aib; X₂ is αMePhe; X₃ is Aib; X₄ is Aib; K at position 28 is chemically modified through conjugation to the ε-amino of the K side-chain with ([2-(2-amino-ethoxy)-ethoxy]-acetyl)₂-(γ-Glu)₁-CO-(CH₂)₂₀-CO₂H; and the C-terminal amino acid is amidated as a C-terminal primary amide (SEQ ID NO:17); or

   Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K;

   wherein X₁ is Aib; X₂ is αMePhe; X₃ is Aib; X₄ is Aib; K at position 28 is chemically modified through conjugation to the ε-amino of the K side-chain with ([2-(2-amino-ethoxy)-ethoxy]-acetyl)₂-(γ-Glu)₃-CO-(CH₂)₁₆-CO₂H; and the C-terminal amino acid is amidated as a C-terminal primary amide (SEQ ID NO:18); or

   Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K;

   wherein X₁ is Aib; X₂ is αMePhe; X₃ is Aib; X₄ is Aib; K at position 28 is chemically modified through conjugation to the ε-amino of the K side-chain with ([2-(2-amino-ethoxy)-ethoxy]-acetyl)₃-(γ-Glu)₁-CO-(CH₂)₁₆-CO₂H; and the C-terminal amino acid is amidated as a C-terminal primary amide (SEQ ID NO:19); or

   Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K;

   wherein X₁ is Aib; X₂ is αMePhe; X₃ is Aib; X₄ is Aib; K at position 28 is chemically modified through conjugation to the ε-amino of the K side-chain with ([2-(2-amino-ethoxy)-ethoxy]-acetyl)₃-(γ-Glu)₁-CO-(CH₂)₂₀-CO₂H; and the C-terminal amino acid is amidated as a C-terminal primary amide (SEQ ID NO:20).
12. The use according to Embodiments 4, the compound is 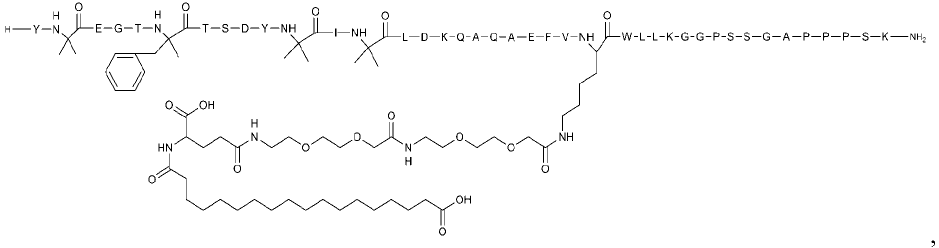 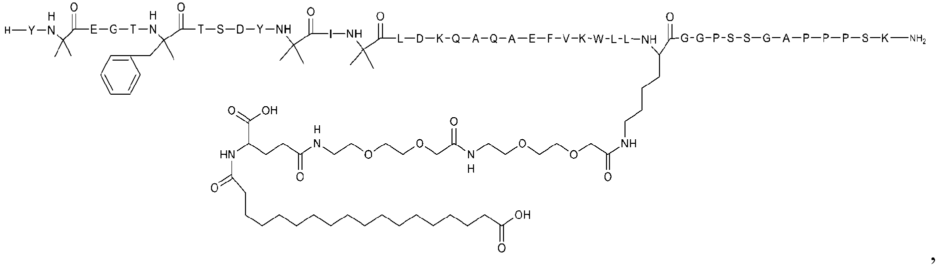 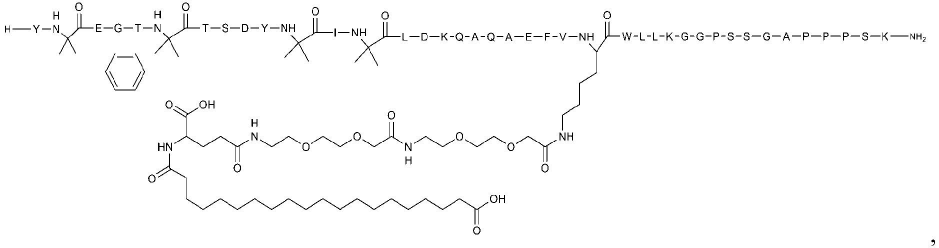 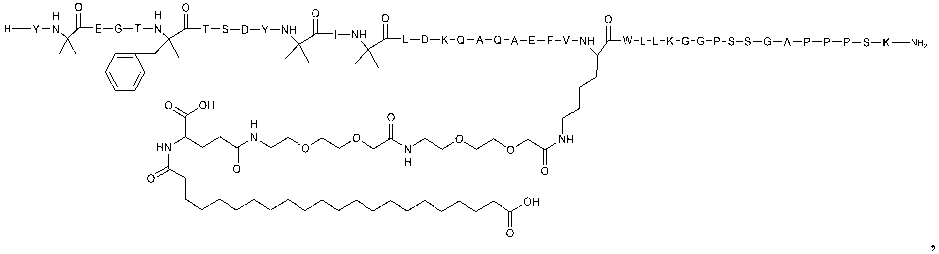 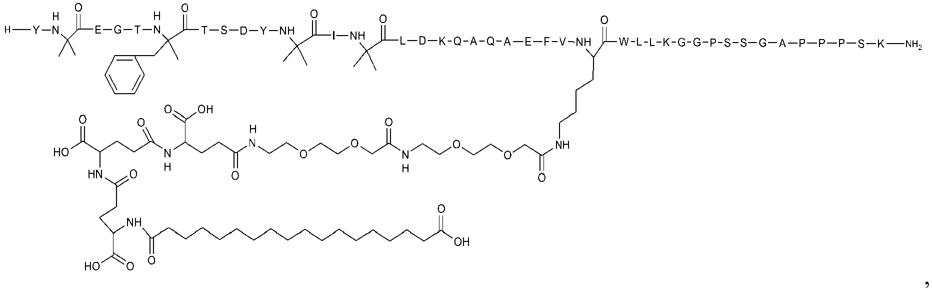 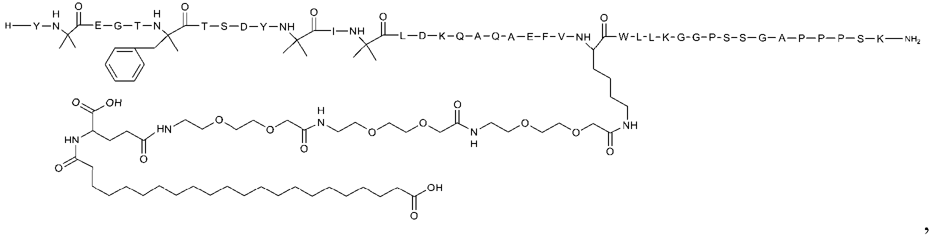 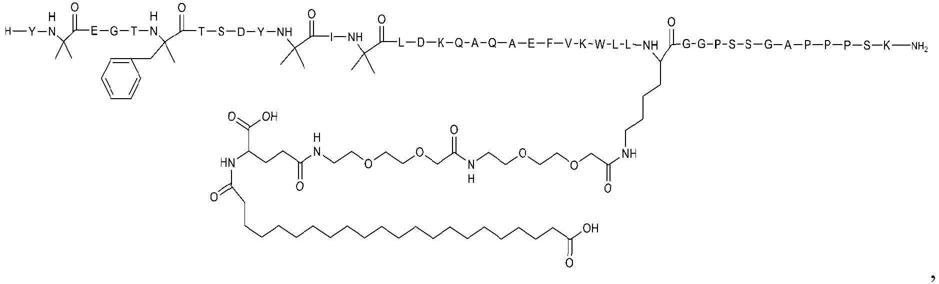 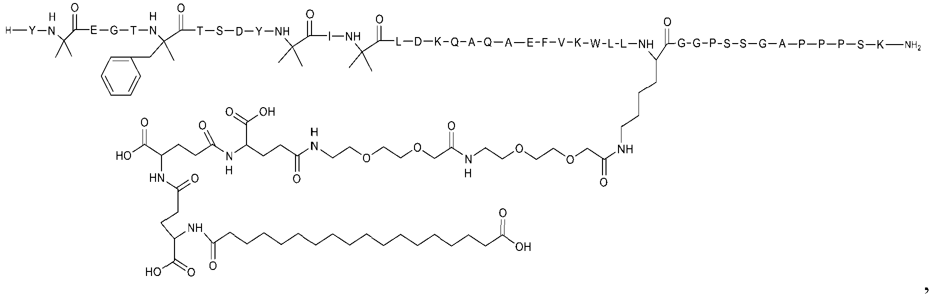 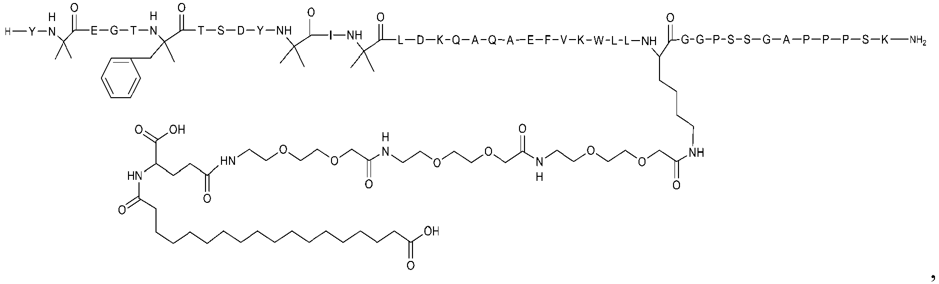 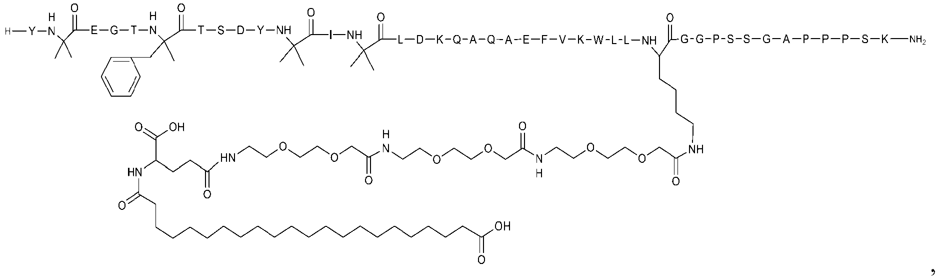 or a pharmaceutically acceptable salt thereof.
13. The use according to any one of Embodiments 1-12, wherein the disease includes hyperglycemia, impaired glucose tolerance, diabetes mellitus (including Type I diabetes mellitus, Type II diabetes mellitus), obesity, hypertension, dyslipidemia, cognitive impairment, atherosclerosis, myocardial infarction, coronary heart disease and other cardiovascular diseases, stroke, inflammatory bowel syndrome, dyspepsia and gastric ulcers.
14. The use according to any one of Embodiments 1-12, wherein the medicament is a medicament for preventing, delaying or treating Type II diabetes mellitus.
15. The use according to any one of Embodiments 1-12, wherein the compound or a pharmaceutically acceptable salt thereof is used for manufacturing a medicament for reducing animal food intake, reducing β-cell apoptosis, increasing β-cell function and β-cell volume and/or restoring glucose sensitivity of β-cells.
16. The use according to any one of Embodiments 1-12, wherein the disease includes metabolic disorders, dyslipidaemia, obesity and/or hepatic steatosis associated with insulin resistance and diabetes mellitus.
17. The use according to any one of Embodiments 1-12, wherein the disease includes osteopenia and bone/joint diseases, such as knee osteoarthritis, hip osteoarthritis, and/or spondylitis deformans.
18. The use according to any one of Embodiments 1-12, wherein the compound or a pharmaceutically acceptable salt thereof is used for chronic weight management as an adjunct to a reduced-calorie diet and increased physical activity in an overweight or obese animal.
19. The use according to any one of Embodiments 1-12, wherein the disease includes: symptomatic obesity, obesity based on simple obesity, obesity-related conditions or diseases, eating disorders, diabetes mellitus (for example, Type I diabetes mellitus, Type II diabetes mellitus, gestational diabetes mellitus, obesity diabetes mellitus), hyperlipidemia (e.g., hypertriglyceridemia, hypercholesterolemia, hyper-LDL-cholesterolemia, low HDL-cholesterolemia, postprandial hyperlipidemia), hypertension, heart failure, complications of diabetes mellitus (for example, neuropathy, nephropathy, retinopathy, diabetic cardiomyopathy, cataract, macroangiopathy, osteopenia, hyperosmolar diabetic coma), infectious disease (for example, respiratory tract infection, urinary tract infection, gastrointestinal infection, superficial soft tissue infection, lower extremity infection), diabetic gangrene, xerostomia, hypacusia, cerebrovascular disorder, peripheral blood circulation disorder, metabolic syndrome (with three or more conditions selected from the group consisting of hypertriglyceridemia (TG), low HDL-cholesterolemia , hypertension, abdominal obesity, and impaired glucose tolerance), and/or sarcopenia.
20. The use according to any one of Embodiments 1-19, wherein the animal is a pet or a companion animal.
21. The use according to Embodiment 20, wherein the pet is selected from dogs, cats, rabbits, pigs, alpacas, horses, sheep and bovines.
22. The use according to any one of Embodiments 1-21, wherein the animal is a dog or a cat.
23. The use according to any one of Embodiments 1-22, wherein the compound or a pharmaceutically acceptable salt thereof is used to treat Type II diabetes mellitus, overweight and/or obesity in cats or dogs.
24. The use according to any one of Embodiments 1-23, wherein the compound or a pharmaceutically acceptable salt thereof is administered in the form of a solution or a suspension.
25. The use according to any one of Embodiments 1-24, wherein the compound or a pharmaceutically acceptable salt thereof is administered by subcutaneous injection;
   preferably, the compound or a pharmaceutically acceptable salt thereof is administered once a week (QW) to cats and dogs.
26. The use according to any one of Embodiments 1-25, wherein the compound or a pharmaceutically acceptable salt thereof is administered to cats or dogs at a dose of about 0.003 to about 2.5 mg/kg, or about 0.003 to about 0.6 mg/kg, for example, about 0.005 to about 0.1mg/kg, about 0.1 to about 0.125mg/kg, about 0.1 to about 0.15mg/kg, about 0.15 to about 0.25mg/kg, about 0.125 to about 0.25mg/kg, about 0.005 to about 2 mg/kg, about 0.005 to about 1.5 mg/kg, about 0.005 to about 1 mg/kg, about 0.005 to about 0.5 mg/kg, about 0.1 to about 2 mg/kg, about 0.1 to about 1.5 mg/kg, about 0.1 to about 1 mg/kg, about 0.1 to about 0.5 mg/kg, about 0.15 to about 2.5 mg/kg, about 0.15 to about 2 mg/kg, about 0.15 to about 1.5 mg/kg, about 0.15 to about 1 mg/kg, about 0.15 to about 0.5 mg/kg, about 0.2 to about 2.5 mg/kg, about 0.2 to about 2 mg/kg, about 0.2 to about 1.5 mg/kg, about 0.2 to about 1 mg/kg, about 0.2 to about 0.5 mg/kg, about 0.25 to about 2.5 mg/kg, about 0.25 to about 2 mg/kg, about 0.25 to about 1.5 mg/kg, about 0.25 to about 1 mg/kg, about 0.25 to about 0.5 mg/kg, about 0.3 to about 2.5 mg/kg, about 0.3 to about 2 mg/kg, about 0.3 to about 1.5 mg/kg, about 0.3 to about 1 mg/kg, about 0.3 to about 0.5 mg/kg, about 0.301 to 2.001 mg/kg, about 0.35 to about 2.5 mg/kg, about 0.35 to about 2 mg/kg, about 0.35 to about 1.5 mg/kg, about 0.35 to about 1 mg/kg, about 0.35 to about 0.5 mg/kg, about 0.4 to about 2.5 mg/kg, about 0.4 to about 2 mg/kg, about 0.4 to about 1.5 mg/kg, about 0.4 to about 1 mg/kg, about 0.4 to about 0.5 mg/kg, about 0.5 to about 2.5 mg/kg, about 0.5 to about 2 mg/kg, about 0.5 to about 1.5 mg/kg, about 0.5 to about 1 mg/kg, about 2.5 mg/kg, about 2.0 mg/kg, about 1.95 mg/kg, about 1.9 mg/kg, about 1.85 mg/kg, about 1.8 mg/kg, about 1.75 mg/kg, about 1.7 mg/kg, about 1.65 mg/kg, about 1.6 mg/kg, about 1.55 mg/kg, about 1.5 mg/kg, about 1.45 mg/kg, about 1.4 mg/kg, about 1.35 mg/kg, about 1.3 mg/kg, about 1.25 mg/kg, about 1.2 mg/kg, about 1.15 mg/kg, about 1.1 mg/kg, about 1.05 mg/kg, about 1.0 mg/kg, about 0.95 mg/kg, about 0.9 mg/kg, about 0.85 mg/kg, about 0.8 mg/kg, about 0.75 mg/kg, about 0.7 mg/kg, about 0.65 mg/kg, about 0.6 mg/kg, about 0.55 mg/kg, about 0.5 mg/kg, about 0.45 mg/kg, about 0.4 mg/kg, about 0.35 mg/kg, about 0.3 mg/kg, about 0.25 mg/kg, about 0.2 mg/kg, about 0.15 mg/kg, or about 0.1 mg/kg.
27. The use according to any one of Embodiments 1-25, wherein the compound or a pharmaceutically acceptable salt thereof is administered to cats or dogs at a dosage regimen of about 0.003 to about 2.5 mg/kg QW, preferably about 0.01 to about 2 mg/kg QW, more preferably about 0.25 to about 2.0 mg/kg QW, and/or, about 0.1 to about 2.0 mg/kg QW, further more preferalby about 0.5 to about 2.0 mg/kg QW, for example, about 0.01 to about 2 mg/kg QW, about 0.01 to about 1.5 mg/kg QW, about 0.01 to about 1 mg/kg QW, about 0.01 to about 0.5 mg/kg QW, about 0.02 to about 2.5 mg/kg QW, about 0.02 to about 2 mg/kg QW, about 0.02 to about 1.5 mg/kg QW, about 0.02 to about 1 mg/kg QW, about 0.02 to about 0.5 mg/kg QW, about 0.021 to about 0.499 mg/kg QW, about 0.03 to about 2.5 mg/kg QW, about 0.03 to about 2 mg/kg QW, about 0.03 to about 1.5 mg/kg QW, about 0.03 to about 1 mg/kg QW, about 0.03 to about 0.5 mg/kg QW, about 0.04 to about 2.5 mg/kg QW, about 0.04 to about 2 mg/kg QW, about 0.04 to about 1.5 mg/kg QW, about 0.04 to about 1 mg/kg QW, about 0.04 to about 0.5 mg/kg QW, about 0.05 to about 2.5 mg/kg QW, about 0.05 to about 2 mg/kg QW, about 0.05 to about 1.5 mg/kg QW, about 0.05 to about 1 mg/kg QW, about 0.05 to about 0.5 mg/kg QW, about 0.06 to about 2.5 mg/kg QW, about 0.06 to about 2 mg/kg QW, about 0.06 to about 1.5 mg/kg QW, about 0.06 to about 1 mg/kg QW, about 0.06 to about 0.5 mg/kg QW, about 0.062 to2.001 mg/kg QW, about 0.07 to about 2.5 mg/kg QW, about 0.07 to about 2 mg/kg QW, about 0.07 to about 1.5 mg/kg QW, about 0.07 to about 1 mg/kg QW, about 0.07 to about 0.5 mg/kg QW, about 0.073 to0.998 mg/kg QW, about 0.08 to about 2.5 mg/kg QW, about 0.08 to about 2 mg/kg QW, about 0.08 to about 1.5 mg/kg QW, about 0.08 to about 1 mg/kg QW, about 0.08 to about 0.5 mg/kg QW, about 0.09 to about 2.5 mg/kg QW, about 0.09 to about 2 mg/kg QW, about 0.09 to about 1.5 mg/kg QW, about 0.09 to about 1 mg/kg QW, about 0.09 to about 0.5 mg/kg QW, about 0.1 to about 2.5 mg/kg QW, about 0.1 to about 2 mg/kg QW, about 0.1 to about 1.5 mg/kg QW, about 0.1 to about 1 mg/kg QW, about 0.1 to about 0.5 mg/kg QW, about 0.15 to about 2.5 mg/kg QW, about 0.15 to about 2 mg/kg QW, about 0.15 to about 1.5 mg/kg QW, about 0.15 to about 1 mg/kg QW, about 0.15 to about 0.5 mg/kg QW, about 0.2 to about 2.5 mg/kg QW, about 0.2 to about 2 mg/kg QW, about 0.2 to about 1.5 mg/kg QW, about 0.2 to about 1 mg/kg QW, about 0.2 to about 0.5 mg/kg QW, about 0.25 to about 2.5 mg/kg QW, about 0.25 to about 2 mg/kg QW, about 0.25 to about 1.5 mg/kg QW, about 0.25 to about 1 mg/kg QW, about 0.25 to about 0.5 mg/kg QW, about 0.3 to about 2.5 mg/kg QW, about 0.3 to about 2 mg/kg QW, about 0.3 to about 1.5 mg/kg QW, about 0.3 to about 1 mg/kg QW, about 0.3 to about 0.5 mg/kg QW, about 0.301 to2.001 mg/kg QW, about 0.35 to about 2.5 mg/kg QW, about 0.35 to about 2 mg/kg QW, about 0.35 to about 1.5 mg/kg QW, about 0.35 to about 1 mg/kg QW, about 0.35 to about 0.5 mg/kg QW, about 0.4 to about 2.5 mg/kg QW, about 0.4 to about 2 mg/kg QW, about 0.4 to about 1.5 mg/kg QW, about 0.4 to about 1 mg/kg QW, about 0.4 to about 0.5 mg/kg QW, about 0.5 to about 2 mg/kg QW, about 0.5 to about 1.5 mg/kg QW, about 0.5 to about 1 mg/kg QW, about 2.5 mg/kg QW, about 2.0 mg/kg QW, about 1.95 mg/kg QW, about 1.9 mg/kg QW, about 1.85 mg/kg QW, about 1.8 mg/kg QW, about 1.75 mg/kg QW, about 1.7 mg/kg QW, about 1.65 mg/kg QW, about 1.6 mg/kg QW, about 1.55 mg/kg QW, about 1.5 mg/kg QW, about 1.45 mg/kg QW, about 1.4 mg/kg QW, about 1.35 mg/kg QW, about 1.3 mg/kg QW, about 1.25 mg/kg QW, about 1.2 mg/kg QW, about 1.15 mg/kg QW, about 1.1 mg/kg QW, about 1.05 mg/kg QW, about 1.0 mg/kg QW, about 0.95 mg/kg QW, about 0.9 mg/kg QW, about 0.85 mg/kg QW, about 0.8 mg/kg QW, about 0.75 mg/kg QW, about 0.7 mg/kg QW, about 0.65 mg/kg QW, about 0.6 mg/kg QW, about 0.55 mg/kg QW, about 0.5 mg/kg QW, about 0.45 mg/kg QW, about 0.4 mg/kg QW, about 0.35 mg/kg QW, about 0.3 mg/kg QW, about 0.25 mg/kg QW, or about 0.2 mg/kg QW.
28. The use according to any one of Embodiments 1-25, wherein the compound or a pharmaceutically acceptable salt thereof is continuously administered to cats or dogs at a frequency of once a week, and the administration period is determined according to the needs of the subject, and the administration period may be 4-8 weeks, or 8-20 weeks, such as 4, 5, 6, 7 or 8 weeks.
29. The use according to any one of Embodiments 1-26, wherein the compound or a pharmaceutically acceptable salt thereof is continuously administered to dogs at a frequency of once a week for 6 weeks, and the dose for each administration may be the same or different.
30. The use according to any one of Embodiments 1-26, wherein the compound or a pharmaceutically acceptable salt thereof is continuously administered to cats at a frequency of once a week for 5 weeks, and the dose for each administration may be the same or different.
31. The use according to Embodiment 29 or 30, wherein the compound or a pharmaceutically acceptable salt thereof is administered to cats or dogs at escalating doses.
32. The use according to any one of Embodiments 28-31, wherein the compound or a pharmaceutically acceptable salt thereof is administered to cats at a dose that
   the first administration and the second administration are identical and at the first dose of about 0.02 to about 0.35 mg/kg QW,
   the third adminisiation is at a second dose of about 0.49 to about 1 mg/kg QW, and the second dose is from about 1.2 to about 50 times of the first dose, preferably from about 10 to about 15 times, such as about 12 times, and
   the fourth administration and the fifth administration are identical and at the third dosge of from about 0.49 to about 2.5 mg/kg QW, wherein the third dose is from about 1.75 to about 2.5 times of the second dose, preferably from about 1.95 to about 2.05 times, more preferably about 2 times.
33. The use according to any one of Embodiments 28-32, wherein the compound or a pharmaceutically acceptable salt thereof is administered to cats at a dose that
   the first dose is about 0.019 mg/kg QW,
   the second dose is about 0.25 mg/kg QW, and
   the third dose is about 0.50 mg/kg QW.
34. The use according to any one of Embodiments 28-32, wherein the compound or a pharmaceutically acceptable salt thereof is administered to cats at a dose that
   the first dose is about 0.075 mg/kg QW,
   the second dose is about 0.50 mg/kg QW, and
   the third dose is about 1.00 mg/kg QW.
35. The use according to any one of Embodiments 28-32, wherein the compound or a pharmaceutically acceptable salt thereof is administered to cats at a dose that
   the first dose is about 0.30 mg/kg QW,
   the second dose is about 1.00 mg/kg QW, and
   the third dose is about 2.00 mg/kg QW.
36. The use according to any one of Embodiments 28-32, wherein the compound or a pharmaceutically acceptable salt thereof is administered to cats at a dosage regimen that
   the dose is about 0.25 to about 2.5 mg/kg; or the dose is about 0.25 to about 2.5 mg per injection;
   the administration frequency is QW; and
   the administration period is 5 weeks.
37. The use according to any one of Embodiments 28-31, wherein the compound or a pharmaceutically acceptable salt thereof is administered to dogs at a dose that
   the first administration and the second administration are identical and at the first dose of about 0.015 to about 0.07 mg/kg QW,
   the third adminisiation is at a second dose of about 0.02 to about 0.14 mg/kg QW, preferably about 0.03 to about 0.1 mg/kg QW, wherein the second dose is from about 1.4 to 2 times of the first dose, preferably from about 1.45 to 1.6 times, more preferably about 1.5 times, and
   the fourth administration to the six administration are identical and at the third dosge of from about 0.49 to about 2.5 mg/kg QW, preferably about 0.495 to about 2.1 mg/kg QW, wherein the third dose is from about 13 to about 25 times of the second dose, preferably from about 16 to 21 times.
38. The use according to Embodiment 36, wherein the compound or a pharmaceutically acceptable salt thereof is administered to dogs at a dose that
   the first dose is about 0.02 mg/kg QW,
   the second dose is about 0.03 mg/kg QW, and
   the third dose is about 0.50 mg/kg QW.
39. The use according to Embodiment 36, wherein the compound or a pharmaceutically acceptable salt thereof is administered to dogs at a dose that
   the first dose is about 0.06 mg/kg QW,
   the second dose is about 0.10 mg/kg QW, and
   the third dose is about 2.00 mg/kg QW.
40. The use according to Embodiment 36, wherein the compound or a pharmaceutically acceptable salt thereof is administered to dogs at a dosage regimen that
   the dose is about 0.1 to about 2.5 mg/kg; or the dose is about 0.1 to about 2.5 mg per injection;
   the administration frequency is QW; and
   the administration period is 6 weeks.
41. The use according to any one of Embodiments 1-40, wherein the compound is selected from SEQ ID NO: 14, SEQ ID NO: 16 and SEQ ID NO: 20, preferably SEQ ID NO: 16.
42. A compound of the following Formula (AI) or a pharmaceutically acceptable salt thereof for preventing or treating a disease in a non-primate and non-rodent animal:

   Y-X₁-E-G-T-X₂-T-S-D-Y-A11-A12-A13-L-D-K-A17-A-Q-A20-E-F-V-K-W-L-L-K-A29-G-P-S- S-G-A-P-P-P-S-K Formula (AI);

   wherein X₁ is Aib; X₂ is αMePhe;
   A11 is Aib or Ala;
   A12 is Ala, Ile, Lys, Phe or Pya(4);
   A13 is Aib, Cha, Leu, αMePhe or αMeTyr;
   A17 is Gln or Ile;
   A20 is Ala or Ser;
   A29 is Gln or Gly,
   1, 2, 3 or 4 Ks, which are selected from the group consisting of K at position 16, K at position 24, K at position 28 and K at position 40, are chemically modified through conjugation to the ε-aminos of the K side-chains with ([2-(2-amino-ethoxy)-ethoxy]-acetyl)ₐ-(γ-Glu)_{b}-CO-(CH₂)_{c}-Z, wherein each a is independently an integer from 0 to 5, each b is independently an integer from 0 to 5, and each c is independently an integer from 10 to 24, wherein Z is independently selected from the group consisting of -CH₃, carboxylic acids or carboxylic acid bioisosteres, phosphonates or sulfonates.
43. The compound or a pharmaceutically acceptable salt thereof according to Embodiment 42, wherein
   A11 is Aib; or/and
   A12 is Ile; or/and
   A13 is Aib; or/and
   A17 is Gln; or/and
   A20 is Ala; or/and
   A29 is Gly; or/and
   a is independently selected from an integer of 1, 2, 3, 4 or 5, b is independently selected from an integer of 1, 2, 3, 4 or 5, and c is independently selected from an integer of 12 to 22; preferably, c is independently selected from 14, 16, 18 or 20.
44. A compound of the following Formula (I) or a pharmaceutically acceptable salt thereof for preventing or treating a disease in a non-primate and non-rodent animal:

   Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K Formula (I);

   wherein X₁ is Aib; X₂ is αMePhe; X₃ is Aib; X₄ is Aib; 1, 2, 3 or 4 Ks, which are selected from the group consisting of K at position 16, K at position 24, K at position 28 and K at position 40, are chemically modified through conjugation to the ε-aminos of the K side-chains with ([2-(2-amino-ethoxy)-ethoxy]-acetyl)ₐ-(γ-Glu)_{b}-CO-(CH₂)_{c}-Z, wherein each a is independently an integer from 0 to 5, each b is independently an integer from 0 to 5, and each c is independently an integer from 10 to 24, wherein Z is independently selected from the group consisting of -CH₃, carboxylic acids or carboxylic acid bioisosteres, phosphonates or sulfonates; and the C-terminal amino acid is amidated as a C-terminal primary amide.
45. The compound or a pharmaceutically acceptable salt thereof according to Embodiment 42, wherein the compound or a pharmaceutically acceptable salt thereof is:

   Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K Formula (I),

   or a pharmaceutically acceptable salt thereof, wherein X₁ is Aib; X₂ is αMePhe; X₃ is Aib; X₄ is Aib; 1 or 2 Ks, which are selected from the group consisting of K at position 16, K at position 24, K at position 28 and K at position 40, are chemically modified through conjugation to the ε-aminos of the K side-chains with ([2-(2-amino-ethoxy)-ethoxy]-acetyl)ₐ-(γ-Glu)_{b}-CO-(CH₂)_{c}-Z, wherein Z is independently selected from the group consisting of -CH₃, carboxylic acids or carboxylic acid bioisosteres, phosphonates or sulfonates, preferably -CO₂H, each a is independently an integer from 0 to 5, each b is independently an integer from 0 to 5, and each c is independently an integer from 10 to 22; and the C-terminal amino acid is amidated as a C-terminal primary amide.
46. The compound or a pharmaceutically acceptable salt thereof according to Embodiment 42, wherein
   K at position 16 is chemically modified through conjugation to the ε-amino of the K side-chain with ([2-(2-amino-ethoxy)-ethoxy]-acetyl)ₐ-(γ-Glu)_{b}-CO-(CH₂)_{c}-CO₂H; or
   K at position 24 is chemically modified through conjugation to the ε-amino of the K side-chain with ([2-(2-amino-ethoxy)-ethoxy]-acetyl)ₐ-(γ-Glu)_{b}-CO-(CH₂)_{c}-CO₂H; or
   K at position 28 is chemically modified through conjugation to the ε-amino of the K side-chain with ([2-(2-amino-ethoxy)-ethoxy]-acetyl)ₐ-(γ-Glu)_{b}-CO-(CH₂)_{c}-CO₂H; or
   K at position 40 is chemically modified through conjugation to the ε-amino of the K side-chain with ([2-(2-amino-ethoxy)-ethoxy]-acetyl)ₐ-(γ-Glu)_{b}-CO-(CH₂)_{c}-CO₂H.
47. The compound or a pharmaceutically acceptable salt thereof according to Embodiment 42, wherein
   K at position 24 and K at position 28 are chemically modified with two modifying chains through conjugation to the ε-aminos of the K side-chains with([2-(2-amino-ethoxy)-ethoxy]-acetyl)ₐ-(γ-Glu)_{b}-CO-(CH₂)_{c}-CO₂H; or
   K at position 16 and K at position 24 are chemically modified with two modifying chains through conjugation to the ε-aminos of the K side-chains with ([2-(2-amino-ethoxy)-ethoxy]-acetyl)ₐ-(γ-Glu)_{b}-CO-(CH₂)_{c}-CO₂H; or
   K at position 16 and K at position 28 are chemically modified with two modifying chains through conjugation to the ε-aminos of the K side-chains with ([2-(2-amino-ethoxy)-ethoxy]-acetyl)ₐ-(γ-Glu)_{b}-CO-(CH₂)_{c}-CO₂H; or
   K at position 16 and K at position 40 are chemically modified with two modifying chains through conjugation to the ε-aminos of the K side-chains with ([2-(2-amino-ethoxy)-ethoxy]-acetyl)ₐ-(γ-Glu)_{b}-CO-(CH₂)_{c}-CO₂H; or
   K at position 24 and K at position 40 are chemically modified with two modifying chains through conjugation to the ε-aminos of the K side-chains with ([2-(2-amino-ethoxy)-ethoxy]-acetyl)ₐ-(γ-Glu)_{b}-CO-(CH₂)_{c}-CO₂H; or
   K at position 28 and K at position 40 are chemically modified with two modifying chains through conjugation to the ε-aminos of the K side-chains with ([2-(2-amino-ethoxy)-ethoxy]-acetyl)ₐ-(γ-Glu)_{b}-CO-(CH₂)_{c}-CO₂H.
48. The compound or a pharmaceutically acceptable salt thereof according to any one of Embodiments 42-45, wherein
   a is independently selected from an integer of 1, 2, 3, 4 or 5, b is independently selected from an integer of 1, 2, 3, 4 or 5, and c is independently selected from an integer of 12 to 22; preferably, c is independently selected from 14, 16, 18 or 20; and/or
   Z is -CO₂H.
49. The compound or a pharmaceutically acceptable salt thereof according to Embodiment 42, wherein the compound or a pharmaceutically acceptable salt thereof is

   Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K Formula (I);

   wherein X₁ is Aib; X₂ is αMePhe; X₃ is Aib; X₄ is Aib; K at position 24 is chemically modified through conjugation to the ε-amino of the K side-chain with ([2-(2-amino-ethoxy)-ethoxy]-acetyl)ₐ-(γ-Glu)_{b}-CO-(CH₂)_{c}-CO₂H, wherein a is independently selected from an integer from 0 to 5, b is independently selected from an integer from 0 to 5, and c is independently selected from an integer from 10 to 24; and the C-terminal amino acid is amidated as a C-terminal primary amide;
   preferably, wherein, X₁ is Aib; X₂ is αMePhe; X₃ is Aib; X₄ is Aib; K at position 24 is chemically modified through conjugation to the ε-amino of the K side-chain with ([2-(2-amino-ethoxy)-ethoxy]-acetyl)ₐ-(γ-Glu)_{b}-CO-(CH₂)_{c}-CO₂H, wherein each a is independently selected from an integer of 2 and 3, each b is independently 1, and each c is independently selected from an integer of 16, 18 and 20, wherein Z is independently selected from carboxylic acids.
50. The compound or a pharmaceutically acceptable salt thereof according to Embodiment 42, the compound is

   Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K Formula (I);

   wherein X₁ is Aib; X₂ is αMePhe; X₃ is Aib; X₄ is Aib; K at position 28 is chemically modified through conjugation to the ε-amino of the K side-chain with ([2-(2-amino-ethoxy)-ethoxy]-acetyl)ₐ-(γ-Glu)_{b}-CO-(CH₂)_{c}-CO₂H, wherein a is independently selected from an integer from 0 to 5, b is independently selected from an integer from 0 to 5, and c is independently selected from an integer from 10 to 24; and the C-terminal amino acid is amidated as a C-terminal primary amide;
   preferably, wherein, X₁ is Aib; X₂ is αMePhe; X₃ is Aib; X₄ is Aib; K at position 28 is chemically modified through conjugation to the ε-amino of the K side-chain with ([2-(2-amino-ethoxy)-ethoxy]-acetyl)ₐ-(γ-Glu)_{b}-CO-(CH₂)_{c}-CO₂H, wherein each a is independently selected from an integer of 2 and 3, each b is independently selected from an integer of 1 and 3, and each c is independently selected from an integer of 16 and 20, wherein Z is independently selected from carboxylic acids.
51. The compound or a pharmaceutically acceptable salt thereof according to any one of Embodiments 46-47, wherein
   a is independently selected from an integer of 1, 2, 3, 4 or 5, b is independently selected from an integer of 1, 2, 3, 4 or 5, and c is independently selected from an integer of 12 to 22; preferably, c is independently selected from 14, 16, 18 or 20.
52. The compound or a pharmaceutically acceptable salt thereof according to Embodiments 42, the compound is

   Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K;

   wherein X₁ is Aib; X₂ is αMePhe; X₃ is Aib; X₄ is Aib; K at position 24 is chemically modified through conjugation to the ε-amino of the K side-chain with ([2-(2-amino-ethoxy)-ethoxy]-acetyl)₂-(γ-Glu)₁-CO-(CH₂)₁₆-CO₂H; and the C-terminal amino acid is amidated as a C-terminal primary amide (SEQ ID NO:7); or

   Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K;

   wherein X₁ is Aib; X₂ is αMePhe; X₃ is Aib; X₄ is Aib; K at position 24 is chemically modified through conjugation to the ε-amino of the K side-chain with ([2-(2-amino-ethoxy)-ethoxy]-acetyl)₂-(γ-Glu)₁-CO-(CH₂)₁₈-CO₂H; and the C-terminal amino acid is amidated as a C-terminal primary amide (SEQ ID NO:13); or

   Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K;

   wherein X₁ is Aib; X₂ is αMePhe; X₃ is Aib; X₄ is Aib; K at position 24 is chemically modified through conjugation to the ε-amino of the K side-chain with ([2-(2-amino-ethoxy)-ethoxy]-acetyl)₂-(γ-Glu)₁-CO-(CH₂)₂₀-CO₂H; and the C-terminal amino acid is amidated as a C-terminal primary amide (SEQ ID NO:14); or

   Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K;

   wherein X₁ is Aib; X₂ is αMePhe; X₃ is Aib; X₄ is Aib; K at position 24 is chemically modified through conjugation to the ε-amino of the K side-chain with ([2-(2-amino-ethoxy)-ethoxy]-acetyl)₂-(γ-Glu)₃-CO-(CH₂)₁₆-CO₂H; and the C-terminal amino acid is amidated as a C-terminal primary amide (SEQ ID NO:15); or

   Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K;

   wherein X₁ is Aib; X₂ is αMePhe; X₃ is Aib; X₄ is Aib; K at position 24 is chemically modified through conjugation to the ε-amino of the K side-chain with ([2-(2-amino-ethoxy)-ethoxy]-acetyl)₃-(γ-Glu)₁-CO-(CH₂)₂₀-CO₂H; and the C-terminal amino acid is amidated as a C-terminal primary amide (SEQ ID NO:16); or

   Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K;

   wherein X₁ is Aib; X₂ is αMePhe; X₃ is Aib; X₄ is Aib; K at position 28 is chemically modified through conjugation to the ε-amino of the K side-chain with ([2-(2-amino-ethoxy)-ethoxy]-acetyl)₂-(γ-Glu)₁-CO-(CH₂)₁₆-CO₂H; and the C-terminal amino acid is amidated as a C-terminal primary amide (SEQ ID NO:8); or

   Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K;

   wherein X₁ is Aib; X₂ is αMePhe; X₃ is Aib; X₄ is Aib; K at position 28 is chemically modified through conjugation to the ε-amino of the K side-chain with ([2-(2-amino-ethoxy)-ethoxy]-acetyl)₂-(γ-Glu)₁-CO-(CH₂)₂₀-CO₂H; and the C-terminal amino acid is amidated as a C-terminal primary amide (SEQ ID NO:17); or

   Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K;

   wherein X₁ is Aib; X₂ is αMePhe; X₃ is Aib; X₄ is Aib; K at position 28 is chemically modified through conjugation to the ε-amino of the K side-chain with ([2-(2-amino-ethoxy)-ethoxy]-acetyl)₂-(γ-Glu)₃-CO-(CH₂)₁₆-CO₂H; and the C-terminal amino acid is amidated as a C-terminal primary amide (SEQ ID NO:18); or

   Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K;

   wherein X₁ is Aib; X₂ is αMePhe; X₃ is Aib; X₄ is Aib; K at position 28 is chemically modified through conjugation to the ε-amino of the K side-chain with ([2-(2-amino-ethoxy)-ethoxy]-acetyl)₃-(γ-Glu)₁-CO-(CH₂)₁₆-CO₂H; and the C-terminal amino acid is amidated as a C-terminal primary amide (SEQ ID NO:19); or

   Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K;

   wherein X₁ is Aib; X₂ is αMePhe; X₃ is Aib; X₄ is Aib; K at position 28 is chemically modified through conjugation to the ε-amino of the K side-chain with ([2-(2-amino-ethoxy)-ethoxy]-acetyl)₃-(γ-Glu)₁-CO-(CH₂)₂₀-CO₂H; and the C-terminal amino acid is amidated as a C-terminal primary amide (SEQ ID NO:20).
53. The compound or a pharmaceutically acceptable salt thereof according to Embodiment 42, the compound is 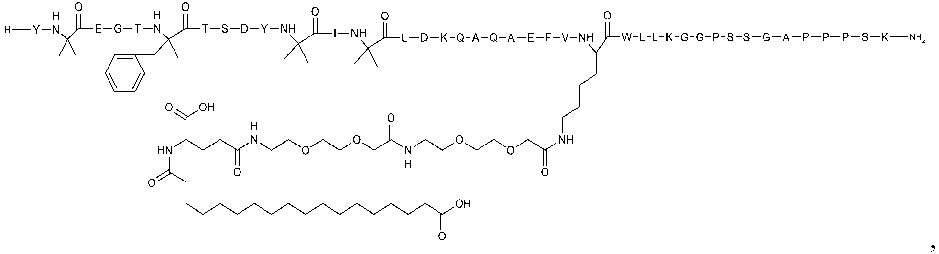 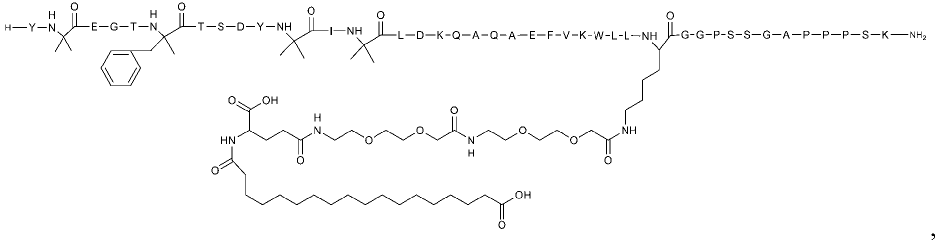 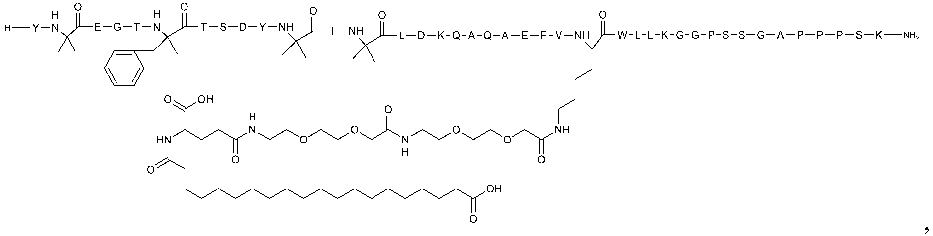 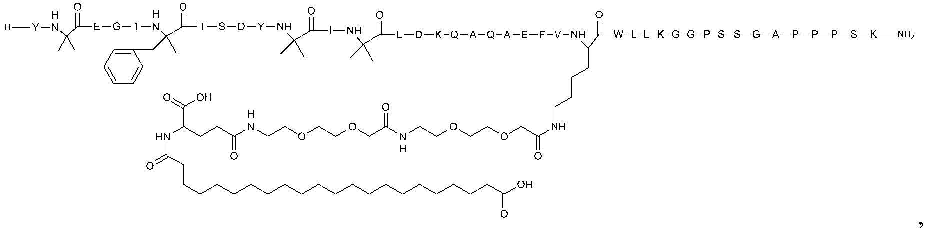 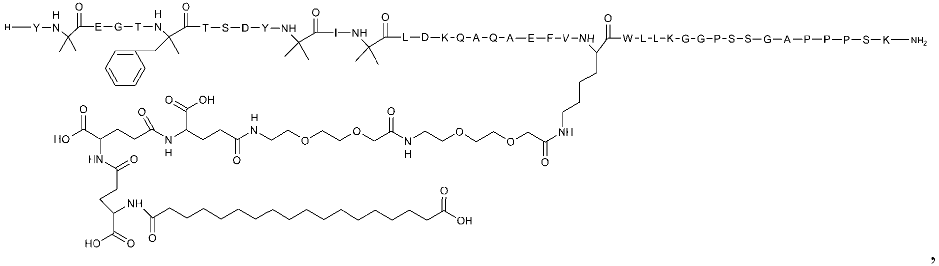 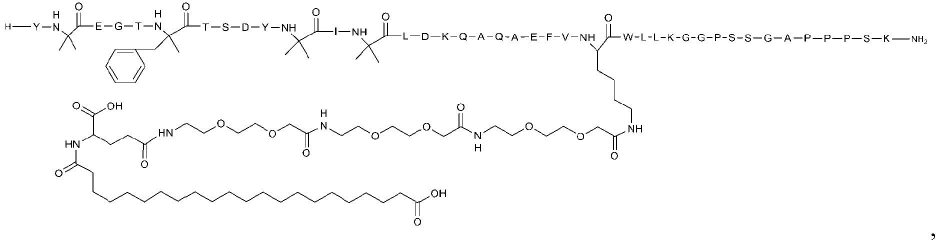 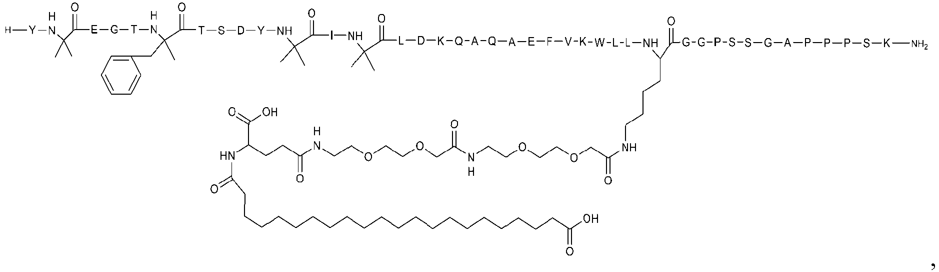 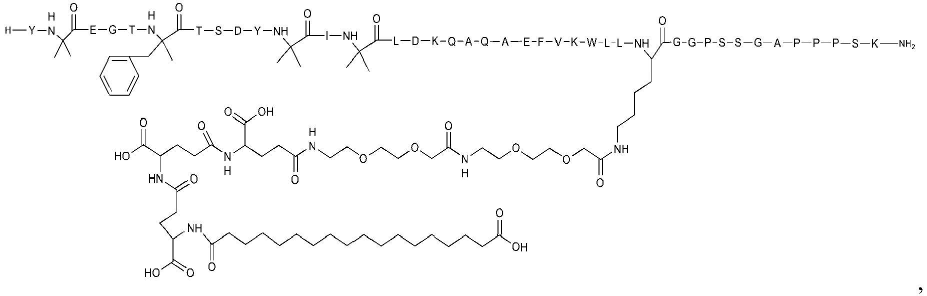 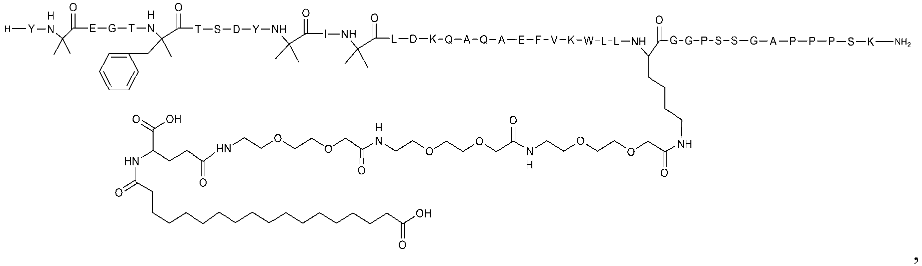 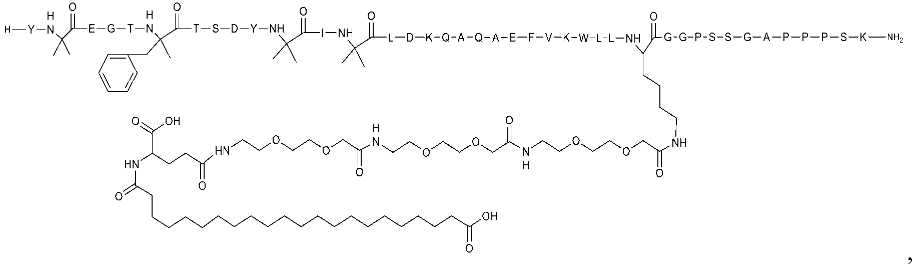 or a pharmaceutically acceptable salt thereof.
54. The compound or a pharmaceutically acceptable salt thereof according to any one of Embodiments 42-53, wherein the disease includes animal hyperglycemia, impaired glucose tolerance, diabetes mellitus (including Type I diabetes mellitus, Type II diabetes mellitus), obesity, hypertension, dyslipidemia, cognitive impairment, atherosclerosis, myocardial infarction, coronary heart disease and other cardiovascular diseases, stroke, inflammatory bowel syndrome, dyspepsia and gastric ulcers.
55. The compound or a pharmaceutically acceptable salt thereof according to any one of Embodiments 42-53, wherein the medicament is a medicament for preventing, delaying or treating Type II diabetes mellitus.
56. The compound or a pharmaceutically acceptable salt thereof according to any one of Embodiments 42-53, wherein the compound or a pharmaceutically acceptable salt thereof is used for reducing animal food intake, reducing β-cell apoptosis, increasing β-cell function and β-cell volume and/or restoring glucose sensitivity of β-cells.
57. The compound or a pharmaceutically acceptable salt thereof according to any one of Embodiments 42-53, wherein the disease includes metabolic disorders, dyslipidaemia, including obesity and/or hepatic steatosis associated with insulin resistance and diabetes mellitus.
58. The compound or a pharmaceutically acceptable salt thereof according to any one of Embodiments 42-53, wherein the disease includes osteopenia and bone/joint diseases, such as knee osteoarthritis, hip osteoarthritis, and/or spondylitis deformans.
59. The compound or a pharmaceutically acceptable salt thereof according to any one of Embodiments 42-53, wherein the compound or a pharmaceutically acceptable salt thereof is used for chronic weight management as an adjunct to a reduced-calorie diet and increased physical activity in an overweight or obese animal.
60. The compound or a pharmaceutically acceptable salt thereof according to any one of Embodiments 42-53, wherein the disease includes: symptomatic obesity, obesity based on simple obesity, obesity-related conditions or diseases, eating disorders, diabetes mellitus (for example, Type I diabetes mellitus, Type II diabetes mellitus, gestational diabetes mellitus, obesity diabetes mellitus), hyperlipidemia (e.g., hypertriglyceridemia, hypercholesterolemia, hyper-LDL-cholesterolemia, low HDL-cholesterolemia, postprandial hyperlipidemia), hypertension, heart failure, complications of diabetes mellitus (for example, neuropathy, nephropathy, retinopathy, diabetic cardiomyopathy, cataract, macroangiopathy, osteopenia, hyperosmolar diabetic coma), infectious disease (for example, respiratory tract infection, urinary tract infection, gastrointestinal infection, superficial soft tissue infection, lower extremity infection), diabetic gangrene, xerostomia, hypacusia, cerebrovascular disorder, peripheral blood circulation disorder, metabolic syndrome (with three or more conditions selected from the group consisting of hypertriglyceridemia (TG), low HDL-cholesterolemia , hypertension, abdominal obesity, and impaired glucose tolerance), and/or sarcopenia.
61. The compound or a pharmaceutically acceptable salt thereof according to any one of Embodiments 42-60, wherein the animal is a pet or a companion animal.
62. The compound or a pharmaceutically acceptable salt thereof according to Embodiment 61, wherein the pet is selected from the group consisting of dogs, cats, rabbits, pigs, alpacas, horses, sheep and bovines.
63. The compound or a pharmaceutically acceptable salt thereof according to any one of Embodiments 42-62, wherein the animal is a dog or a cat.
64. The compound or a pharmaceutically acceptable salt thereof according to any one of Embodiments 42-63, wherein the compound or a pharmaceutically acceptable salt thereof is used to treat Type II diabetes mellitus, overweight or and/obesity in cats or dogs.
65. The compound or a pharmaceutically acceptable salt thereof according to any one of Embodiments 42-64, wherein the compound or a pharmaceutically acceptable salt thereof is administered in the form of a solution or a suspension.
66. The compound or a pharmaceutically acceptable salt thereof according to any one of Embodiments 42-65, wherein the compound or a pharmaceutically acceptable salt thereof is administered by subcutaneous injection;
   preferably, the compound or a pharmaceutically acceptable salt thereof is administered once a week to cats and dogs.
67. The compound or a pharmaceutically acceptable salt thereof according to any one of Embodiments 42-66, wherein the compound or a pharmaceutically acceptable salt thereof is administered to cats or dogs at a dose of about 0.003 to about 2.5 mg/kg, or about 0.003 to about 0.6 mg/kg, for example, about 0.005 to about 0.1mg/kg, about 0.1 to about 0.125mg/kg, about 0.1 to about 0.15mg/kg, about 0.15 to about 0.25mg/kg, about 0.125 to about 0.25mg/kg, about 0.005 to about 2 mg/kg, about 0.005 to about 1.5 mg/kg, about 0.005 to about 1 mg/kg, about 0.005 to about 0.5 mg/kg, about 0.1 to about 2 mg/kg, about 0.1 to about 1.5 mg/kg, about 0.1 to about 1 mg/kg, about 0.1 to about 0.5 mg/kg, about 0.15 to about 2.5 mg/kg, about 0.15 to about 2 mg/kg, about 0.15 to about 1.5 mg/kg, about 0.15 to about 1 mg/kg, about 0.15 to about 0.5 mg/kg, about 0.2 to about 2.5 mg/kg, about 0.2 to about 2 mg/kg, about 0.2 to about 1.5 mg/kg, about 0.2 to about 1 mg/kg, about 0.2 to about 0.5 mg/kg, about 0.25 to about 2.5 mg/kg, about 0.25 to about 2 mg/kg, about 0.25 to about 1.5 mg/kg, about 0.25 to about 1 mg/kg, about 0.25 to about 0.5 mg/kg, about 0.3 to about 2.5 mg/kg, about 0.3 to about 2 mg/kg, about 0.3 to about 1.5 mg/kg, about 0.3 to about 1 mg/kg, about 0.3 to about 0.5 mg/kg, about 0.301 to 2.001 mg/kg, about 0.35 to about 2.5 mg/kg, about 0.35 to about 2 mg/kg, about 0.35 to about 1.5 mg/kg, about 0.35 to about 1 mg/kg, about 0.35 to about 0.5 mg/kg, about 0.4 to about 2.5 mg/kg, about 0.4 to about 2 mg/kg, about 0.4 to about 1.5 mg/kg, about 0.4 to about 1 mg/kg, about 0.4 to about 0.5 mg/kg, about 0.5 to about 2.5 mg/kg, about 0.5 to about 2 mg/kg, about 0.5 to about 1.5 mg/kg, about 0.5 to about 1 mg/kg, about 2.5 mg/kg, about 2.0 mg/kg, about 1.95 mg/kg, about 1.9 mg/kg, about 1.85 mg/kg, about 1.8 mg/kg, about 1.75 mg/kg, about 1.7 mg/kg, about 1.65 mg/kg, about 1.6 mg/kg, about 1.55 mg/kg, about 1.5 mg/kg, about 1.45 mg/kg, about 1.4 mg/kg, about 1.35 mg/kg, about 1.3 mg/kg, about 1.25 mg/kg, about 1.2 mg/kg, about 1.15 mg/kg, about 1.1 mg/kg, about 1.05 mg/kg, about 1.0 mg/kg, about 0.95 mg/kg, about 0.9 mg/kg, about 0.85 mg/kg, about 0.8 mg/kg, about 0.75 mg/kg, about 0.7 mg/kg, about 0.65 mg/kg, about 0.6 mg/kg, about 0.55 mg/kg, about 0.5 mg/kg, about 0.45 mg/kg, about 0.4 mg/kg, about 0.35 mg/kg, about 0.3 mg/kg, about 0.25 mg/kg, about 0.2 mg/kg, about 0.15 mg/kg, or about 0.1 mg/kg.
68. The compound or a pharmaceutically acceptable salt thereof according to any one of Embodiments 42-66, wherein the compound or a pharmaceutically acceptable salt thereof is administered to cats or dogs at a dosage regimen of about 0.003 to about 2.5 mg/kg QW, preferably about 0.01 to about 2 mg/kg QW, more preferably about 0.25 to about 2.0 mg/kg QW, and/or, about 0.1 to about 2.0 mg/kg QW, further more preferalby about 0.5 to about 2.0 mg/kg QW, for example, about 0.01 to about 2 mg/kg QW, about 0.01 to about 1.5 mg/kg QW, about 0.01 to about 1 mg/kg QW, about 0.01 to about 0.5 mg/kg QW, about 0.02 to about 2.5 mg/kg QW, about 0.02 to about 2 mg/kg QW, about 0.02 to about 1.5 mg/kg QW, about 0.02 to about 1 mg/kg QW, about 0.02 to about 0.5 mg/kg QW, about 0.021 to about 0.499 mg/kg QW, about 0.03 to about 2.5 mg/kg QW, about 0.03 to about 2 mg/kg QW, about 0.03 to about 1.5 mg/kg QW, about 0.03 to about 1 mg/kg QW, about 0.03 to about 0.5 mg/kg QW, about 0.04 to about 2.5 mg/kg QW, about 0.04 to about 2 mg/kg QW, about 0.04 to about 1.5 mg/kg QW, about 0.04 to about 1 mg/kg QW, about 0.04 to about 0.5 mg/kg QW, about 0.05 to about 2.5 mg/kg QW, about 0.05 to about 2 mg/kg QW, about 0.05 to about 1.5 mg/kg QW, about 0.05 to about 1 mg/kg QW, about 0.05 to about 0.5 mg/kg QW, about 0.06 to about 2.5 mg/kg QW, about 0.06 to about 2 mg/kg QW, about 0.06 to about 1.5 mg/kg QW, about 0.06 to about 1 mg/kg QW, about 0.06 to about 0.5 mg/kg QW, about 0.062 to2.001 mg/kg QW, about 0.07 to about 2.5 mg/kg QW, about 0.07 to about 2 mg/kg QW, about 0.07 to about 1.5 mg/kg QW, about 0.07 to about 1 mg/kg QW, about 0.07 to about 0.5 mg/kg QW, about 0.073 to0.998 mg/kg QW, about 0.08 to about 2.5 mg/kg QW, about 0.08 to about 2 mg/kg QW, about 0.08 to about 1.5 mg/kg QW, about 0.08 to about 1 mg/kg QW, about 0.08 to about 0.5 mg/kg QW, about 0.09 to about 2.5 mg/kg QW, about 0.09 to about 2 mg/kg QW, about 0.09 to about 1.5 mg/kg QW, about 0.09 to about 1 mg/kg QW, about 0.09 to about 0.5 mg/kg QW, about 0.1 to about 2.5 mg/kg QW, about 0.1 to about 2 mg/kg QW, about 0.1 to about 1.5 mg/kg QW, about 0.1 to about 1 mg/kg QW, about 0.1 to about 0.5 mg/kg QW, about 0.15 to about 2.5 mg/kg QW, about 0.15 to about 2 mg/kg QW, about 0.15 to about 1.5 mg/kg QW, about 0.15 to about 1 mg/kg QW, about 0.15 to about 0.5 mg/kg QW, about 0.2 to about 2.5 mg/kg QW, about 0.2 to about 2 mg/kg QW, about 0.2 to about 1.5 mg/kg QW, about 0.2 to about 1 mg/kg QW, about 0.2 to about 0.5 mg/kg QW, about 0.25 to about 2.5 mg/kg QW, about 0.25 to about 2 mg/kg QW, about 0.25 to about 1.5 mg/kg QW, about 0.25 to about 1 mg/kg QW, about 0.25 to about 0.5 mg/kg QW, about 0.3 to about 2.5 mg/kg QW, about 0.3 to about 2 mg/kg QW, about 0.3 to about 1.5 mg/kg QW, about 0.3 to about 1 mg/kg QW, about 0.3 to about 0.5 mg/kg QW, about 0.301 to2.001 mg/kg QW, about 0.35 to about 2.5 mg/kg QW, about 0.35 to about 2 mg/kg QW, about 0.35 to about 1.5 mg/kg QW, about 0.35 to about 1 mg/kg QW, about 0.35 to about 0.5 mg/kg QW, about 0.4 to about 2.5 mg/kg QW, about 0.4 to about 2 mg/kg QW, about 0.4 to about 1.5 mg/kg QW, about 0.4 to about 1 mg/kg QW, about 0.4 to about 0.5 mg/kg QW, about 0.5 to about 2 mg/kg QW, about 0.5 to about 1.5 mg/kg QW, about 0.5 to about 1 mg/kg QW, about 2.5 mg/kg QW, about 2.0 mg/kg QW, about 1.95 mg/kg QW, about 1.9 mg/kg QW, about 1.85 mg/kg QW, about 1.8 mg/kg QW, about 1.75 mg/kg QW, about 1.7 mg/kg QW, about 1.65 mg/kg QW, about 1.6 mg/kg QW, about 1.55 mg/kg QW, about 1.5 mg/kg QW, about 1.45 mg/kg QW, about 1.4 mg/kg QW, about 1.35 mg/kg QW, about 1.3 mg/kg QW, about 1.25 mg/kg QW, about 1.2 mg/kg QW, about 1.15 mg/kg QW, about 1.1 mg/kg QW, about 1.05 mg/kg QW, about 1.0 mg/kg QW, about 0.95 mg/kg QW, about 0.9 mg/kg QW, about 0.85 mg/kg QW, about 0.8 mg/kg QW, about 0.75 mg/kg QW, about 0.7 mg/kg QW, about 0.65 mg/kg QW, about 0.6 mg/kg QW, about 0.55 mg/kg QW, about 0.5 mg/kg QW, about 0.45 mg/kg QW, about 0.4 mg/kg QW, about 0.35 mg/kg QW, about 0.3 mg/kg QW, about 0.25 mg/kg QW, or about 0.2 mg/kg QW.
69. The compound or a pharmaceutically acceptable salt thereof according to any one of Embodiments 42-68, wherein the compound or a pharmaceutically acceptable salt thereof is continuously administered to cats or dogs at a frequency of once a week, and the administration period is determined according to the needs of the subject, and the administration period may be 4-8 weeks, or 8-20 weeks, such as 4, 5, 6, 7 or 8 weeks.
70. The compound or a pharmaceutically acceptable salt thereof according to any one of Embodiments 42-68, wherein the compound or a pharmaceutically acceptable salt thereof is continuously administered to dogs at a frequency of once a week for 6 weeks, and the dose for each administration may be the same or different.
71. The compound or a pharmaceutically acceptable salt thereof according to any one of Embodiments 42-68, wherein the compound or a pharmaceutically acceptable salt thereof is continuously administered to cats at a frequency of once a week for 5 weeks, and the dose for each administration may be the same or different.
72. The compound or a pharmaceutically acceptable salt thereof according to Embodiment 68 or 69, wherein the compound or a pharmaceutically acceptable salt thereof is administered to cats or dogs at escalating doses.
73. The compound or a pharmaceutically acceptable salt thereof according to any one of Embodiments 68-72, wherein the compound or a pharmaceutically acceptable salt thereof is administered to cats at a dose that
   the first administration and the second administration are identical and at the first dose of about 0.02 to about 0.35 mg/kg QW,
   the third adminisiation is at a second dose of about 0.49 to about 1 mg/kg QW, and the second dose is from about 1.2 to about 50 times of the first dose, preferably from about 10 to about 15 times, such as about 12 times, and
   the fourth administration and the fifth administration are identical and at the third dosge of from about 0.49 to about 2.5 mg/kg QW, wherein the third dose is from about 1.75 to about 2.5 times of the second dose, preferably from about 1.95 to about 2.05 times, more preferably about 2 times.
74. The compound or a pharmaceutically acceptable salt thereof according to any one of Embodiments 68-73, wherein the compound or a pharmaceutically acceptable salt thereof is administered to cats at a dose that
   the first dose is about 0.019 mg/kg QW,
   the second dose is about 0.25 mg/kg QW, and
   the third dose is about 0.50 mg/kg QW.
75. The compound or a pharmaceutically acceptable salt thereof according to any one of Embodiments 68-73, wherein the compound or a pharmaceutically acceptable salt thereof is administered to cats at a dose that
   the first dose is about 0.075 mg/kg QW,
   the second dose is about 0.50 mg/kg QW, and
   the third dose is about 1.00 mg/kg QW.
76. The compound or a pharmaceutically acceptable salt thereof according to any one of Embodiments 68-73, wherein the compound or a pharmaceutically acceptable salt thereof is administered to cats at a dose that
   the first dose is about 0.30 mg/kg QW,
   the second dose is about 1.00 mg/kg QW, and
   the third dose is about 2.00 mg/kg QW.
77. The compound or a pharmaceutically acceptable salt thereof according to any one of Embodiments 65-70, wherein the compound or a pharmaceutically acceptable salt thereof is administered to cats at a dosage regimen that
   the dose is about 0.25 to about 2.5 mg/kg; or the dose is about 0.25 to about 2.5 mg per injection;
   the administration frequency is QW; and
   the administration period is 5 weeks.
78. The compound or a pharmaceutically acceptable salt thereof according to any one of Embodiments 68-72, wherein the compound or a pharmaceutically acceptable salt thereof is administered to dogs at a dose that
   the first administration and the second administration are identical and at the first dose of about 0.015 to about 0.07 mg/kg QW,
   the third adminisiation is at a second dose of about 0.02 to about 0.14 mg/kg QW, preferably about 0.03 to about 0.1 mg/kg QW, wherein the second dose is from about 1.4 to 2 times of the first dose, preferably from about 1.45 to 1.6 times, more preferably about 1.5 times, and
   the fourth administration to the six administration are identical and at the third dosge of from about 0.49 to about 2.5 mg/kg QW, preferably about 0.495 to about 2.1 mg/kg QW, wherein the third dose is from about 13 to about 25 times of the second dose, preferably from about 16 to 21 times.
79. The compound or a pharmaceutically acceptable salt thereof according to Embodiment 78, wherein the compound or a pharmaceutically acceptable salt thereof is administered to dogs at a dose that
   the first dose is about 0.02 mg/kg QW,
   the second dose is about 0.03 mg/kg QW, and
   the third dose is about 0.50 mg/kg QW.
80. The compound or a pharmaceutically acceptable salt thereof according to Embodiment 78, wherein the compound or a pharmaceutically acceptable salt thereof is administered to dogs at a dose that
   the first dose is about 0.06 mg/kg QW,
   the second dose is about 0.10mg/kg QW, and
   the third dose is about 2.00 mg/kg QW.
81. The compound or a pharmaceutically acceptable salt thereof according to Embodiment 78, wherein the compound or a pharmaceutically acceptable salt thereof is administered to dogs at a dosage regimen that
   the dose is about 0.1 to about 2.5 mg/kg; or the dose is about 0.1 to about 2.5 mg per injection;
   the administration frequency is QW; and
   the administration period is 6 weeks.
82. The compound or a pharmaceutically acceptable salt thereof according to any one of Embodiments 42-81, wherein the compound is selected from SEQ ID NO: 14, SEQ ID NO: 16 and SEQ ID NO: 20, preferably SEQ ID NO: 16.
83. A method or preventing or treating a disease in a non-primate and non-rodent animal, comprising administering to the animal in need thereof, an effective amount of a compound of the following Formula (AI), or a pharmaceutically acceptable salt thereof:

   Y-X₁-E-G-T-X₂-T-S-D-Y-A11-A12-A13-L-D-K-A17-A-Q-A20-E-F-V-K-W-L-L-K-A29-G-P-S- S-G-A-P-P-P-S-K Formula (AI);

   wherein X₁ is Aib; X₂ is αMePhe;
   A11 is Aib or Ala;
   A12 is Ala, Ile, Lys, Phe or Pya(4);
   A13 is Aib, Cha, Leu, αMePhe or αMeTyr;
   A17 is Gln or Ile;
   A20 is Ala or Ser;
   A29 is Gln or Gly,
   1, 2, 3 or 4 Ks, which are selected from the group consisting of K at position 16, K at position 24, K at position 28 and K at position 40, are chemically modified through conjugation to the ε-aminos of the K side-chains with ([2-(2-amino-ethoxy)-ethoxy]-acetyl)ₐ-(γ-Glu)_{b}-CO-(CH₂)_{c}-Z, wherein each a is independently an integer from 0 to 5, each b is independently an integer from 0 to 5, and each c is independently an integer from 10 to 24, wherein Z is independently selected from the group consisting of -CH₃, carboxylic acids or carboxylic acid bioisosteres, phosphonates or sulfonates.
84. The method according to Embodiment 83, wherein
   A11 is Aib; or/and
   A12 is Ile; or/and
   A13 is Aib; or/and
   A17 is Gln; or/and
   A20 is Ala; or/and
   A29 is Gly; or/and
   a is independently selected from an integer of 1, 2, 3, 4 or 5, b is independently selected from an integer of 1, 2, 3, 4 or 5, and c is independently selected from an integer of 12 to 22; preferably, c is independently selected from 14, 16, 18 or 20.
85. A method for preventing or treating a disease in a non-primate and non-rodent animal, comprising administering to the animal in need thereof, an effective amount of a compound of the following Formula (I), or a pharmaceutically acceptable salt thereof:

   Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K Formula (I);

   wherein X₁ is Aib; X₂ is αMePhe; X₃ is Aib; X₄ is Aib; 1, 2, 3 or 4 Ks, which are selected from the group consisting of K at position 16, K at position 24, K at position 28 and K at position 40, are chemically modified through conjugation to the ε-aminos of the K side-chains with ([2-(2-amino-ethoxy)-ethoxy]-acetyl)ₐ-(γ-Glu)_{b}-CO-(CH₂)_{c}-Z, wherein each a is independently an integer from 0 to 5, each b is independently an integer from 0 to 5, and each c is independently an integer from 10 to 24, wherein Z is independently selected from the group consisting of -CH₃, carboxylic acids or carboxylic acid bioisosteres, phosphonates or sulfonates; and the C-terminal amino acid is amidated as a C-terminal primary amide.
86. The method according to Embodiment 83, wherein the compound is:

   Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K Formula (I),

   or a pharmaceutically acceptable salt thereof, wherein X₁ is Aib; X₂ is αMePhe; X₃ is Aib; X₄ is Aib; 1 or 2 Ks, which are selected from the group consisting of K at position 16, K at position 24, K at position 28 and K at position 40, are chemically modified through conjugation to the ε-aminos of the K side-chains with ([2-(2-amino-ethoxy)-ethoxy]-acetyl)ₐ-(γ-Glu)_{b}-CO-(CH₂)_{c}-Z, wherein Z is independently selected from the group consisting of -CH₃, carboxylic acids or carboxylic acid bioisosteres, phosphonates or sulfonates, preferably -CO₂H, each a is independently an integer from 0 to 5, each b is independently an integer from 0 to 5, and each c is independently an integer from 10 to 22; and the C-terminal amino acid is amidated as a C-terminal primary amide.
87. The method according to Embodiment 84, wherein
   K at position 16 is chemically modified through conjugation to the ε-amino of the K side-chain with ([2-(2-amino-ethoxy)-ethoxy]-acetyl)ₐ-(γ-Glu)_{b}-CO-(CH₂)_{c}-CO₂H; or
   K at position 24 is chemically modified through conjugation to the ε-amino of the K side-chain with ([2-(2-amino-ethoxy)-ethoxy]-acetyl)ₐ-(γ-Glu)_{b}-CO-(CH₂)_{c}-CO₂H; or
   K at position 28 is chemically modified through conjugation to the ε-amino of the K side-chain with ([2-(2-amino-ethoxy)-ethoxy]-acetyl)ₐ-(γ-Glu)_{b}-CO-(CH₂)_{c}-CO₂H; or
   K at position 40 is chemically modified through conjugation to the ε-amino of the K side-chain with ([2-(2-amino-ethoxy)-ethoxy]-acetyl)ₐ-(γ-Glu)_{b}-CO-(CH₂)_{c}-CO₂H.
88. The method according to Embodiment 84, wherein
   K at position 24 and K at position 28 are chemically modified with two modifying chains through conjugation to the ε-aminos of the K side-chains with([2-(2-amino-ethoxy)-ethoxy]-acetyl)ₐ-(γ-Glu)_{b}-CO-(CH₂)_{c}-CO₂H; or
   K at position 16 and K at position 24 are chemically modified with two modifying chains through conjugation to the ε-aminos of the K side-chains with ([2-(2-amino-ethoxy)-ethoxy]-acetyl)ₐ-(γ-Glu)_{b}-CO-(CH₂)_{c}-CO₂H; or
   K at position 16 and K at position 28 are chemically modified with two modifying chains through conjugation to the ε-aminos of the K side-chains with ([2-(2-amino-ethoxy)-ethoxy]-acetyl)ₐ-(γ-Glu)_{b}-CO-(CH₂)_{c}-CO₂H; or
   K at position 16 and K at position 40 are chemically modified with two modifying chains through conjugation to the ε-aminos of the K side-chains with ([2-(2-amino-ethoxy)-ethoxy]-acetyl)ₐ-(γ-Glu)_{b}-CO-(CH₂)_{c}-CO₂H; or
   K at position 24 and K at position 40 are chemically modified with two modifying chains through conjugation to the ε-aminos of the K side-chains with ([2-(2-amino-ethoxy)-ethoxy]-acetyl)ₐ-(γ-Glu)_{b}-CO-(CH₂)_{c}-CO₂H; or
   K at position 28 and K at position 40 are chemically modified with two modifying chains through conjugation to the ε-aminos of the K side-chains with ([2-(2-amino-ethoxy)-ethoxy]-acetyl)ₐ-(γ-Glu)_{b}-CO-(CH₂)_{c}-CO₂H.
89. The method according to any one of Embodiments 83-86, wherein
   a is independently selected from an integer of 1, 2, 3, 4 or 5, b is independently selected from an integer of 1, 2, 3, 4 or 5, and c is independently selected from an integer of 12 to 22; preferably, c is independently selected from 14, 16, 18 or 20; and/or
   Z is -CO₂H.
90. The method according to Embodiment 84, wherein the compound is

   Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K Formula (I);

   wherein X₁ is Aib; X₂ is αMePhe; X₃ is Aib; X₄ is Aib; K at position 24 is chemically modified through conjugation to the ε-amino of the K side-chain with ([2-(2-amino-ethoxy)-ethoxy]-acetyl)ₐ-(γ-Glu)_{b}-CO-(CH₂)_{c}-CO₂H, wherein a is independently selected from an integer from 0 to 5, b is independently selected from an integer from 0 to 5, and c is independently selected from an integer from 10 to 24; and the C-terminal amino acid is amidated as a C-terminal primary amide;
   preferably, wherein, X₁ is Aib; X₂ is αMePhe; X₃ is Aib; X₄ is Aib; K at position 24 is chemically modified through conjugation to the ε-amino of the K side-chain with ([2-(2-amino-ethoxy)-ethoxy]-acetyl)ₐ-(γ-Glu)_{b}-CO-(CH₂)_{c}-CO₂H, wherein each a is independently selected from an integer of 2 and 3, each b is independently 1, and each c is independently selected from an integer of 16, 18 and 20, wherein Z is independently selected from carboxylic acids.
91. The method according to Embodiment 84, the compound is

   Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K Formula (I);

   wherein X₁ is Aib; X₂ is αMePhe; X₃ is Aib; X₄ is Aib; K at position 28 is chemically modified through conjugation to the ε-amino of the K side-chain with ([2-(2-amino-ethoxy)-ethoxy]-acetyl)ₐ-(γ-Glu)_{b}-CO-(CH₂)_{c}-CO₂H, wherein a is independently selected from an integer from 0 to 5, b is independently selected from an integer from 0 to 5, and c is independently selected from an integer from 10 to 24; and the C-terminal amino acid is amidated as a C-terminal primary amide;
   preferably, wherein, X₁ is Aib; X₂ is αMePhe; X₃ is Aib; X₄ is Aib; K at position 28 is chemically modified through conjugation to the ε-amino of the K side-chain with ([2-(2-amino-ethoxy)-ethoxy]-acetyl)ₐ-(γ-Glu)_{b}-CO-(CH₂)_{c}-CO₂H, wherein each a is independently selected from an integer of 2 and 3, each b is independently selected from an integer of 1 and 3, and each c is independently selected from an integer of 16 and 20, wherein Z is independently selected from carboxylic acids.
92. The method according to any one of Embodiments 88-89, wherein
   a is independently selected from an integer of 1, 2, 3, 4 or 5, b is independently selected from an integer of 1, 2, 3, 4 or 5, and c is independently selected from an integer of 12 to 22; preferably, c is independently selected from 14, 16, 18 or 20.
93. The method according to Embodiments 84, the compound is

   Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K;

   wherein X₁ is Aib; X₂ is αMePhe; X₃ is Aib; X₄ is Aib; K at position 24 is chemically modified through conjugation to the ε-amino of the K side-chain with ([2-(2-amino-ethoxy)-ethoxy]-acetyl)₂-(γ-Glu)₁-CO-(CH₂)₁₆-CO₂H; and the C-terminal amino acid is amidated as a C-terminal primary amide (SEQ ID NO:7); or

   Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K;

   wherein X₁ is Aib; X₂ is αMePhe; X₃ is Aib; X₄ is Aib; K at position 24 is chemically modified through conjugation to the ε-amino of the K side-chain with ([2-(2-amino-ethoxy)-ethoxy]-acetyl)₂-(γ-Glu)₁-CO-(CH₂)₁₈-CO₂H; and the C-terminal amino acid is amidated as a C-terminal primary amide (SEQ ID NO:13); or

   Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K;

   wherein X₁ is Aib; X₂ is αMePhe; X₃ is Aib; X₄ is Aib; K at position 24 is chemically modified through conjugation to the ε-amino of the K side-chain with ([2-(2-amino-ethoxy)-ethoxy]-acetyl)₂-(γ-Glu)₁-CO-(CH₂)₂₀-CO₂H; and the C-terminal amino acid is amidated as a C-terminal primary amide (SEQ ID NO:14); or

   Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K;

   wherein X₁ is Aib; X₂ is αMePhe; X₃ is Aib; X₄ is Aib; K at position 24 is chemically modified through conjugation to the ε-amino of the K side-chain with ([2-(2-amino-ethoxy)-ethoxy]-acetyl)₂-(γ-Glu)₃-CO-(CH₂)₁₆-CO₂H; and the C-terminal amino acid is amidated as a C-terminal primary amide (SEQ ID NO:15); or

   Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K;

   wherein X₁ is Aib; X₂ is αMePhe; X₃ is Aib; X₄ is Aib; K at position 24 is chemically modified through conjugation to the ε-amino of the K side-chain with ([2-(2-amino-ethoxy)-ethoxy]-acetyl)₃-(γ-Glu)₁-CO-(CH₂)₂₀-CO₂H; and the C-terminal amino acid is amidated as a C-terminal primary amide (SEQ ID NO:16); or

   Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K;

   wherein X₁ is Aib; X₂ is αMePhe; X₃ is Aib; X₄ is Aib; K at position 28 is chemically modified through conjugation to the ε-amino of the K side-chain with ([2-(2-amino-ethoxy)-ethoxy]-acetyl)₂-(γ-Glu)₁-CO-(CH₂)₁₆-CO₂H; and the C-terminal amino acid is amidated as a C-terminal primary amide (SEQ ID NO:8); or

   Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K;

   wherein X₁ is Aib; X₂ is αMePhe; X₃ is Aib; X₄ is Aib; K at position 28 is chemically modified through conjugation to the ε-amino of the K side-chain with ([2-(2-amino-ethoxy)-ethoxy]-acetyl)₂-(γ-Glu)₁-CO-(CH₂)₂₀-CO₂H; and the C-terminal amino acid is amidated as a C-terminal primary amide (SEQ ID NO:17); or

   Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K;

   wherein X₁ is Aib; X₂ is αMePhe; X₃ is Aib; X₄ is Aib; K at position 28 is chemically modified through conjugation to the ε-amino of the K side-chain with ([2-(2-amino-ethoxy)-ethoxy]-acetyl)₂-(γ-Glu)₃-CO-(CH₂)₁₆-CO₂H; and the C-terminal amino acid is amidated as a C-terminal primary amide (SEQ ID NO:18); or

   Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K;

   wherein X₁ is Aib; X₂ is αMePhe; X₃ is Aib; X₄ is Aib; K at position 28 is chemically modified through conjugation to the ε-amino of the K side-chain with ([2-(2-amino-ethoxy)-ethoxy]-acetyl)₃-(γ-Glu)₁-CO-(CH₂)₁₆-CO₂H; and the C-terminal amino acid is amidated as a C-terminal primary amide (SEQ ID NO:19); or

   Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K;

   wherein X₁ is Aib; X₂ is αMePhe; X₃ is Aib; X₄ is Aib; K at position 28 is chemically modified through conjugation to the ε-amino of the K side-chain with ([2-(2-amino-ethoxy)-ethoxy]-acetyl)₃-(γ-Glu)₁-CO-(CH₂)₂₀-CO₂H; and the C-terminal amino acid is amidated as a C-terminal primary amide (SEQ ID NO:20).
94. The method according to Embodiments 84, the compound is 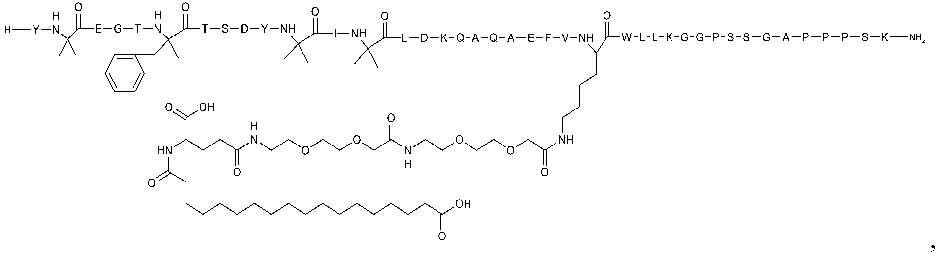 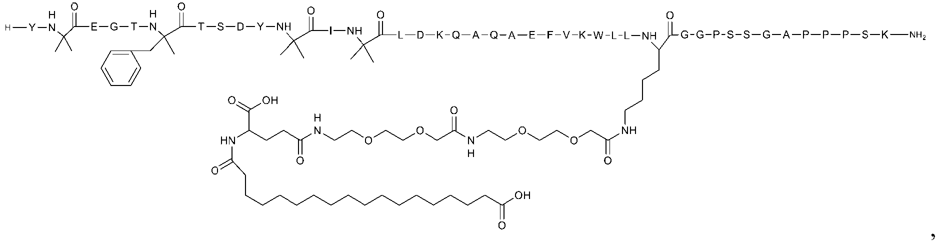 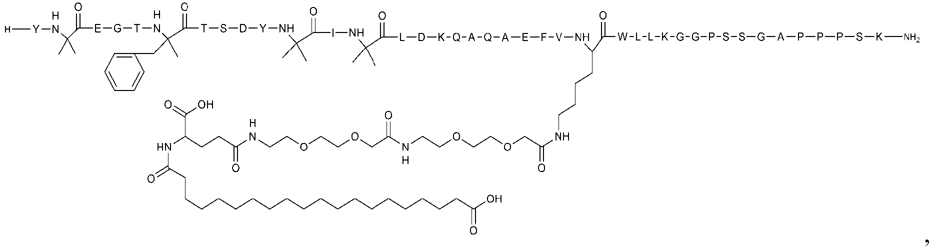 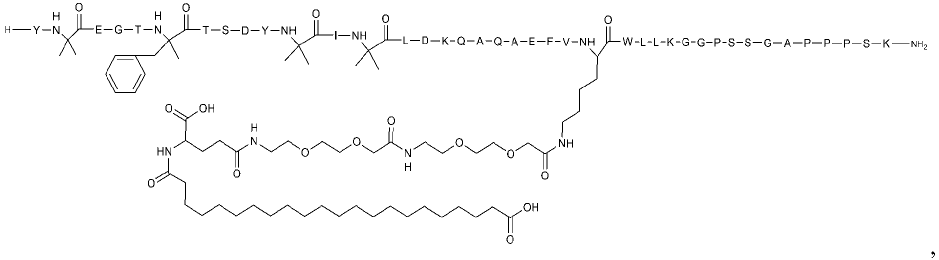 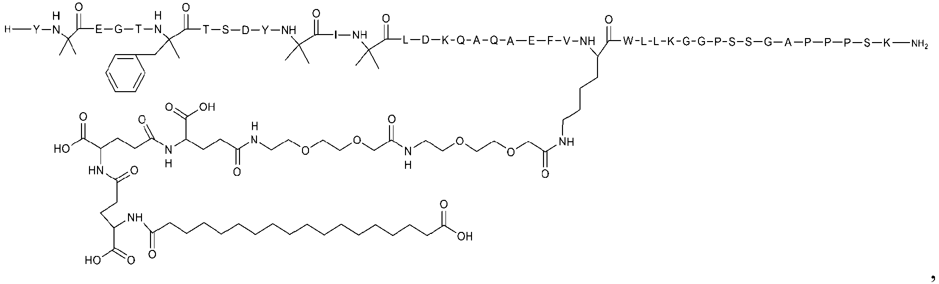 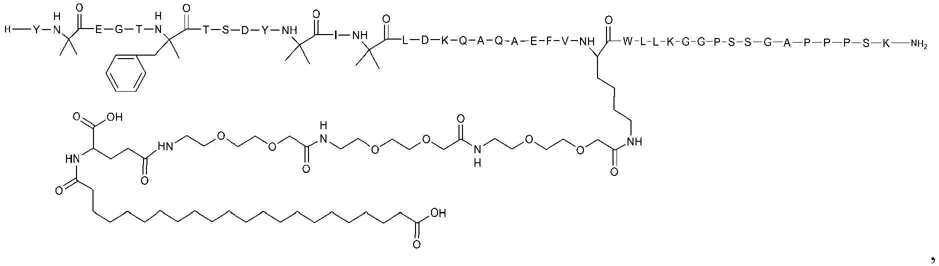 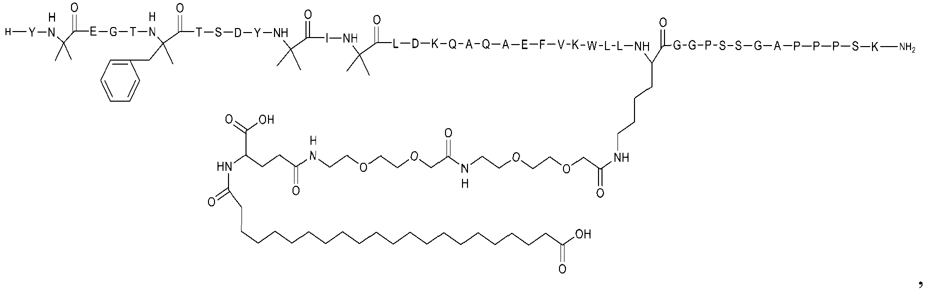 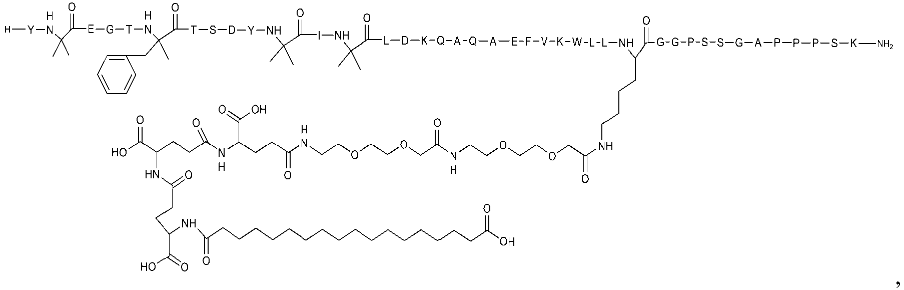 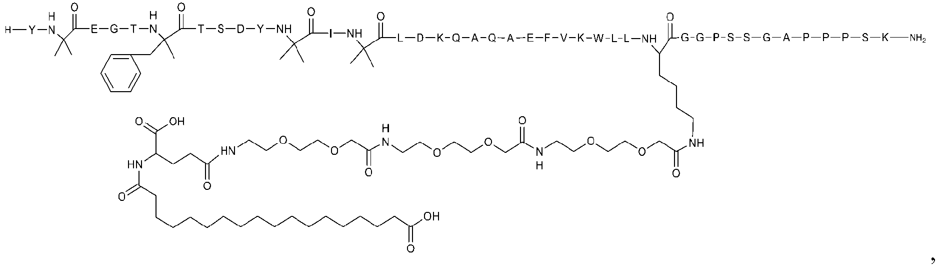 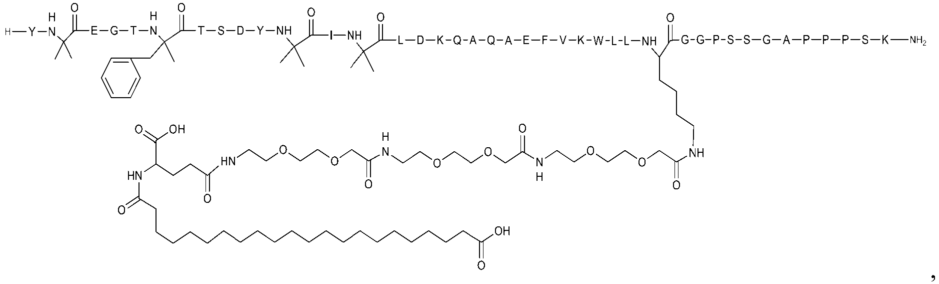 or a pharmaceutically acceptable salt thereof.
95. The method according to any one of Embodiments 83-94, wherein the disease includes animal hyperglycemia, impaired glucose tolerance, diabetes mellitus (including Type I diabetes mellitus, Type II diabetes mellitus), obesity, hypertension, dyslipidemia, cognitive impairment, atherosclerosis, myocardial infarction, coronary heart disease and other cardiovascular diseases, stroke, inflammatory bowel syndrome, dyspepsia and gastric ulcers.
96. The method according to any one of Embodiments 83-94, wherein the medicament is a medicament for preventing, delaying or treating Type II diabetes mellitus.
97. The method according to any one of Embodiments 83-94, wherein the compound or a pharmaceutically acceptable salt thereof is used for a medicament for reducing animal food intake, reducing β-cell apoptosis, increasing β-cell function and β-cell volume and/or restoring glucose sensitivity of β-cells.
98. The method according to any one of Embodiments 83-94, wherein the disease includes animal metabolic disorders, including dyslipidaemia, obesity and/or hepatic steatosis associated with insulin resistance and diabetes mellitus.
99. The method according to any one of Embodiments 83-94, wherein the disease includes osteopenia and bone/joint diseases, such as knee osteoarthritis, hip osteoarthritis, and/or spondylitis deformans.
100. The method according to any one of Embodiments 83-94, wherein the compound or a pharmaceutically acceptable salt thereof is used for chronic weight management as an adjunct to a reduced-calorie diet and increased physical activity in an overweight or obese animal.
101. The method according to any one of Embodiments 83-94, wherein the disease includes: symptomatic obesity, obesity based on simple obesity, obesity-related conditions or diseases, eating disorders, diabetes mellitus (for example, Type I diabetes mellitus, Type II diabetes mellitus, gestational diabetes mellitus, obesity diabetes mellitus), hyperlipidemia (e.g., hypertriglyceridemia, hypercholesterolemia, hyper-LDL-cholesterolemia, low HDL-cholesterolemia, postprandial hyperlipidemia), hypertension, heart failure, complications of diabetes mellitus (for example, neuropathy, nephropathy, retinopathy, diabetic cardiomyopathy, cataract, macroangiopathy, osteopenia, hyperosmolar diabetic coma), infectious disease (for example, respiratory tract infection, urinary tract infection, gastrointestinal infection, superficial soft tissue infection, lower extremity infection), diabetic gangrene, xerostomia, hypacusia, cerebrovascular disorder, peripheral blood circulation disorder, metabolic syndrome (with three or more conditions selected from the group consisting of hypertriglyceridemia (TG), low HDL-cholesterolemia , hypertension, abdominal obesity, and impaired glucose tolerance), and/or sarcopenia.
102. The method according to any one of Embodiments 82-101, wherein the animal is a pet or a companion animal.
103. The method according to Embodiment 102, wherein the pet is selected from dogs, cats, rabbits, pigs, alpacas, horses, sheep and bovines.
104. The method according to any one of Embodiments 83-101, wherein the animal is a dog or a cat.
105. The method according to any one of Embodiments 83-104, wherein the compound or a pharmaceutically acceptable salt thereof is used to treat Type II diabetes mellitus, overweight or and/obesity in cats or dogs.
106. The method according to any one of Embodiments 83-105, wherein the compound or a pharmaceutically acceptable salt thereof is administered in the form of a solution or a suspension.
107. The method according to any one of Embodiments 83-106, wherein the compound or a pharmaceutically acceptable salt thereof is administered by subcutaneous injection.
108. The method according to any one of Embodiments 82-106, wherein the compound or a pharmaceutically acceptable salt thereof is administered to cats or dogs at a dose of about 0.003 to about 2.5 mg/kg, or about 0.003 to about 0.6 mg/kg, for example, about 0.005 to about 0.1mg/kg, about 0.1 to about 0.125mg/kg, about 0.1 to about 0.15mg/kg, about 0.15 to about 0.25mg/kg, about 0.125 to about 0.25mg/kg, about 0.005 to about 2 mg/kg, about 0.005 to about 1.5 mg/kg, about 0.005 to about 1 mg/kg, about 0.005 to about 0.5 mg/kg, about 0.1 to about 2 mg/kg, about 0.1 to about 1.5 mg/kg, about 0.1 to about 1 mg/kg, about 0.1 to about 0.5 mg/kg, about 0.15 to about 2.5 mg/kg, about 0.15 to about 2 mg/kg, about 0.15 to about 1.5 mg/kg, about 0.15 to about 1 mg/kg, about 0.15 to about 0.5 mg/kg, about 0.2 to about 2.5 mg/kg, about 0.2 to about 2 mg/kg, about 0.2 to about 1.5 mg/kg, about 0.2 to about 1 mg/kg, about 0.2 to about 0.5 mg/kg, about 0.25 to about 2.5 mg/kg, about 0.25 to about 2 mg/kg, about 0.25 to about 1.5 mg/kg, about 0.25 to about 1 mg/kg, about 0.25 to about 0.5 mg/kg, about 0.3 to about 2.5 mg/kg, about 0.3 to about 2 mg/kg, about 0.3 to about 1.5 mg/kg, about 0.3 to about 1 mg/kg, about 0.3 to about 0.5 mg/kg, about 0.301 to 2.001 mg/kg, about 0.35 to about 2.5 mg/kg, about 0.35 to about 2 mg/kg, about 0.35 to about 1.5 mg/kg, about 0.35 to about 1 mg/kg, about 0.35 to about 0.5 mg/kg, about 0.4 to about 2.5 mg/kg, about 0.4 to about 2 mg/kg, about 0.4 to about 1.5 mg/kg, about 0.4 to about 1 mg/kg, about 0.4 to about 0.5 mg/kg, about 0.5 to about 2.5 mg/kg, about 0.5 to about 2 mg/kg, about 0.5 to about 1.5 mg/kg, about 0.5 to about 1 mg/kg, about 2.5 mg/kg, about 2.0 mg/kg, about 1.95 mg/kg, about 1.9 mg/kg, about 1.85 mg/kg, about 1.8 mg/kg, about 1.75 mg/kg, about 1.7 mg/kg, about 1.65 mg/kg, about 1.6 mg/kg, about 1.55 mg/kg, about 1.5 mg/kg, about 1.45 mg/kg, about 1.4 mg/kg, about 1.35 mg/kg, about 1.3 mg/kg, about 1.25 mg/kg, about 1.2 mg/kg, about 1.15 mg/kg, about 1.1 mg/kg, about 1.05 mg/kg, about 1.0 mg/kg, about 0.95 mg/kg, about 0.9 mg/kg, about 0.85 mg/kg, about 0.8 mg/kg, about 0.75 mg/kg, about 0.7 mg/kg, about 0.65 mg/kg, about 0.6 mg/kg, about 0.55 mg/kg, about 0.5 mg/kg, about 0.45 mg/kg, about 0.4 mg/kg, about 0.35 mg/kg, about 0.3 mg/kg, about 0.25 mg/kg, about 0.2 mg/kg, about 0.15 mg/kg, or about 0.1 mg/kg.
109. The method according to any one of Embodiments 82-106, wherein the compound or a pharmaceutically acceptable salt thereof is administered to cats or dogs once a week.
110. The method according to any one of Embodiments 82-106, wherein the compound or a pharmaceutically acceptable salt thereof is administered to cats or dogs at a dosage regimen of about 0.003 to about 2.5 mg/kg QW, preferably about 0.01 to about 2 mg/kg QW, more preferably about 0.25 to about 2.0 mg/kg QW, and/or, about 0.1 to about 2.0 mg/kg QW, further more preferalby about 0.5 to about 2.0 mg/kg QW, for example, about 0.01 to about 2 mg/kg QW, about 0.01 to about 1.5 mg/kg QW, about 0.01 to about 1 mg/kg QW, about 0.01 to about 0.5 mg/kg QW, about 0.02 to about 2.5 mg/kg QW, about 0.02 to about 2 mg/kg QW, about 0.02 to about 1.5 mg/kg QW, about 0.02 to about 1 mg/kg QW, about 0.02 to about 0.5 mg/kg QW, about 0.021 to about 0.499 mg/kg QW, about 0.03 to about 2.5 mg/kg QW, about 0.03 to about 2 mg/kg QW, about 0.03 to about 1.5 mg/kg QW, about 0.03 to about 1 mg/kg QW, about 0.03 to about 0.5 mg/kg QW, about 0.04 to about 2.5 mg/kg QW, about 0.04 to about 2 mg/kg QW, about 0.04 to about 1.5 mg/kg QW, about 0.04 to about 1 mg/kg QW, about 0.04 to about 0.5 mg/kg QW, about 0.05 to about 2.5 mg/kg QW, about 0.05 to about 2 mg/kg QW, about 0.05 to about 1.5 mg/kg QW, about 0.05 to about 1 mg/kg QW, about 0.05 to about 0.5 mg/kg QW, about 0.06 to about 2.5 mg/kg QW, about 0.06 to about 2 mg/kg QW, about 0.06 to about 1.5 mg/kg QW, about 0.06 to about 1 mg/kg QW, about 0.06 to about 0.5 mg/kg QW, about 0.062 to2.001 mg/kg QW, about 0.07 to about 2.5 mg/kg QW, about 0.07 to about 2 mg/kg QW, about 0.07 to about 1.5 mg/kg QW, about 0.07 to about 1 mg/kg QW, about 0.07 to about 0.5 mg/kg QW, about 0.073 to0.998 mg/kg QW, about 0.08 to about 2.5 mg/kg QW, about 0.08 to about 2 mg/kg QW, about 0.08 to about 1.5 mg/kg QW, about 0.08 to about 1 mg/kg QW, about 0.08 to about 0.5 mg/kg QW, about 0.09 to about 2.5 mg/kg QW, about 0.09 to about 2 mg/kg QW, about 0.09 to about 1.5 mg/kg QW, about 0.09 to about 1 mg/kg QW, about 0.09 to about 0.5 mg/kg QW, about 0.1 to about 2.5 mg/kg QW, about 0.1 to about 2 mg/kg QW, about 0.1 to about 1.5 mg/kg QW, about 0.1 to about 1 mg/kg QW, about 0.1 to about 0.5 mg/kg QW, about 0.15 to about 2.5 mg/kg QW, about 0.15 to about 2 mg/kg QW, about 0.15 to about 1.5 mg/kg QW, about 0.15 to about 1 mg/kg QW, about 0.15 to about 0.5 mg/kg QW, about 0.2 to about 2.5 mg/kg QW, about 0.2 to about 2 mg/kg QW, about 0.2 to about 1.5 mg/kg QW, about 0.2 to about 1 mg/kg QW, about 0.2 to about 0.5 mg/kg QW, about 0.25 to about 2.5 mg/kg QW, about 0.25 to about 2 mg/kg QW, about 0.25 to about 1.5 mg/kg QW, about 0.25 to about 1 mg/kg QW, about 0.25 to about 0.5 mg/kg QW, about 0.3 to about 2.5 mg/kg QW, about 0.3 to about 2 mg/kg QW, about 0.3 to about 1.5 mg/kg QW, about 0.3 to about 1 mg/kg QW, about 0.3 to about 0.5 mg/kg QW, about 0.301 to2.001 mg/kg QW, about 0.35 to about 2.5 mg/kg QW, about 0.35 to about 2 mg/kg QW, about 0.35 to about 1.5 mg/kg QW, about 0.35 to about 1 mg/kg QW, about 0.35 to about 0.5 mg/kg QW, about 0.4 to about 2.5 mg/kg QW, about 0.4 to about 2 mg/kg QW, about 0.4 to about 1.5 mg/kg QW, about 0.4 to about 1 mg/kg QW, about 0.4 to about 0.5 mg/kg QW, about 0.5 to about 2 mg/kg QW, about 0.5 to about 1.5 mg/kg QW, about 0.5 to about 1 mg/kg QW, about 2.5 mg/kg QW, about 2.0 mg/kg QW, about 1.95 mg/kg QW, about 1.9 mg/kg QW, about 1.85 mg/kg QW, about 1.8 mg/kg QW, about 1.75 mg/kg QW, about 1.7 mg/kg QW, about 1.65 mg/kg QW, about 1.6 mg/kg QW, about 1.55 mg/kg QW, about 1.5 mg/kg QW, about 1.45 mg/kg QW, about 1.4 mg/kg QW, about 1.35 mg/kg QW, about 1.3 mg/kg QW, about 1.25 mg/kg QW, about 1.2 mg/kg QW, about 1.15 mg/kg QW, about 1.1 mg/kg QW, about 1.05 mg/kg QW, about 1.0 mg/kg QW, about 0.95 mg/kg QW, about 0.9 mg/kg QW, about 0.85 mg/kg QW, about 0.8 mg/kg QW, about 0.75 mg/kg QW, about 0.7 mg/kg QW, about 0.65 mg/kg QW, about 0.6 mg/kg QW, about 0.55 mg/kg QW, about 0.5 mg/kg QW, about 0.45 mg/kg QW, about 0.4 mg/kg QW, about 0.35 mg/kg QW, about 0.3 mg/kg QW, about 0.25 mg/kg QW, or about 0.2 mg/kg QW.
111. The method according to any one of Embodiments 82-106, wherein the compound or a pharmaceutically acceptable salt thereof is continuously administered to cats or dogs at a frequency of once a week, and the administration period is determined according to the needs of the subject, and the administration period may be 4-8 weeks, or 8-20 weeks, such as 4, 5, 6, 7 or 8 weeks.
112. The method according to any one of Embodiments 82-106, wherein the compound or a pharmaceutically acceptable salt thereof is continuously administered to dogs at a frequency of once a week for 6 weeks, and the dose for each administration may be the same or different.
113. The method according to any one of Embodiments 82-106, wherein the compound or a pharmaceutically acceptable salt thereof is continuously administered to cats at a frequency of once a week for 5 weeks, and the dose for each administration may be the same or different.
114. The method according to Embodiment 112 or 113, wherein the compound or a pharmaceutically acceptable salt thereof is administered to cats or dogs at escalating doses.
115. The method according to any one of Embodiments 82-106, wherein the compound or a pharmaceutically acceptable salt thereof is administered to cats at a dose that
   the first administration and the second administration are identical and at the first dose of about 0.02 to about 0.35 mg/kg QW,
   the third adminisiation is at a second dose of about 0.49 to about 1 mg/kg QW, and the second dose is from about 1.2 to about 50 times of the first dose, preferably from about 10 to about 15 times, such as about 12 times, and
   the fourth administration and the fifth administration are identical and at the third dosge of from about 0.49 to about 2.5 mg/kg QW, wherein the third dose is from about 1.75 to about 2.5 times of the second dose, preferably from about 1.95 to about 2.05 times, more preferably about 2 times.
116. The method according to any one of Embodiments 82-106, wherein the compound or a pharmaceutically acceptable salt thereof is administered to cats at a dose that
   the first dose is about 0.019 mg/kg QW,
   the second dose is about 0.25 mg/kg QW, and
   the third dose is about 0.5 mg/kg QW.
117. The method according to any one of Embodiments 82-106, wherein the compound or a pharmaceutically acceptable salt thereof is administered to cats at a dose that
   the first dose is about 0.075 mg/kg QW,
   the second dose is about 0.50 mg/kg QW, and
   the third dose is about 1.00 mg/kg QW.
118. The method according to any one of Embodiments 82-106, wherein the compound or a pharmaceutically acceptable salt thereof is administered to cats at a dose that
   the first dose is about 0.30 mg/kg QW,
   the second dose is about 1.00 mg/kg QW, and
   the third dose is about 2.00 mg/kg QW.
119. The method according to any one of Embodiments 82-106, wherein the compound or a pharmaceutically acceptable salt thereof is administered to cats at a dosage regimen that
   the dose is about 0.25 to about 2.5 mg/kg; or the dose is about 0.25 to about 2.5 mg per injection;
   the administration frequency is QW; and
   the administration period is 5 weeks.
120. The method according to any one of Embodiments 82-106, wherein the compound or a pharmaceutically acceptable salt thereof is administered to dogs at a dose that
   the first administration and the second administration are identical and at the first dose of about 0.015 to about 0.07 mg/kg QW,
   the third adminisiation is at a second dose of about 0.02 to about 0.14 mg/kg QW, preferably about 0.03 to about 0.1 mg/kg QW, wherein the second dose is from about 1.4 to 2 times of the first dose, preferably from about 1.45 to 1.6 times, more preferably about 1.5 times, and
   the fourth administration to the six administration are identical and at the third dosge of from about 0.49 to about 2.5 mg/kg QW, preferably about 0.495 to about 2.1 mg/kg QW, wherein the third dose is from about 13 to about 25 times of the second dose, preferably from about 16 to 21 times.
121. The method according to Embodiments 120, wherein the compound or a pharmaceutically acceptable salt thereof is administered to dogs at a dose that
   the first dose is about 0.02 mg/kg QW,
   the second dose is about 0.03 mg/kg QW, and
   the third dose is about 0.50 mg/kg QW.
122. The method according to Embodiments 120, wherein the compound or a pharmaceutically acceptable salt thereof is administered to dogs at a dose that
   the first dose is about 0.06 mg/kg QW,
   the second dose is about 0.10 mg/kg QW, and
   the third dose is about 2.00 mg/kg QW.
123. The method according to Embodiments 120, wherein the compound or a pharmaceutically acceptable salt thereof is administered to dogs at a dosage regimen that
   the dose is about 0.1 to about 2.5 mg/kg; or the dose is about 0.1 to about 2.5 mg per injection;
   the administration frequency is QW; and
   the administration period is 6 weeks.
124. The method according to any one of Embodiments 1-40, wherein the compound is selected from SEQ ID NO: 14, SEQ ID NO: 16 and SEQ ID NO: 20, preferably SEQ ID NO: 16.

### DESCRIPTION OF DRAWINGS

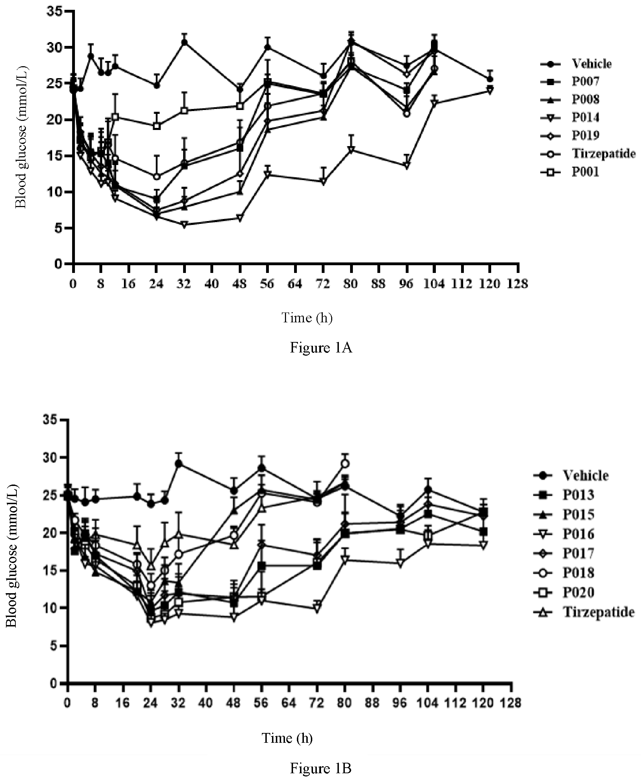

FIGS. 1A-1B show blood glucose changes in db/db mice after administration. FIG. 1A shows the blood glucose changes in db/db mice after a single subcutaneous administration of P007, P008, P014, P019, Tirzepatide and P001 at 10 nmol/kg; and FIG. 1B shows the blood glucose changes in db/db mice after a single subcutaneous administration of P013, P015, P016, P017, P018, P020 and Tirzepatide at 10 nmol/kg.

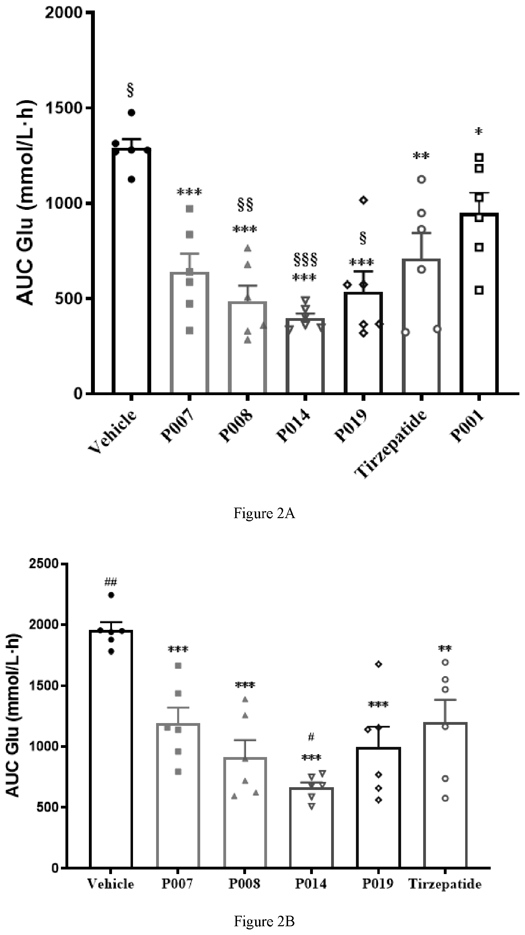

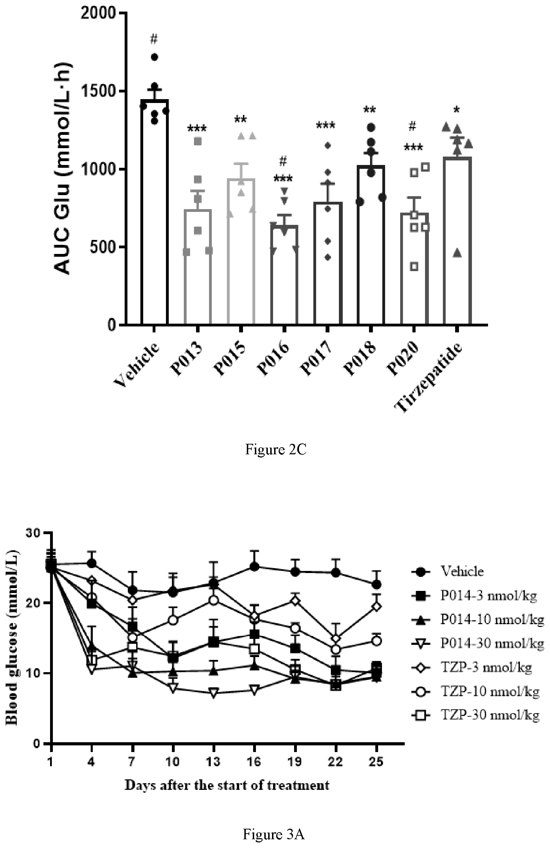

FIGS. 2A-2C show blood glucose AUC in db/db mice after administration. FIG. 2A shows the AUC from 0 to 48 hours after a single subcutaneous administration of P007, P008, P014, P019, Tirzepatide and P001 at 10 nmol/kg; FIG. 2B shows the AUC from 0 to 72 hours after a single subcutaneous administration of P007, P008, P014, P019 and Tirzepatide at 10 nmol/kg; and FIG. 2C shows the AUC from 0 to 56 hours after a single subcutaneous administration of P013, P015, P016, P017, P018, P020 and Tirzepatide at 10 nmol/kg. In FIG. 2A, T-Test, **p*<0.05, ***p*<0.01, ****p*<0.001, *vs.* Vehicle; § *p*<0.05, §§ *p*<0.01, § §§ *p*<0.001, *vs.* P001. In FIG. 2B, T-Test, **p*<0.05, ***p*<0.01, ****p*<0.001, *vs.* Vehicle; #*p*<0.05, ##*p*<0.01, *vs.* Tirzepatide. In FIG. 2C, T-Test, **p*<0.05, ***p*<0.01, ****p*<0.001 *vs.* Vehicle; #*p*<0.05, vs. Tirzepatide.

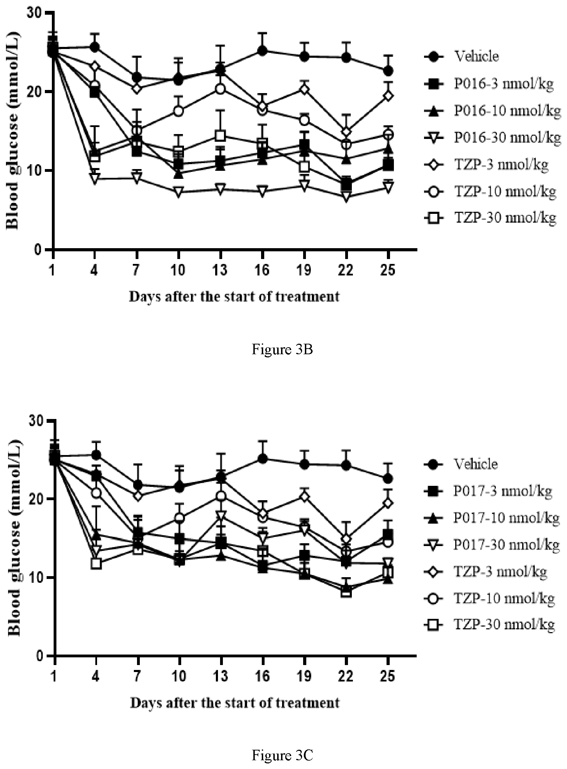

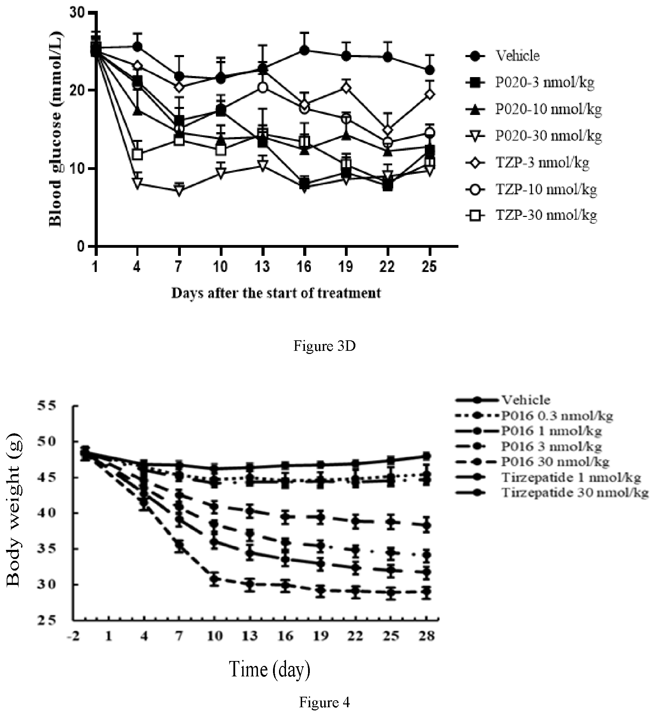

FIGS. 3A-3D show changes in blood glucose in db/db mice after multiple administrations. FIG. 3A compares the changes of blood glucose in db/db mice after multiple subcutaneous administrations of P014 and Tirzepatide; FIG. 3B compares the changes of blood glucose in db/db mice after multiple subcutaneous administrations of P016 and Tirzepatide; FIG. 3C compares changes in blood glucose in db/db mice after multiple subcutaneous administrations of P017 and Tirzepatide; and FIG. 3D compares changes in blood glucose in db/db mice after multiple subcutaneous administrations of P020 and Tirzepatide.
FIG. 4 shows body weight trends of DIO mice after multiple subcutaneous administrations of P016 (0.3, 1, 3, 30 nmol/kg) and Tirzepatide (1, 30 nmol/kg) at a frequency of once every 3 days.

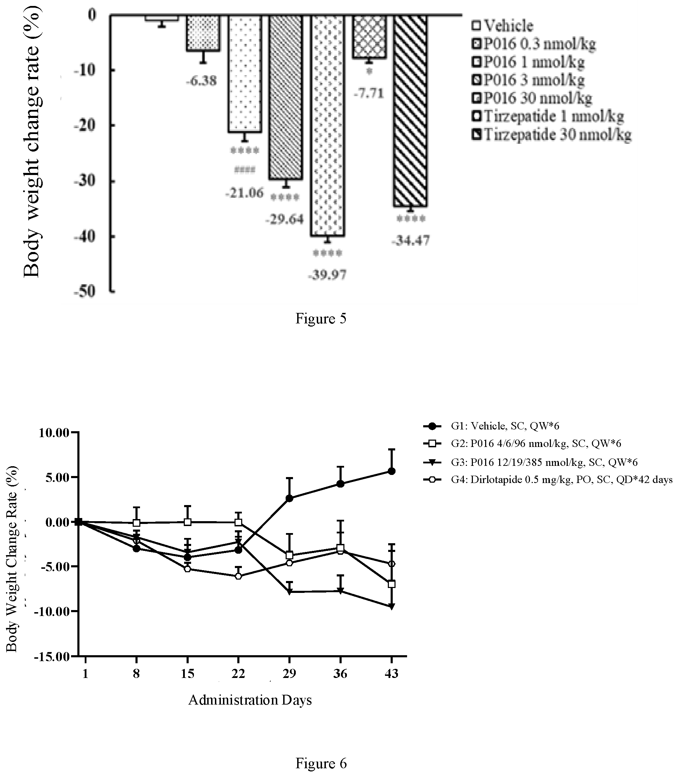

FIG. 5 shows body weight change rate (%) of DIO mice on day 28 (D28) after multiple subcutaneous administrations of P016 (0.3, 1, 3, 30 nmol/kg) and Tirzepatide (1, 30 nmol/kg) at a frequency of once every 3 days. **p*≤0.05, **** *p*≤0.0001, *vs.* Vehicle group; ####*p*≤0.0001 *vs.* positive control Tirzepatide at the same dose.
FIG. 6 shows weekly trends of body weight change of obese dogs after 6 continuous subcutaneous administrations of P016 (4, 4, 6, 96, 96, 96 nmol/kg; or 12, 12, 19, 385, 385, 385 nmol/kg) QW, and positive control Tirzepatide is continuously administered at a frequency of once every day (QD) for 42 days.

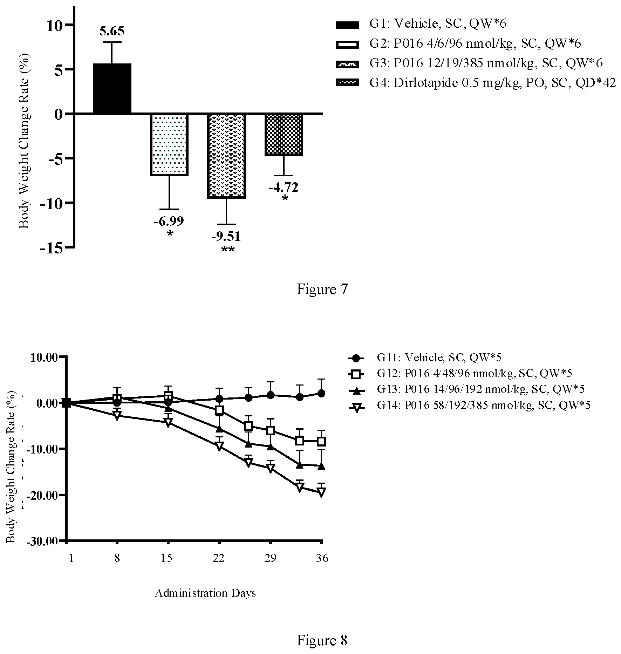

FIG. 7 shows weekly trends of body weight change rate (%) of obese dogs after 6 continuous subcutaneous administrations of P016 (4, 4, 6, 96, 96, 96 nmol/kg; or 12, 12, 19, 385, 385, 385 nmol/kg) QW, and positive control Tirzepatide is continuously administered at a frequency of once every day (QD) for 42 days. **p* < 0.05,***p* < 0.01, *vs.* Vehicle group.
FIG. 8 shows weekly trends of body weight change of obese cats after 5 continuous subcutaneous administrations of P016 (4, 4, 48, 96, 96 nmol/kg; or 14, 14, 96, 192, 192 nmol/kg;or 58, 58, 192, 385, 385 nmol/kg) QW.

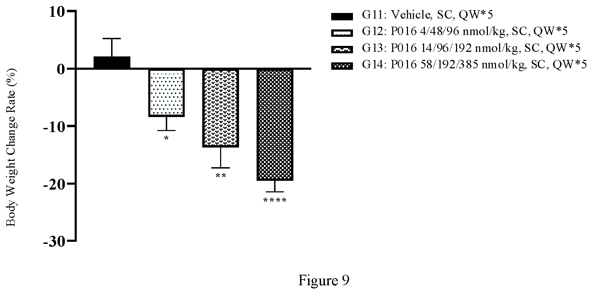

FIG. 9 shows weekly trends of body weight change rate (%) of obese cats after 5 continuous subcutaneous administrations of P016 (4, 4, 48, 96, 96 nmol/kg; or 14, 14, 96, 192, 192 nmol/kg; or 58, 58, 192, 385, 385 nmol/kg) QW. **p* < 0.05, ***p* < 0.01, *****p* < 0.0001, *vs.* Vehicle group.

The present invention also includes novel intermediates and methods useful in the synthesis of compounds or pharmaceutically acceptable salts thereof according to the present invention. The intermediates and compounds according to the present invention can be prepared by various methods known in the art. In particular, methods using chemical synthesis are exemplified in the Examples below. The specific synthetic steps of each pathway as described can be combined in various ways to prepare compounds or salts thereof according to the present invention. Reagents and starting materials are readily available to those skilled in the art. It should be understood that these examples are not intended to limit the scope of the present invention in any way. The mass spectrometer is performed on an Agilent 1260/6110 liquid chromatography mass spectrometer, and the scanning range is between 100 and 1500.

### Example 1:

Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K;

wherein X₁ is Aib; X₂ is αMePhe; X₃ is Aib; X₄ is Aib; K at position 24 is chemically modified through conjugation to the ε-amino of the K side-chain with ([2-(2-amino-ethoxy)-ethoxy]-acetyl)₂-(γ-Glu)₃-CO-(CH₂)₁₆-CO₂H; and the C-terminal amino acid is amidated as a C-terminal primary amide (SEQ ID NO:15).

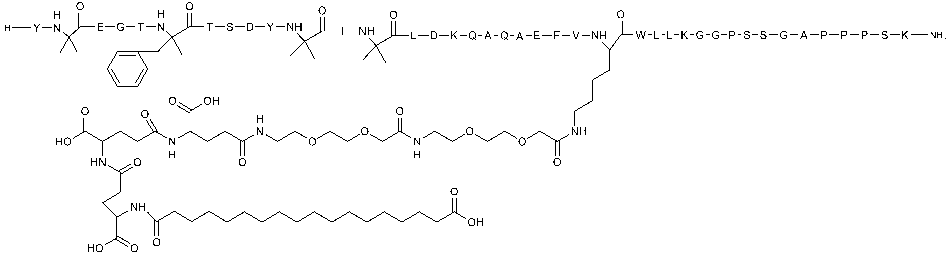

The above structure contains the standard single letter amino acid code with exception of residues Aib2, αMePhe6, Aib11, Aib13 and K24 where the structures of amino acid residues Aib2, αMePhe6, Aib11, Aib13 and K24 have been expanded.

### (1) Synthesis of Peptide-resin Intermediate 1

Fmoc-Rink Linker-Nle-MBHA resin (S=0.49 mmol/g) was charged, swelled with an appropriate amount of DCM, washed with DCM for 2 to 3 times, de-protected with 20% PIP/DMF solution for 30 minutes, filtered and washed to obtain a NH₂-Rink linker-Nle-MBHA resin with Fmoc removed, which was suctioned to remove the solvent for further use.

4 equivalents of Fmoc-Lys(Boc)-OH and HOBt were charged respectively, and dissolved in an appropriate amount of DMF/DCM. Separately, 4 equivalents of DIC was charged and double diluted with DCM. The resultant diluent was slowly added to the DMF/DCM solution, and reacted under stirring at -5 to 0°C for no less than 60 minutes so as to be activated for further use.

The activated Fmoc-Lys(Boc)-OH solution was added to the NH₂-Rink linker-Nle-MBHA resin. The reaction temperature was controlled at 10 to 30°C. The coupling reaction was performed for 240 to 480 minutes. After filtration and washing, a Fmoc-Lys(Boc)-Rink linker-Nle-MBHA resin was obtained. The resin was de-protected with 20% PIP/DMF solution for 30 minutes. After filtration and washing, a Lys(Boc)-Rink linker-Nle-MBHA resin with Fmoc removed was obtained.

According to the reaction conditions as stated above, each amino acid was successively coupled starting from the second amino acid at the C-terminus to the N-terminus. If the coupling was incomplete (a color development reaction occurs), a second condensation was carried out using HBTU/DIEA to ensure that each amino acid was completely condensed. The couplings were successively performed in the following order: Fmoc-Ser(tBu)-OH, Fmoc-Pro-OH, Fmoc-Pro-OH, Fmoc-Pro-OH, Fmoc-Ala-OH.H₂O, Fmoc-Gly-OH, Fmoc-Ser(tBu)-OH, Fmoc-Ser(tBu)-OH, Fmoc-Pro-OH, Fmoc-Gly-Gly-OH, Fmoc-Lys(Boc)-OH, Fmoc-Leu-OH, Fmoc-Leu-OH, Fmoc-Trp(Boc)-OH, Fmoc-Lys(Mtt)-OH, Fmoc-Val-OH, Fmoc-Phe-OH, Fmoc-Glu(OtBu)-OH.H₂O, Fmoc-Ala-OH.H₂O, Fmoc-Gln(Trt)-OH, Fmoc-Ala-OH.H₂O, Fmoc-Gln(Trt)-OH, Fmoc-Lys(Boc)-OH, Fmoc-Asp(OtBu)-OH, Fmoc-Leu-OH, Fmoc-Aib-OH, Fmoc-Ile-OH, Fmoc-Aib-OH, Fmoc-Tyr(tBu)-OH, Fmoc-Asp(OtBu)-OH, Fmoc-Ser(tBu)-OH, Fmoc-Thr(tBu)-OH, Fmoc-α-Me-Phe-OH, Fmoc-Thr(tBu)-OH, Fmoc-Glu(OtBu) - Gly-OH, Fmoc-Aib-OH and Boc-Tyr(tBu)-OH.

The following resin was obtained: Boc-Tyr(tBu)-Aib-Glu(OtBu)-Gly-Thr(tBu)-αMePhe-Thr(tBu)-Ser(tBu)-Asp(OtBu)-Tyr(tBu)-Aib-Ile-Aib-Leu-Asp(OtBu)-Lys(Boc)-Gln(Trt)-Ala-Gln(Trt)-Ala-Glu(OtBu)-Phe-Val-Lys(Mtt)-Trp(Boc)-Leu-Leu-Lys(Boc)-Gly-Gly-Pro-Ser(tBu)-Ser(tBu)-Gly-Ala-Pro-Pro-Pro-Ser(tBu)-Lys(Boc)-Rink Linker-Nle-MBHA resin.

When the above condensation was complete, the Mtt protecting groups were removed with 50% HFIP/DCM solution for 30 minutes. After washing and filtration, the following peptide-resin intermediate 1 was obtained:
Boc-Tyr(tBu)-Aib-Glu(OtBu)-Gly-Thr(tBu)-αMePhe-Thr(tBu)-Ser(tBu)-Asp(OtBu)-Tyr(tBu)-Aib-Ile-Aib-Leu-Asp(OtBu)-Lys(Boc)-Gln(Trt)-Ala-Gln(Trt)-Ala-Glu(OtBu)-Phe-Val- Lys-Trp(Boc)-Leu-Leu-Lys(Boc)-Gly-Gly-Pro-Ser(tBu)-Ser(tBu)-Gly-Ala-Pro-Pro-Pro-Ser(tBu)-Lys(Boc)-Rink Linker-Nle-MBHA Resin.

### (2) Modification Steps with Modifying chain

4 equivalents of Fmoc-AEEA-OH and HOBt were charged, and dissolved in an appropriate amount of DMF/DCM. Separately, 4 equivalents of DIC was charged and double diluted with DCM. The diluent was slowly added to the DMF/DCM solution, and reacted under stirring at -5 to 0°C for no less than 60 minutes so as to be activated for further use.

The activated Fmoc-AEEA-OH solution was added to the previously swollen and washed peptide-resin intermediate 1. The reaction temperature was controlled at 10 to 30°C. The coupling reaction was performed for 240 to 480 minutes. The resin was filtrated and washed. The Fmoc protecting groups were removed with 20% PIP/DMF solution for 30 minutes. The resin was filtrated and washed. According to the reaction conditions as stated above, the activated Fmoc-AEEA-OH, Fmoc-AEEA-OH, Fmoc-Glu(α-OtBu)-OH, Fmoc-Glu(α-OtBu)-OH, Fmoc-Glu(α-OtBu)-OH and mono-tert-butyl octadecanedioate were successively coupled on the resin, Fmoc deprotected, washed with DCM and dried to obtain the following P015 peptide-resin: Boc-Tyr(tBu)-Aib-Glu(OtBu)-Gly-Thr(tBu)-αMePhe-Thr(tBu)-Ser(tBu)-Asp(OtBu)-Tyr(tBu)-Aib-Ile-Aib-Leu-Asp(OtBu)-Lys(Boc)-Gln(Trt)-Ala-Gln(Trt)-Ala-Glu(OtBu)-Phe-Val-Lys(tBuO-Ste-γ-Glu(α-OtBu)-γ-Glu(α-OtBu)-γ-Glu(α-OtBu)-AEEA-AEEA)-Trp(Boc)-Leu-Leu-Lys(Boc)-Gly-Gly-Pro-Ser(tBu)-Ser(tBu)-Gly-Ala-Pro-Pro-Pro-Ser(tBu)-Lys(Boc)-Rink-Linker-Nle-MBHA Resin.

The P015 peptide-resin was charged. 12-15 mL/g cleavage reagent for the peptide-resin (TFA:EDT:TIS:H₂O, 94:2:2:2 v/v) was added and reacted with stirring at 25±5°C for 4 hours. The reaction mixture was filtered with a sand core funnel. The filtrate was collected. The resin was washed with a small amount of TFA for three times, and the filtrates were combined and concentrated under reduced pressure. Methyl tert-butyl ether (MBTE) was added, and the resulting precipitate was washed with MBTE for 3 to 4 times, and dried by evaporating MBTE. The crude product was dried under reduced pressure at room temperature to constant weight to obtain the crude P015 product, which was subsequently purified and dried to obtain the P015 sample (the molecular weight determined by MS was 5255.2).

### Example 2:

Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K;

wherein X₁ is Aib; X₂ is αMePhe; X₃ is Aib; X₄ is Aib; K at position 28 are chemically modified through conjugation to the ε-aminos of the K side-chains with ([2-(2-amino-ethoxy)-ethoxy]-acetyl)₃-(γ-Glu)₁-CO-(CH₂)₁₆-CO₂H; and the C-terminal amino acid is amidated as a C-terminal primary amide (SEQ ID NO:19).

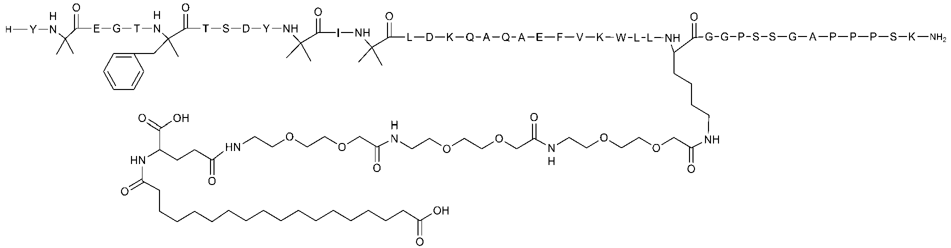

The above structure contains the standard single letter amino acid code with exception of residues Aib2, αMePhe6, Aib11, Aib13 and K28 where the structures of amino acid residues Aib2, αMePhe6, Aib11, Aib13 and K28 have been expanded.

The preparation method applies a Fmoc solid-phase polypeptide synthesis strategy, said method comprising:

### (1) Synthesis of Peptide-resin Intermediate 2

Fmoc-Rink Linker-Nle-MBHA resin (S=0.49 mmol/g) was charged, swelled with an appropriate amount of DCM, washed with DCM for 2 to 3 times, de-protected with 20% PIP/DMF solution for 30 mins, washed and filtered to obtain a NH₂-Rink linker-Nle-MBHA resin with Fmoc removed, which was suctioned to remove the solvent for further use.

4 equivalents of Fmoc-Lys(Boc)-OH and HOBt were charged respectively, and dissolve in an appropriate amount of DMF/DCM. Separately, 4 equivalents of DIC was charged and double diluted with DCM. The diluent was slowly added to the DMF/DCM solution, and reacted under stirring at -5 to 0°C for no less than 60 minutes so as to be activated for further use.

The activated Fmoc-Lys(Boc)-OH solution was added to the NH₂-Rink linker-Nle-MBHA resin. The reaction temperature was controlled at 10 to 30°C. The coupling reaction was performed for 240 to 480 minutes. After filtration and washing, a Fmoc-Lys(Boc)-Rink linker-Nle-MBHA resin was obtained. The resin was de-protected with 20% PIP/DMF solution for 30 min. After filtration and washing, a Lys(Boc)-Rink linker-Nle-MBHA resin with Fmoc removed was obtained.

According to the reaction conditions as stated above, each amino acid was successively coupled starting from the second amino acid at the C-terminus to the N-terminus. If the coupling was incomplete (a color development reaction occurs), a second condensation was carried out using HBTU/DIEA to ensure that each amino acid was completely condensed. The coupling were successively performed in the following order: Fmoc-Ser(tBu)-OH, Fmoc-Pro-OH, Fmoc-Pro-OH, Fmoc-Pro-OH, Fmoc-Ala-OH.H₂O, Fmoc-Gly-OH, Fmoc-Ser(tBu)-OH, Fmoc-Ser(tBu)-OH, Fmoc-Pro-OH, Fmoc-Gly-Gly-OH, Fmoc-Lys(Mtt)-OH, Fmoc-Leu-OH, Fmoc-Leu-OH, Fmoc-Trp(Boc)-OH, Fmoc-Lys(Boc)-OH, Fmoc-Val-OH, Fmoc-Phe-OH, Fmoc-Glu(OtBu)-OH.H₂O, Fmoc-Ala-OH.H₂O, Fmoc-Gln(Trt)-OH, Fmoc-Ala-OH.H₂O, Fmoc-Gln(Trt)-OH, Fmoc-Lys(Boc)-OH, Fmoc-Asp(OtBu)-OH, Fmoc-Leu-OH, Fmoc-Aib-OH, Fmoc-Ile-OH, Fmoc-Aib-OH, Fmoc-Tyr(tBu)-OH, Fmoc-Asp(OtBu)-OH, Fmoc-Ser(tBu)-OH, Fmoc-Thr(tBu)-OH, Fmoc-α-Me-Phe-OH, Fmoc-Thr(tBu)-OH, Fmoc-Glu(OtBu) - Gly-OH, Fmoc-Aib-OH and Boc-Tyr(tBu)-OH.

The following resin was obtained:

Boc-Tyr(tBu)-Aib-Glu(OtBu)-Gly-Thr(tBu)-αMePhe-Thr(tBu)-Ser(tBu)-Asp(OtBu)-Tyr(tBu)-
Aib-Ile-Aib-Leu-Asp(OtBu)-Lys(Boc)-Gln(Trt)-Ala-Gln(Trt)-Ala-Glu(OtBu)-Phe-Val-Lys(Boc)-
Trp(Boc)-Leu-Leu-Lys(Mtt)-Gly-Gly-Pro-Ser(tBu)-Ser(tBu)-Gly-Ala-Pro-Pro-Pro-Ser(tBu)-
Lys(Boc)-Rink Linker-Nle-MBHA Resin.

When the above condensation was complete, the Mtt protecting groups were removed with 50% HFIP/DCM solution for 30 minutes. After washing and filtration, the following peptide-resin intermediate 2 was obtained: Boc-Tyr(tBu)-Aib-Glu(OtBu)-Gly-Thr(tBu)-αMePhe-Thr(tBu)-Ser(tBu)-Asp(OtBu)-Tyr(tBu)-Aib-Ile-Aib-Leu-Asp(OtBu)-Lys(Boc)-Gln(Trt)-Ala-Gln(Trt)-Ala-Glu(OtBu)-Phe-Val-Lys(Boc)-Trp(Boc)-Leu-Leu-Lys-Gly-Gly-Pro-Ser(tBu)-Ser(tBu)-Gly-Ala-Pro-Pro-Pro-Ser(tBu)-Lys(Boc)-Rink Linker-Nle-MBHA Resin.

### (2) Modification Steps with Modifying chain

4 equivalents of Fmoc-AEEA-OH and HOBt were charged, and dissolve in an appropriate amount of DMF/DCM. Separately, 4 equivalents of DIC was charged and double diluted with DCM. The diluent was slowly added to the DMF/DCM solution, and reacted under stirring at -5 to 0°C for no less than 60 minutes so as to be activated for further use.

The activated Fmoc-AEEA-OH solution was added to the previously swollen and washed peptide-resin intermediate 2. The reaction temperature was controlled at 10 to 30°C. The coupling reaction was performed for 240 to 480 minutes. The resin was filtrated and washed. The Fmoc protecting groups were removed with 20% PIP/DMF solution for 30 minutes. The resin was filtrated and washed. According to the reaction conditions as stated above, the activated Fmoc-AEEA-OH, Fmoc-AEEA-OH, Fmoc-AEEA-OH, Fmoc-Glu(α-OtBu)-OH and mono-tert-butyl octadecanedioate were successively coupled on the resin, Fmoc deprotected, washed with DCM and dried to obtain the following P019 peptide-resin: Boc-Tyr(tBu)-Aib-Glu(OtBu)-Gly-Thr(tBu)-αMePhe-Thr(tBu)-Ser(tBu)-Asp(OtBu)-Tyr(tBu)-Aib-Ile-Aib-Leu-Asp(OtBu)-Lys(Boc)-Gln(Trt)-Ala-Gln(Trt)-Ala-Glu(OtBu)-Phe-Val-Lys(tBuO-Ste-γ-Glu(α-OtBu)-AEEA-AEEA-AEEA)-Trp(Boc)-Leu-Leu-Lys(Boc)-Gly-Gly-Pro-Ser(tBu)-Ser(tBu)-Gly-Ala-Pro-Pro-Pro-Ser(tBu)-Lys(Boc)-Rink-Linker-Nle-MBHA Resin.

The P019 peptide-resin was charged. 12-15 mL/g cleavage reagent for the peptide-resin (TFA:EDT:TIS:H₂O, 94:2:2:2 v/v) was added and reacted with stirring at 25±5°C for 4 hours. The reaction mixture was filtered with a sand core funnel. The filtrate was collected. The resin was washed with a small amount of TFA three times, and the filtrates were combined and concentrated under reduced pressure. Methyl tert-butyl ether (MBTE) was added, and the resulting precipitate was washed with MBTE for 3 to 4 times, and dried by evaporating MBTE. The crude product was dried under reduced pressure at room temperature to constant weight to obtain the crude P019 product, which was subsequently purified and dried to obtain the P019 sample (the molecular weight determined by MS was 5142.0).

### Example 3

Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K;

wherein X₁ is Aib; X₂ is αMePhe; X₃ is Aib; X₄ is Aib; K at position 24 is chemically modified through conjugation to the ε-amino of the K side-chain with ([2-(2-amino-ethoxy)-ethoxy]-acetyl)₂-(γ-Glu)₁-CO-(CH₂)₁₆-CO₂H; and the C-terminal amino acid is amidated as a C-terminal primary amide (SEQ ID NO:7).

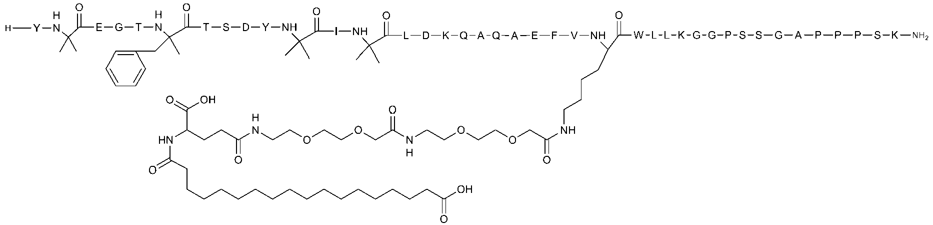

The above structure contains the standard single letter amino acid code with exception of residues Aib2, αMePhe6, Aib11, Aib13 and K24 where the structures of amino acid residues Aib2, αMePhe6, Aib11, Aib13 and K24 have been expanded.

Similar to the method described in Example 1 above, the peptide-resin intermediate 1 was used to carry out the preparation steps to synthesize the peptide of SEQ ID NO: 7 of the present invention (the molecular weight determined by MS was 4997.2). The difference was that the modification process with the modifying chain was sequentially coupling Fmoc-AEEA-OH, Fmoc-AEEA-OH, Fmoc-Glu(α-OtBu)-OH and mono-tert-butyl octadecanedioate on the resin. The resultant product was de-protected from Fmoc group, and washed with DCM to obtain P007 peptide-resin. Other steps were similar.

### Example 4

Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K;

wherein X₁ is Aib; X₂ is αMePhe; X₃ is Aib; X₄ is Aib; K at position 24 is chemically modified through conjugation to the ε-amino of the K side-chain with ([2-(2-amino-ethoxy)-ethoxy]-acetyl)₂-(γ-Glu)₁-CO-(CH₂)₁₈-CO₂H; and the C-terminal amino acid is amidated as a C-terminal primary amide (SEQ ID NO:13).

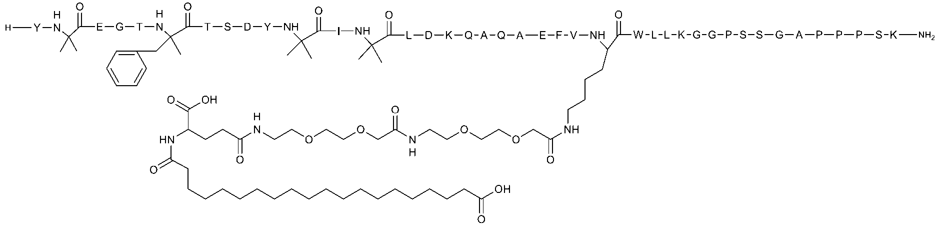

The above structure contains the standard single letter amino acid code with exception of residues Aib2, αMePhe6, Aib11, Aib13 and K24 where the structures of amino acid residues Aib2, αMePhe6, Aib11, Aib13 and K24 have been expanded.

Similar to the method described in Example 1 above, the peptide-resin intermediate 1 was used to carry out the preparation steps to synthesize the peptide of SEQ ID NO: 13 of the present invention (the molecular weight determined by MS was 5025.2). The difference was that the modification process with the modifying chain was sequentially coupling Fmoc-AEEA-OH, Fmoc-AEEA-OH, Fmoc-Glu(α-OtBu)-OH and eicosanedioic acid mono-tert-butyl ester on the resin. The resultant product was de-protected from Fmoc group, and washed with DCM to obtain P013 peptide-resin. Other steps were similar.

### Example 5:

Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K;

wherein X₁ is Aib; X₂ is αMePhe; X₃ is Aib; X₄ is Aib; K at position 24 is chemically modified through conjugation to the ε-amino of the K side-chain with ([2-(2-amino-ethoxy)-ethoxy]-acetyl)₂-(γ-Glu)₁-CO-(CH₂)₂₀-CO₂H; and the C-terminal amino acid is amidated as a C-terminal primary amide (SEQ ID NO:14).

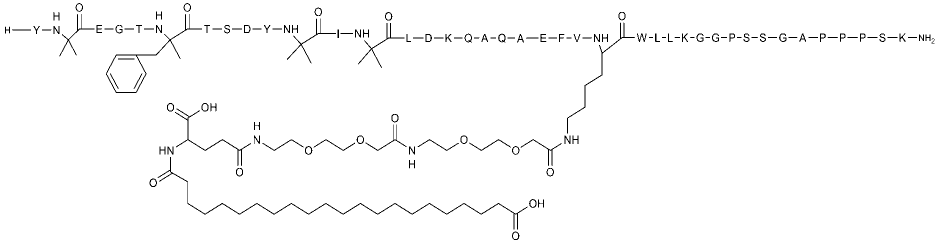

The above structure contains the standard single letter amino acid code with exception of residues Aib2, αMePhe6, Aib11, Aib13 and K24 where the structures of amino acid residues Aib2, αMePhe6, Aib11, Aib13 and K24 have been expanded.

Similar to the method described in Example 1 above, the peptide-resin intermediate 1 was used to carry out the preparation steps to synthesize the peptide of SEQ ID NO: 14 of the present invention (the molecular weight determined by MS was 5052.4). The difference was that the modification process with the modifying chain was sequentially coupling Fmoc-AEEA-OH, Fmoc-AEEA-OH, Fmoc-Glu(α-OtBu)-OH and docosanedioic acid mono-tert-butyl ester on the resin. The resultant product was de-protected from Fmoc group, and washed with DCM to obtain P014 peptide-resin. Other steps were similar.

### Example 6:

Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K;

wherein X₁ is Aib; X₂ is αMePhe; X₃ is Aib; X₄ is Aib; K at position 24 is chemically modified through conjugation to the ε-amino of the K side-chain with ([2-(2-amino-ethoxy)-ethoxy]-acetyl)₃-(γ-Glu)₁-CO-(CH₂)₂₀-CO₂H; and the C-terminal amino acid is amidated as a C-terminal primary amide (SEQ ID NO:16).

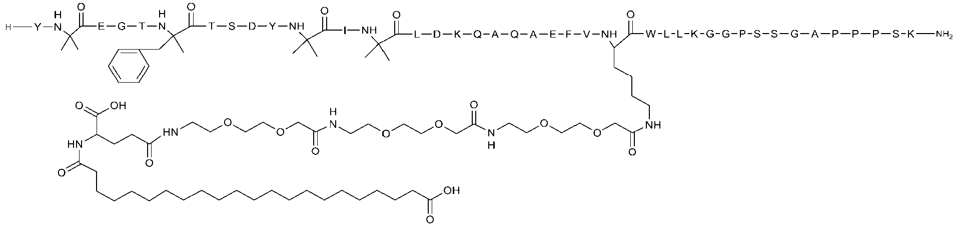

The above structure contains the standard single letter amino acid code with exception of residues Aib2, αMePhe6, Aib11, Aib13 and K24 where the structures of amino acid residues Aib2, αMePhe6, Aib11, Aib13 and K24 have been expanded.

Similar to the method described in Example 1 above, the peptide-resin intermediate 1 was used to carry out the preparation steps to synthesize the peptide of SEQ ID NO: 16 of the present invention (the molecular weight determined by MS was 5198.0). The difference was that the modification process with the modifying chain was sequentially coupling Fmoc-AEEA-OH, Fmoc-AEEA-OH, Fmoc-AEEA-OH, Fmoc-Glu(α-OtBu)-OH and docosanedioic acid mono-tert-butyl ester on the resin. The resultant product was de-protected from Fmoc group, and washed with DCM to obtain P016 peptide-resin. Other steps were similar.

### Example 7:

Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K;

wherein X₁ is Aib; X₂ is αMePhe; X₃ is Aib; X₄ is Aib; K at position 28 were chemically modified through conjugation to the ε-aminos of the K side-chains with ([2-(2-amino-ethoxy)-ethoxy]-acetyl)₂-(γ-Glu)₁-CO-(CH₂)₁₆-CO₂H; and the C-terminal amino acid is amidated as a C-terminal primary amide (SEQ ID NO:8).

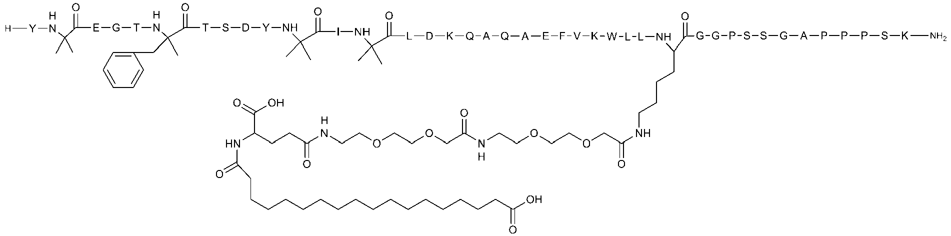

The above structure contains the standard single letter amino acid code with exception of residues Aib2, αMePhe6, Aib11, Aib13 and K28 where the structures of amino acid residues Aib2, αMePhe6, Aib11, Aib13 and K28 have been expanded.

Similar to the method described in Example 2 above, the peptide-resin intermediate 2 was used to carry out the preparation steps to synthesize the peptide of SEQ ID NO: 8 of the present invention (the molecular weight determined by MS was 4996.8). The difference was that the modification process with the modifying chain was sequentially coupling Fmoc-AEEA-OH, Fmoc-AEEA-OH, Fmoc-Glu(α-OtBu)-OH and mono-tert-butyl octadecanedioate on the resin. The resultant product was de-protected from Fmoc group, and washed with DCM to obtain P008 peptide-resin. Other steps were similar.

### Example 8:

Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K;

wherein X₁ is Aib; X₂ is αMePhe; X₃ is Aib; X₄ is Aib; K at position 28 were chemically modified through conjugation to the ε-aminos of the K side-chains with ([2-(2-amino-ethoxy)-ethoxy]-acetyl)₂-(γ-Glu)₁-CO-(CH₂)₂₀-CO₂H; and the C-terminal amino acid is amidated as a C-terminal primary amide (SEQ ID NO: 17).

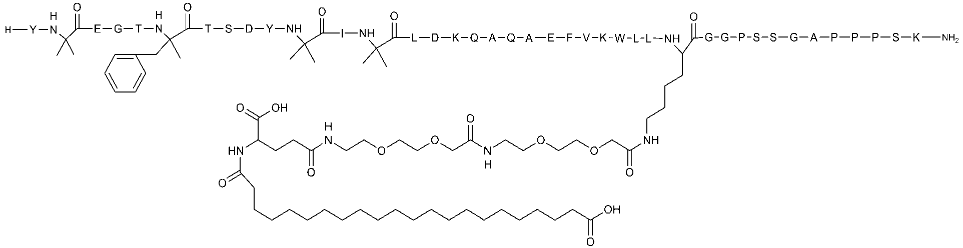

The above structure contains the standard single letter amino acid code with exception of residues Aib2, αMePhe6, Aib11, Aib13 and K28 where the structures of amino acid residues Aib2, αMePhe6, Aib11, Aib13 and K28 have been expanded.

Similar to the method described in Example 2 above, the peptide-resin intermediate 2 was used to carry out the preparation steps to synthesize the peptide of SEQ ID NO: 17 of the present invention (the molecular weight determined by MS was 5052.8). The difference was that the modification process with the modifying chain was sequentially coupling Fmoc-AEEA-OH, Fmoc-AEEA-OH, Fmoc-Glu(α-OtBu)-OH and docosanedioic acid mono-tert-butyl ester on the resin. The resultant product was de-protected from Fmoc group, and washed with DCM to obtain P017 peptide-resin. Other steps were similar.

### Example 9:

Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K;

wherein X₁ is Aib; X₂ is αMePhe; X₃ is Aib; X₄ is Aib; K at position 28 were chemically modified through conjugation to the ε-aminos of the K side-chains with ([2-(2-amino-ethoxy)-ethoxy]-acetyl)₂-(γ-Glu)₃-CO-(CH₂)₁₆-CO₂H; and the C-terminal amino acid is amidated as a C-terminal primary amide (SEQ ID NO:18).

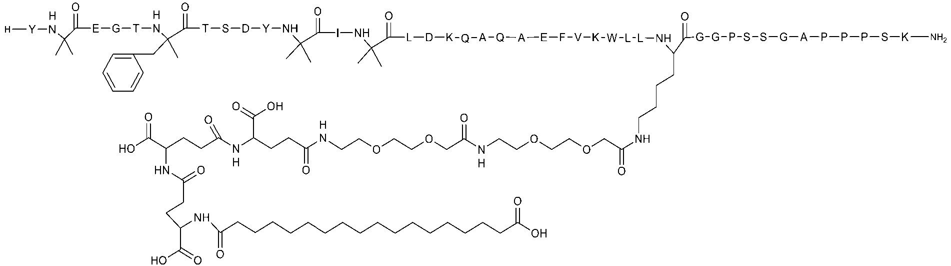

The above structure contains the standard single letter amino acid code with exception of residues Aib2, αMePhe6, Aib11, Aib13 and K28 where the structures of amino acid residues Aib2, αMePhe6, Aib11, Aib13 and K28 have been expanded.

Similar to the method described in Example 2 above, the peptide-resin intermediate 2 was used to carry out the preparation steps to synthesize the peptide of SEQ ID NO: 18 of the present invention (the molecular weight determined by MS was 5255.2). The difference was that the modification process with the modifying chain was sequentially coupling Fmoc-AEEA-OH, Fmoc-AEEA-OH, Fmoc-Glu(α-OtBu)-OH, Fmoc-Glu(α-OtBu)-OH , Fmoc-Glu(α-OtBu)-OH and mono-tert-butyl octadecanedioate on the resin. The resultant product was de-protected from Fmoc group, and washed with DCM to obtain P018 peptide-resin. Other steps were similar.

### Example 10:

Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K; wherein X₁ is Aib; X₂ is αMePhe; X₃ is Aib; X₄ is Aib; K at position 28 were chemically modified through conjugation to the ε-aminos of the K side-chains with ([2-(2-amino-ethoxy)-ethoxy]- acetyl)₃-(γ-Glu)₁-CO-(CH₂)₂₀-CO₂H; and the C-terminal amino acid is amidated as a C-terminal primary amide (SEQ ID NO:20).

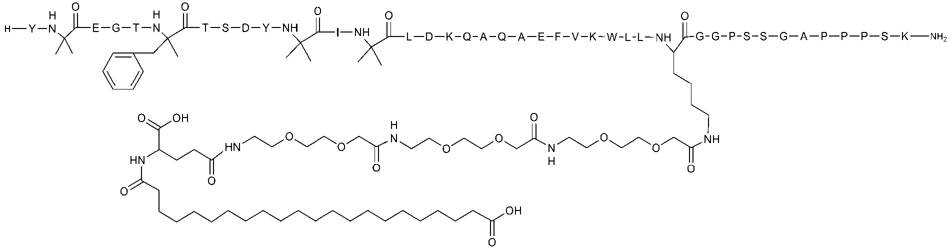

The above structure contains the standard single letter amino acid code with exception of residues Aib2, αMePhe6, Aib11, Aibl3 and K28 where the structures of amino acid residues Aib2, αMePhe6, Aib11, Aib13 and K28 have been expanded.

Similar to the method described in Example 2 above, the peptide-resin intermediate 2 was used to carry out the preparation steps to synthesize the peptide of SEQ ID NO: 20 of the present invention (the molecular weight determined by MS was 5198.4). The difference was that the modification process with the modifying chain was sequentially coupling Fmoc-AEEA-OH, Fmoc-AEEA-OH, Fmoc-AEEA-OH, Fmoc-Glu(α-OtBu)-OH and docosanedioic acid ester mono-tert-butyl on the resin. The resultant product was de-protected from Fmoc group, and washed with DCM to obtain P020 peptide-resin. Other steps were similar.

In some embodiments of the present invention, as for the preparation of the peptide-resin intermediate 3 (a peptide-resin intermediate wherein the modifying chain was bonded to the Lys at position 16), that was Boc-Tyr(tBu)-Aib-Glu(OtBu)-Gly-Thr(tBu)-αMePhe-Thr(tBu)-Ser(tBu)-Asp(OtBu)-Tyr(tBu)-Aib-Ile-Aib-Leu-Asp(OtBu)-Lys-Gln(Trt)-Ala-Gln(Trt)-Ala-Glu(OtBu)-Phe-Val-Lys(Boc)-Trp(Boc)-Leu-Leu-Lys(Boc)-Gly-Gly-Pro-Ser(tBu)-Ser(tBu)-Gly-Ala-Pro-Pro-Pro-Ser(tBu)-Lys(Boc)-Rink Linker-Nle-MBHA Resin, the preparation method for peptide-resin intermediate 1 in Example 1 was referred to.

As for the preparation of the peptide-resin intermediate 4 (a peptide-resin intermediate wherein the modifying chain was bonded to the Lys at position 40), that was Boc-Tyr(tBu)-Aib-Glu(OtBu)-Gly-Thr(tBu)-αMePhe-Thr(tBu)-Ser(tBu)-Asp(OtBu)-Tyr(tBu)-Aib-Ile-Aib-Leu-Asp(OtBu)-Lys(Boc)-Gln(Trt)-Ala-Gln(Trt)-Ala-Glu(OtBu)-Phe-Val-Lys(Boc)-Trp(Boc)-Leu-Leu-Lys(Boc)-Gly-Gly-Pro-Ser(tBu)-Ser(tBu)-Gly-Ala-Pro-Pro-Pro-Ser(tBu)-Lys-Rink Linker-Nle-MBHA Resin, the preparation method for peptide-resin intermediate 1 in Example 1 was referred to.

Other dual agonist compounds in the present invention can be prepared according to the methods described above.

### Related Assays

Provided below were the conditions and data in several assays for Examples.

### I. IN VITRO FUNCTION

### (I) In Vitro Binding Activity to Human GlP-1 and GIP Receptors

The in vitro binding potency of compounds according to the present invention to human GIP and GLP-1 receptors was evaluated by measuring the binding affinities Ki, using crude cellular membranes obtained from clonal cell lines over-expressing either the human GLP1R cDNA or human GIP-R cDNA.
1) In Vitro Binding Activity to Human GLP-1 Receptor
   hGLP-1 and the compound of the present invention were dissolved in DMSO and stored at -80°C. 89 µL of membrane dissolved in binding buffer (50 mM Hepes, pH 7.4, 5 mM MgCl₂ , 5 mM EDTA, 0.005% TWEEN, 0.005% HSA) was transferred to a 96-well test plate (5 µg/well). The compound was serially diluted in DMSO, and then, 1 µL of the diluted compound or 100% DMSO was added to the test plate containing the membrane solution. 10 µL of [¹²⁵I] GLP-1 was further added (the final concentration in the reaction was 0.15 nM). The test plate was left at room temperature for 90 minutes. The membrane complexes were harvested into a GF/B plate pre-coated with 0.5% PEI by using a Cell Harvester, and washed for 3 times with 500 µL of pre-cooled elution buffer (50 mM Hepes, pH 7.4, 500 mM NaCl) at 4°C. After drying at 37°C for 2 hours, 50 µL of scintillation fluid was added to each well. The plate was read with a MicroBeta2 scintillation counter after sealing and settling for at least 1 hour so as to determine the membrane bound radioligand level.

The absolute IC₅₀ concentration of the compound was derived by non-linear regression of the binding percent of [¹²⁵I]GLP-1 *versus* the concentration of the compound added. The IC₅₀ concentration was converted to Ki (Ki was the inhibition constant) using the Cheng-Prusoff equation.

### 2) In Vitro Binding Activity to Human GIP Receptor

hGIP and the compound of the present invention were dissolved in DMSO and stored at -80°C. 98 µL of membrane dissolved in binding buffer (50 mM HEPES pH 7.4, 5 mM MgCl₂, 1 mM CaCl₂, 0.1% BSA, 0.005% Tween-20) was transferred to a 96-well test plate (15 µg/well). The compound was serially diluted in DMSO, and then, 2 µL of the diluted compounds or 100% DMSO was added to the test plate containing the membrane solution. 100 µL of [¹²⁵I]GIP was further added (the final concentration in the reaction was 0.0315 nM). The test plate was left at room temperature for 90 minutes. The membrane complexes were harvested into a GF/B plate pre-coated with 0.5% PEI by using a Cell Harvester, and washed for 3 times with 500 µL of pre-cooled elution buffer (50 mM Tris-HCl pH 7.4, 125 mM NaCl) at 4°C. After drying at 37°C for 2 hours, 50 µL of scintillation fluid was added to each well. The plate was read with a MicroBeta2 scintillation counter after sealing and settling for at least 1 hour so as to determine the membrane bound radioligand level.

The absolute IC₅₀ concentration of the compound was derived by non-linear regression of the binding percent of [¹²⁵I]GIP *versus* the concentration of the compound added. The IC₅₀ concentration was converted to Ki (Ki was the inhibition constant) using the Cheng-Prusoff equation.

**Table 1. Receptor Binding Affinity, Ki Ratio**

| Compound | GLP-1R | GIPR |
|---|---|---|
| | Ki ratio | Ki ratio |
| GLP-1 | 1.00 | NA |
| GIP | NA | 1.00 |
| P001 | 19.52 | 8.82 |
| Tirzepatide | 0.28 | 0.06 |
| P007 | 0.47 | NA |
| P008 | 1.09 | 0.16 |
| P013 | 0.28 | 0.14 |
| P014 | 0.37 | 0.08 |
| P015 | 0.17 | NA |
| P016 | 0.63 | 0.11 |
| P017 | 0.56 | 0.04 |
| P018 | 1.45 | 0.05 |
| P019 | 0.93 | 0.09 |
| P020 | 1.36 | 0.05 |

| | | |
|---|---|---|
| NA: not detected Ki ratio: the ratio of the Ki value of the endogenous ligand to the Ki value of the test compound | | |

The binding affinities of all compounds to the receptors were expressed by Ki ratios, and a larger Ki ratio of the compound indicates a stronger binding affinity. As compared with P001, the binding affinities of the compounds according to the present invention to both human GLP-1R and GIPR were decreased to a certain extent (see Table 1).

As compared with Tirzepatide, the binding affinities of the other 9 compounds according to the present invention, except for P015, to human GLP-1R were slightly stronger than that of Tirzepatide. In addition, the binding affinities of the five compounds, namely, P008, P013, P014, P016 and P019, to GIPR were also slightly stronger than that of Tirzepatide (see Table 1).

To sum up, the binding affinities to human GLP-1R and GIPR of the compounds with different modifying chains according to the present invention were lower than that of P001 in some degree, but were slightly stronger than or equivalent to that of Tirzepatide.

### (II) Agonistic Activity on hGLP-1R and hGIPR

For compounds according to the present invention, the in vitro functional activities on human GIP and GLP-1 receptors were determined in HEK-293 clonal cell lines expressing these receptors.

### 1) Agonistic Activity on Human GLP-1 Receptor (cAMP Reporter Gene Assay)

The agonistic activities of the compounds according to the present invention on human GLP-1R were determined by cAMP reporter gene assay, and P001, Tirzepatide and endogenous ligand GLP-1 were used as references.

HEK293/CRE/GLP-1R cells were inoculated in a 96-well plate at 50,000 cells/well (80 µL/well), and incubated overnight in a 37°C, 5% CO₂ incubator. 20 µL/well of the test medium (DMEM containing 0.1% casein) containing a compound (the compound according to the present invention, P001, Tirzepatide or GLP-1) was added to the 96-well plate, and continued to incubate in a 37°C, 5% CO₂ incubator for 6 hours and equilibrated to room temperature. The supernatant was removed. 50 µL/well Bright-Glo reagent was added, shaked and lysed at room temperature for 10 minutes. The luminescence was read using an Envision microplate reader, and the luciferase activity was measured.

The response value to 100 nM GLP-1 was set as 100% response value, and GraphPad was used to perform nonlinear regression according to the response percentage and the concentration of the compound added to obtain the EC₅₀ value for each compound.

### 2) Agonistic Activity on Human GIP Receptor (LANCE Ultra cAMP Assay)

The agonistic activities of the compounds according to the present invention on human GIPR were determined by a LANCE Ultra cAMP Kit, and P001, Tirzepatide and endogenous ligand GIP were used as references.

The in vitro agonistic activities of the compounds according to the present invention on GIPR was determined in HEK293 cells stably expressing human GIPR (HEK293/GIPR cells). HEK293/GIPR cells were formulated in HBSS buffer (0.1% Casein, 500 µM IBMX, 5 mM HEPES), and inoculated into a 384-well cell culture plate at 1000 cells/well (5 µL/well). 5 µL of HBSS buffer containing 2× compounds was added to the above-mentioned 384-well cell culture plate. After been sealed, the plate was placed into a 37°C, 5% CO₂ incubator for about 30 minutes. After the incubation was completed, 5 µL of cAMP-Eu working solution and 5 µL of cAMP-Ulight working solution were added sequentially, and the plate was shaken for uniform mixing. The incubation was performed for 1 hour at 25°C. The signal values at 665 nm and 615 nm were read on an Envision microplate reader. The ratio of the signal value at 665 nm to the signal value at 615 was calculated, and converted into a cAMP concentration by using the cAMP standard curve. The response value to 1 µM GIP was set as 100% response value, and GraphPad was used to perform nonlinear regression according to the response percentage and the concentration of the compound added to obtain the EC₅₀ value for each compound.

**Table 2. Agonist Activity on Human GLP-1 and GIP Receptors, EC₅₀ Values**

| Compound | GLP-1R | GIPR |
|---|---|---|
| | EC₅₀ value | EC₅₀ value |
| GLP-1 | 1.00 | NA |
| GIP | NA | 1.00 |
| P001 | 52.09 | 7.13 |
| Tirzepatide | 0.74 | 0.51 |
| P007 | 5.84 | 1.15 |
| P008 | 4.79 | 1.67 |
| P013 | 2.41 | 0.86 |
| P014 | 1.73 | 0.25 |
| P015 | 2.44 | 0.17 |
| P016 | 1.56 | 0.56 |
| P017 | 1.35 | 0.16 |
| P018 | 3.63 | 2.55 |
| P019 | 4.95 | 1.68 |
| P020 | 1.41 | 0.22 |

| | | |
|---|---|---|
| NA: not detected EC₅₀ value: the ratio of the EC₅₀ value of the endogenous ligand to the EC₅₀ value of the test compound | | |

The agonistic activity of all compounds against human GLP-1 and GIP receptors was expressed by EC₅₀ value, and the larger the EC₅₀ value, the stronger the activity of the compound. As compared with P001, the compound according to the present invention shows a certain degree of decrease in the agonistic activity on human GLP-1R and GIPR (see Table 2).

As compared with Tirzepatide, all compounds according to the present invention have stronger agonistic activity on human GLP-1R. In addition, the agonistic activity of the six compounds, namely, P007, P008, P013, P016, P018 and P019, on human GIPR was also slightly stronger than that of Tirzepatide (see Table 2).

To sum up, the agonistic activity on human GLP-1R and GIPR of the compounds with different modifying chains according to the present invention was lower than that of P001 in some degree, but 6 compounds according to the present invention were stronger than Tirzepatide in terms of agonistic activity on human GLP-1R and GIPR.

As shown by in vitro function assay, since the 10 compounds according to the present invention do not differ significantly in activity, the compounds were further screened using a single administration hypoglycaemic assay in db/db mice.

### (III) Agonistic activity on GLP-1R and GIPR of different species

For human, cat, dog, mouse, rat, rabbit, and monkey GLP-1 and GIP receptors, the in vitro functional activities of the compounds according to the present invention on these receptors were determined in cells stably expressing these receptors and in CHO-K1 cells transiently transfecting these receptors.

### 1) Agonistic activity on human, dog, mouse, rat, rabbit, and monkey GLP-1 receptors (LANCE Ultra cAMP assay)

The LANCE Ultra cAMP Kit was used to determine the agonistic activity of the compound of the present invention on human, dog, mouse, rat, rabbit, and monkey GLP-1R, and the endogenous ligand GLP-1 was used as a reference.

The in vitro agonistic activity of the compound of the present invention on GLP-1R was measured in cells stably expressing human, dog, mouse, rat, rabbit, and monkey GLP-1R (human GLP-1R-HEK, dog GLP-1R-HEK, mouse GLP-1R-HEK, rat GLP-1R- in HEK, rabbit GLP-1R-CHO, monkey GLP-1R-HEK cells). Human GLP-1R-HEK, dog GLP-1R-HEK, mouse GLP-1R-HEK, rat GLP-1R-HEK, rabbit GLP-1R-CHO, and monkey GLP-1R-HEK cells were resuspended in 1×Casein Stimulation Buffer (HBSS, 5 mM HEPES, 0.5 mM IBMX, 0.1% casein, pH7.4), and seeded into a 384-well cell culture plate at 9 µL/well (cell numbers being 1000, 1000, 1000, 1500, 1000, 1000 cells/well, respectively). 1 µL of 1×Casein Stimulation Buffer containing 10× compound was added to the above-mentioned 384-well cell culture plate. The plate was incubated at 37 °C for 30 minutes. After the incubation was completed, 5 µL of Eu-cAMP working solution and 5 µL of Ulight^{TM}-cAMP working solution were added sequentially, centrifuged and then incubated at room temperature for 1 hour. The readings at 665 nm and 620 nm were determined with a 330 nm excitation light on a microplate reader. The 665 nm/620 nm reading ratio was calculated. The response value at the maximum concentration of GLP-1 was set as 100% response value. GraphPad was used to perform nonlinear regression according to the response percentage and the concentration of the compound added to obtain the EC₅₀ value for each compound.

### 2) Agonistic activity on human, dog, mouse, rat, rabbit, and monkey GIP receptors (LANCE Ultra cAMP assay)

The LANCE Ultra cAMP Kit was used to determine the agonistic activity of the compound of the present invention on human, dog, mouse, rat, rabbit, and monkey GIPR, and the endogenous ligand GIP was used as a reference.

CHO-K1 cells were seeded in a 6-well plate at 0.6×10⁶ cells/well and incubated overnight in an incubator at a temperature of 37 °C and a carbon dioxide concentration of 5%. The next day, CHO-K1 cell media were replaced. Then two tubes A and B were prepared for each plasmid. 100 µL Opti-MEM was added to tube A, then 4 µL Lipofectamine^{™} 3000 was added and mixed uniformly. 100 µL Opti-MEM was firstly added to tube B, then 2 µg of one of the plasmids (human GIPR-pcDNA5 (+) plasmid, dog GIPR-pcDNA5 (+) plasmid, mouse GIPR-pcDNA5 (+) plasmid, rat GIPR-pcDNA5 (+) plasmid, rabbit GIPR-pcDNA5 (+) plasmid, monkey GIPR-pcDNA5 (+) plasmid) was added and mixed uniformly, and 4 µL P3000^{™} was added to tube B and mixed uniformly (the ratio of plasmid to transfection reagent was 1 µg: 2 µL). The diluted solution in tube A was added to the diluted solution in tube B, mixed evenly, and incubated at room temperature for 15 minutes. Finally, the mixture was gently added to the cells whose media had been replaced, shaked gently to mix, and then incubated overnight in an incubator at a temperature of 37 °C and a carbon dioxide concentration of 5%. Cells were used for compound functional activity assay 18-20 hours after transfection.

18-20 hours after transfection, human GIPR-CHO, dog GIPR-CHO, mouse GIPR-CHO, rat GIPR-CHO, rabbit GIPR-CHO, and monkey GIPR-CHO cells were resuspended in 1×Casein Stimulation Buffer (HBSS, 5 mM HEPES, 0.5 mM IBMX, 0.1% casein, pH7.4), and seeded into a 384-well cell culture plate at 9 µL/well (cell numbers being 1000, 2000, 2000, 2000, 2000, 2000 cells/well, respectively). 1 µL of 1×Casein Stimulation Buffer containing 10× compound was added to the above-mentioned 384-well cell culture plate. The plate was incubated at 37°C for 30 minutes. After the incubation was completed, 5 µL of Eu-cAMP working solution and 5 µL of Ulight^{TM}-cAMP working solution were added sequentially, centrifuged and then incubated at room temperature for 1 hour. The readings at 665 nm and 620 nm were determined with a 330 nm excitation light on a microplate reader. The 665 nm/620 nm reading ratio was calculated. The response value of the maximum concentration of GIP was set as 100% response value. GraphPad was used to perform nonlinear regression according to the response percentage and the concentration of the compound added to obtain the EC₅₀ value for each compound.

For human and cat GLP-1 and GIP receptors, the in vitro functional activities of the compounds according to the present invention on these receptors were determined in CHO cells transiently transfecting these receptors.

### 3) Agonistic activity on human and cat GLP-1 and GIP receptors (LANCE Ultra cAMP assay)

The LANCE Ultra cAMP Kit was used to determine the agonistic activity of the compound of the present invention against human and cat GLP-1 and GIP receptors, and the endogenous ligands GLP-1R and GIP were used as references.

CHO-K1 cells were seeded in a 6-well plate at 0.6×10⁶ cells/well and incubated overnight in an incubator at a temperature of 37 °C and a carbon dioxide concentration of 5%. The next day, CHO-K1 cell media were replaced. Then two tubes A and B were prepared for each plasmid. 100 µL Opti-MEM was added to tube A, then 4 µL Lipofectamine^{™} 3000 was added and mixed uniformly. 100 µL Opti-MEM was firstly added to tube B, then 2 µg of one of the plasmids (human GLP-1R-pcDNA5 (+) plasmid, cat GLP-1R-pcDNA5 (+) plasmid, human GIPR-pcDNA5 (+) plasmid, cat GIPR-pcDNA5 (+) plasmid) was added and mixed uniformly, and 4 µL P3000^{™} was added to tube B and mixed uniformly (the ratio of plasmid to transfection reagent was 1 µg: 2 µL). The diluted solution in tube A was added to the diluted solution in tube B, mixed evenly, and incubated at room temperature for 15 minutes. Finally, the mixture was gently added to the cells whose media had been replaced, shaked gently to mix, and then incubated overnight in an incubator at a temperature of 37 °C and a carbon dioxide concentration of 5%. Cells were used for compound functional activity assay 18-20 hours after transfection.

18-20 hours after transfection, human GLP-1R-CHO, cat GLP-1R-CHO, human GIPR-CHO, cat GIPR-CHO cells were resuspended in 1×Casein Stimulation Buffer (HBSS, 5 mM HEPES, 0.5 mM IBMX, 0.1% casein, pH7.4), and seeded into a 384-well cell culture plate at 9 µL/well (cell numbers being 2000, 2000, 1000, 2000 cells/well, respectively). 1 µL of 1×Casein Stimulation Buffer containing 10× compound was added to the above-mentioned 384-well cell culture plate. The plate was incubated at 37°C for 30 minutes. After the incubation was completed, 5 µL of Eu-cAMP working solution and 5 µL of Ulight^{TM-}cAMP working solution were added sequentially, centrifuged and then incubated at room temperature for 1 hour. The readings at 665 nm and 620 nm were determined with a 330 nm excitation light on a microplate reader. The 665 nm/620 nm reading ratio was calculated. The response value of the maximum concentration of GLP-1R or GIP was set as 100% response value. GraphPad was used to perform nonlinear regression according to the response percentage and the concentration of the compound added to obtain the EC₅₀ value for each compound.

**Table 3. Agonistic activity on GLP-1Rs and GIPRs of different species, EC₅₀ ratio**

| | Human | Cat | Dog | Mouse | Rat | Rabbit | Monkey |
|---|---|---|---|---|---|---|---|
| Compound | GLP-1R EC₅₀ ratio | | | | | | |
| GLP-1 | 1.00 | 1.09 | 4.09 | 3.22 | 8.04 | 2.53 | 2.19 |
| P016 | 1.00 | 2.98 | 4.52 | 1.09 | 1.59 | 1.05 | 2.66 |
| Tirzepatide | 1.00 | 31.4 | 84.84 | 0.57 | 1.21 | 0.68 | 3.31 |

| Compound | GIPR EC₅₀ ratio | | | | | | |
|---|---|---|---|---|---|---|---|
| GIP | 1.00 | 5.61 | 5.58 | 68.74 | 2.38 | 7.18 | 14.38 |
| P016 | 1.00 | 3.59 | 2.88 | 12.49 | 2.22 | 5.34 | 5.77 |
| Tirzepatide | 1.00 | 8.05 | 6.13 | 220.87 | 9.53 | 16.04 | 7.38 |

| | | | | | | | |
|---|---|---|---|---|---|---|---|
| EC₅₀ value: the ratio of EC₅₀ value of other species to human EC₅₀ value | | | | | | | |

The agonistic activities of all compounds on GLP-1 receptors and GIP receptors were expressed as the EC₅₀ ratio. The smaller the EC₅₀ value, the stronger the activity of the compound.

Compared with the agonistic activity on human receptors, the agonistic activities of compound P016 according to the present invention on mouse, rat, rabbit, cat and monkey GLP-1R were comparable to its agonistic activity on human GLP-1R, and the agonistic activity on dog GLP-1R was slightly weaker (4.52 times weaker). The agonistic activities of P016 on rat and dog GIPR were comparable to that on human GIPR, and the agonistic activities on cat, rabbit, monkey, and mouse GIPR were slightly weaker (3.59, 5.34, 5.77 and 12.49 times weaker, respectively). The agonistic activities of Tirzepatide on mouse, rat, rabbit and monkey GLP-1Rs were comparable to that on human GLP-1R, but its agonistic activities on cat and dog GLP-1Rs were >30 times or >80 times weaker than that on human GLP-1R (i.e., agonistic activity being less than 1/30 or less than 1/80). Tirzepatide's agonistic activities on dog, monkey, cat, rat and rabbit GIPR were slightly weaker than that on human GIPR (6.13, 7.38, 8.05, 9.53 and 16.04 times weaker, respectively). Tirzepatide's agonistic activity on mouse GIPR was >220 times weaker than that on human GIPR (i.e., agonistic activity was less than 1/220) (see Table 3).

In summary, the compound of the present invention has strong agonistic activities on cat, dog, mouse, rat, rabbit and monkey GLP-1R and GIPR, which were comparable to the agonistic activities on human GLP-1R and GIPR (1.05-12.5 times weaker). It can be seen that cats, dogs, mice, rats, rabbits and monkeys were non-human relevant species of the compound of the present invention. The agonistic activities of Tirzepatide on the tested species vary greatly, especially the agonistic activities of Tirzepatide on cat and dog GLP-1R were much weaker than its agonistic activity on human GLP-1R.

### 4) Sequence Alignment of GLP-1R and GIPR of different species

GLP-1R/GIPR of cat, dog, mouse, rat, rabbit, monkey, pig, alpaca, horse, sheep, and bovine were compared with sequences of human GLP-1R/GIPR by using NCBI (National Center for Biotechnology Information) BLAST. It was found that the homology between GLP-1R/GIPR of these species and human GLP-1R/GIPR was >80%. Combining the results in Table 3, it was predicted that the agonistic effects of the compound of the present invention on the GLP-1R/GIPR of pig, alpaca, horse, sheep, and bovine were comparable to or slightly weaker than that on human GLP-1R/GIPR. It was speculated that the compound of the present invention has a therapeutic function on type II diabetes melitus, obesity and other related diseases in animals of these species.

**Table 4. Homology alignment result of GLP-1R and GIPR of different species with human GLP-1R/GIPR**

| | Human | Cat | Dog | Mouse | Rat | Rabbit |
|---|---|---|---|---|---|---|
| GLP-1R | 100% | 92.22% | 89.60% | 92.01% | 90.93% | 92.44% |
| GIPR | 100% | 85.44% | 88.76% | 80.98% | 80.98% | 92.44% |
| | Cynomolgus Monkey | pig | alpaca | horse | sheep | bovine |
| GLP-1R | 98.06% | 93.09% | 91.76% | 90.93% | 91.36% 84.02% | 91.36% |
| GIPR | 97.42% | 81.89% | 84.88% | 89.19% | 81.89% | 81.68% |
| | | | | | | 81.68% |
| | | 87.61% | | | | 87.39% |
| | | 90.61% | | | | 84.23% |

| | | | | | | |
|---|---|---|---|---|---|---|
| Note: sheep GLP-1R, pig GIPR, and bovine GIPR have 2, 3 and 4 transcripts, respectively. | | | | | | |

### II. Pharmacokinetics

### (I) Pharmacokinetics Following Single Administration in SD Rat

After subcutaneous (s.c.) or intravenous (i.v.) administration to SD rats, plasma was collected and the concentration of the drug in plasma was detected to demonstrate the in vivo pharmacokinetic properties of the compound according to the present invention. The compound was dissolved in a phosphate buffer, and filtered through a membrane filter (PTFE, 0.45 µm) to obtain a 25 nmol/mL solution of the compound. The compound was administrated to male SD rats (230-268 g, n=3) at a dose of 50.0 nmol/kg (s.c.) or 25.0 nmol/kg (i.v.). About 150 µL of whole blood was collected from the internal jugular vein into an EDTA-K₂ anticoagulant tube at predose, 0.0833 (only for i.v.), 0.25, 0.5, 1, 2, 4, 6, 8, 24, 48, 72, 96 and 120 hours respectivley. The blood samples were centrifuged at 1500 g at 4°C for 10 minutes to obtain plasma, which was stored at -90°C to -60°C for further analysis and assay.

### Sample Treatment

### (1) Method for Treating the Samples for Tirzepatide, P007, P008, P013 and P014

30.0 µL of thawed plasma sample was taken. 150 µL of acetonitrile solution (containing 5 ng·mL⁻¹ Verapamil , 50 ng·mL⁻¹ Glibenclamide, 200 ng·mL⁻¹ Tolbutamide and 200 ng·mL⁻¹ Diclofenac) were added to precipitate protein. The mixture was vortexed for 5 minutes, and centrifuged at 3700 rpm for 8 minutes. 70.0 µL of the supernatant was taken, into which 70.0 µL of 0.5% aqueous formic acid solution was added. The mixture was vortexed for 5 minutes. 15 µL of the mixture solution was sampled and injected into the LC-MS/MS to determine the drug concentration in plasma.

### (2) Method for Treating the Samples for P015, P016, P017 and P018

20.0 µL of thawed plasma sample was taken. 60 µL of acetonitrile solution (containing 5 ng·mL⁻¹ Verapamil, 50 ng·mL⁻¹ Glibenclamide, 200 ng·mL⁻¹ Tolbutamide and 200 ng·mL⁻¹ Diclofenac) were added to precipitate protein. The mixture was vortexed for 1 minute, and centrifuged at 13000 rpm for 8 minutes. 60.0 µL of the supernatant was taken, into which 60.0 µL of 0.5% aqueous formic acid solution was added. The mixture was vortexed for 10 minutes. 10.0 µL of the mixture solution was sampled and injected into the LC-MS/MS to determine the drug concentration in plasma.

### (3) Method for Treating the Samples for P019 and P020

30.0 µL of thawed plasma sample was taken. 150 µL of acetonitrile solution (containing 5 ng·mL⁻¹ Verapamil, 50 ng·mL⁻¹ Glibenclamide, 200 ng·mL⁻¹ Tolbutamide and 200 ng·mL⁻¹ Diclofenac) were added to precipitate protein. The mixture was vortexed for 5 minutes, and centrifuged at 3700 rpm for 8 minutes. 70.0 µL of the supernatant was taken, into which 70.0 µL of 0.5% aqueous formic acid solution was added. The mixture was vortexed for 5 minutes. 10.0 µL of the mixture solution was sampled and injected into the LC-MS/MS to determine the drug concentration in plasma.

Based on the drug concentration in plasma of the compounds in SD rats, the pharmacokinetic parameters were calculated by applying the software Winnolin 8.2 in a non-compartmental model, and the results were shown in Table 5 and Table 6 below.

**Table 5 Pharmacokinetic Parameters after Subcutaneous Injection of Different Polypeptide Compounds to Rats**

| PK | Unit | Tirzepatide | P007 | P008 | P013 | P014 |
|---|---|---|---|---|---|---|
| parameters | | | | | | |
| Dosage | nmol/kg | 50 | 50 | 50 | 50 | 50 |
| T_{1/2} | h | 11.4±1.79 | 15.0±2.89 | 18.7±4.82 | 15.0±0.737 | 14.8±0.503 |
| Tₘₐₓ | h | 7.33±1.15 | 7.33±1.15 | 13.3±9.24 | 24.0±0 | 24.0±0 |
| Cₘₐₓ | nM | 128±42.8 | 169±14.7 | 149±20.5 | 142±28.2 | 170±16.4 |
| AUC₀₋ₜ | nM·h | 3610±1200 | 6150±776 | 5400±1030 | 5740±1070 | 6230±651 |
| AUC_{0-inf} | nM·h | 3670±1190 | 6630±439 | 6680 | 6080±1180 | 6620±712 |
| MRT₀₋ₜ | h | 19.0±0.265 | 21.4±1.87 | 21.0±1.85 | 26.2±0.700 | 26.2±0.781 |
| MRT_{0-inf} | h | 20.3±0.755 | 25.7±1.69 | 25.4 | 29.9±1.33 | 30.0±1.01 |

**Table 6 Pharmacokinetic Parameters after Subcutaneous Injection of Different Polypeptide Compounds to Rats**

| PK parameters | Unit | P015 | P016 | P017 | P018 | P019 | P020 |
|---|---|---|---|---|---|---|---|
| Dosage | nmol/ kg | 50 | 50 | 50 | 50 | 50 | 50 |
| T_{1/2} | h | 16.1±1.21 | 14.1±0.153 | 18.7±2.69 | 18.3±6.77 | 10.9±1.92 | 17.9±0.57 |
| Tₘₐₓ | h | 24.0±0 | 24.0±0 | 24.0±0 | 24.0±0 | 13.3±9.24 | 24.0±0 |
| Cₘₐₓ | nM | 213±21.3 | 119±7.00 | 112±15.5 | 175±45.1 | 302±89.9 | 139±28.1 |
| AUC₀₋ₜ | nM·h | 8830±862 | 4840±270 | 4190±242 | 7490±1360 | 7910±545 | 5620±1690 |
| AUC_{0-inf} | nM·h | 9490±1090 | 5070±262 | 4530±28.28 | 7940±1230 | 8630±601 | 7070±28.3 |
| MRT₀₋ₜ | h | 26.8±0.557 | 28.1±1.12 | 27.9±2.99 | 28.5±4.45 | 17.1±2.55 | 29.5±4.68 |
| MRT_{0_inf} | h | 31.4±1.35 | 31.1±1.00 | 36.9±3.46 | 33.4±7.00 | 18.3±2.76 | 37.1±0.636 |

The pharmacokinetics study on rats by subcutaneously injecting the compounds according to the present invention shows that: the half-life of P007, P008, P013, P014, P015, P016, P017, P018 and P020 were greater than the half-life of Tirzepatide (increased by about 23.7% to 64.0%). As compared with Tirzepatide, a commercially available dual-target product, the compounds according to the present invention modified by the modifying chain can provide a longer half-life in plasma. In addition, as compared with P001, the compound according to the present invention has a substantially longer half-life. The half-life was prolonged by more than 15 times, more than 20 times, or even more than 30 times.

### (II) Pharmacokinetics in C57 mice

After subcutaneous or intravenous administration to C57 mice, plasma was collected and the drug concentration in plasma was determined to illustrate the in vivo pharmacokinetic properties of the compound according to the present invention. The compound was dissolved in a phosphate buffer containing 0.1% Tween 20, and filtered through a filter membrane (PTFE, 0.45 µm) to obtain a 15 nmol/mL compound solution. About 100 µL of whole blood was collected from the orbital venous plexu into an EDTA-K₂ anticoagulant tube at predose, 0.5, 1, 2, 4, 8, 24, 48 and 72 hours respectively. The blood samples were centrifuged at 1500 g at 4°C for 10 minutes to obtain plasma, which was stored at -90°C to -60°C for further analysis and assay.

### Sample Treatment

### (1) Method for Treating the Samples for Tirzepatide, P014, P016 and P020

20.0 µL of thawed plasma sample was taken. 20 µL of 50% aqueous methanol solution (containing 100 ng mL⁻¹ Tolbutamide) and 60 µL of acetonitrile were added sequentially to precipitate protein. The mixture was vortexed for 5 minutes, and centrifuged at 4815 rpm for 8 minutes. 50.0 µL of the supernatant was taken, into which 50.0 µL of 0.5% aqueous formic acid solution was added. The mixture was vortexed for 5 minutes. 50 µL of the mixture solution was sampled and injected into the LC-MS/MS to determine the drug concentration in plasma.

Based on the drug concentration in plasma of the compounds in C57 mice, the pharmacokinetic parameters were calculated by applying the software Winnolin 8.3 in a non-compartmental model, and the results were shown in Table 7 and Table 8 below.

**Table 7 Average Pharmacokinetic Parameters after Single Subcutaneous Administration to Mice**

| PK Parameters | Unit | Tirzepatide | P014 | P016 | P020 |
|---|---|---|---|---|---|
| Cₘₐₓ | ng/mL | 871 | 851.84 | 433.67 | 835.67 |
| Tₘₐₓ | hr | 6 | 6 | 8 | 4.25 |
| T_{1/2} | hr | 9.21 | 20.33 | 23.05 | 19.89 |
| AUC₍₀₋ₜ₎ | ng·hr/mL | 19413.9 | 27316.4 | 15914.8 | 18950.6 |
| AUC_{(0-∞)} | ng·hr/mL | 19704.7 | 30150.5 | 18160.6 | 21093.3 |
| MRT₍₀₋ₜ₎ | hr | 14.48 | 22.94 | 25.98 | 21.84 |
| MRT_{(0-∞)} | hr | 15.29 | 30.28 | 35.91 | 29.36 |

**Table 8 Average Pharmacokinetic Parameters after Single Intravenous administration to Mice**

| PK Parameters | Unit | Tirzepatide | P014 | P016 | P020 |
|---|---|---|---|---|---|
| C₀ | ng/mL | 1901.94 | 1943.93 | 1147.89 | 1819.74 |
| T_{1/2} | hr | 10.47 | 17.20 | 20.15 | 16.66 |
| AUC₍₀₋ₜ₎ | ng·hr/mL | 12723.1 | 17458.9 | 11082.1 | 16281.7 |
| AUC_{(0-∞)} | ng·hr/mL | 12822.1 | 18245.6 | 12083.3 | 17369.9 |
| MRT₍₀₋ₜ₎ | hr | 11.39 | 15.72 | 19.92 | 16.80 |
| MRT_{(0-∞)} | hr | 12.00 | 19.215 | 26.64 | 21.77 |

The pharmacokinetics study on mice by subcutaneously injecting the compounds according to the present invention shows that: the half-life of P014, P016 and P020 were greater than the half-life of Tirzepatide (increased by 116.0% to 150.3%). That means that the half-life of these compounds were significantly greater than that of Tirzepatide. As compared with Tirzepatide, a commercially available dual-target product, the compounds according to the present invention modified by the modifying chain can provide a longer half-life in plasma.

### (III) Pharmacokinetics in Cynomolgus Monkeys

After subcutaneous or intravenous administration to cynomolgus monkeys, plasma was collected and the drug concentration in plasma was determined to illustrate the in vivo pharmacokinetic properties of the compound according to the present invention. The compound was dissolved in 1.4%PG in 8 mM Na₂HPO₄ Buffer (pH 7.39), and filtered through a filter membrane (PTFE, 0.45 µm) to obtain 25 and 12.5 nmol/mL solution of the compound respectively. The obtained solutions were administered to cynomolgus monkeys (4-8 kg) at a dose of 25 nmol/kg subcutaneously (s.c.) or 12.5 nmol/kg intravenously (i.v.). About 500 µL of whole blood was collected from the cephalic vein or other appropriate veins into an EDTA-K₂ anticoagulant tube at predose, 2 hours, 12 hours, 24 hours, 32 hours, 48 hours, 3 days(72 hours), 4 days(96 hours), 6 days(144 hours), 9 days(216 hours), 12 days(288 hours), 15 days(360 hours) and 18 days(432 hours), respectively. The blood samples were centrifuged at 2200 g for 10 minutes to obtain a plasma, which was stored at -90°C to -60°C for further analysis and assay.

### Sample Treatment

### (1) Method for Treating the Samples for Tirzepatide, P014, P016, P017 and P020

70.0 µL of thawed plasma sample was taken. 70 µL of acetonitrile (containing 1 ng/ml of the internal standard) was added to precipitate protein. The mixture was vortexed for 1 minute, and centrifuged at 14000 rpm for 10 minutes. 60.0 µL of the supernatant was taken, into which 60.0 µL of 0.5% aqueous formic acid solution was added. The mixture was vortexed for 5 minutes. 10 µL of the mixture solution was sampled and injected into the LC-MS/MS to determine the drug concentration in plasma.

Based on the drug concentration in plasma of the compounds in the cynomolgus monkeys, the pharmacokinetic parameters were calculated by applying the software Winnolin 8.2 in a non-compartmental model, and the results were shown in Table 9 and Table 10 below.

**Table 9 Average Pharmacokinetic Parameters after Single Subcutaneous Administration to Cynomolgus Monkeys**

| PK Parameters | Unit | Tirzepatide | P014 | P016 | P017 | P020 |
|---|---|---|---|---|---|---|
| Tₘₐₓ | hr | 22.67± 10.10 | 16.00±6.93 | 40.00±13.90 | 48.00±24.00 | 22.70±10.10 |
| Cₘₐₓ | ng/mL | 1297.45± 300.00 | 951.00±283 | 2997.00± 481.00 | 1655.00± 295.00 | 1349.00±77.00 |
| T1/2 | hr | 62.54±6.07 | 52.70±6.45 | 60.40±12.80 | 44.30±4.80 | 67.40±9.47 |
| AUC₍₀₋ₜ₎ | hr*ng/ mL | 148063.24± 38207.00 | 87690.00± 24556.00 | 319272.00± 41475.00 | 153476.00± 28919.00 | 161575.00± 10637.00 |
| AUC_{(0-∞)} | hr*ng/ mL | 150081.26± 39104.00 | 88432.00± 24213.00 | 322800.00± 44567.00 | 154121.00± 28747.00 | 163878.00± 12089.00 |
| MRT₍₀₋ₜ₎ | hr | 99.62± 6.26 | 74.20±13.83 | 96.50±15.90 | 76.40±12.70 | 97.50±9.67 |
| MRT_{(0-∞)} | hr | 104.89± 7.58 | 77.40±13.46 | 101.00±19.2 | 77.8012.20 | 103.00±13.10 |

**Table 10 Average Pharmacokinetic Parameters after Single Intravanous Administration to Cynomolgus Monkeys**

| PK Parameters | Unit | Tirzepatide | P016 |
|---|---|---|---|
| C₀ | ng/mL | 1167.70±159.90 | 1699.50±334.94 |
| T_{1/2} | hr | 59.53±5.22 | 59.81±8.95 |
| AUC₍₀₋ₜ₎ | hr*ng/mL | 57554.64±11825.48 | 78169.25±20321.82 |
| AUC_{(0-∞)} | hr*ng/mL | 58529.59±11927.99 | 80032.34±21063.24 |
| MRT₍₀₋ₜ₎ | hr | 68.34±8.61 | 65.29±8.85 |
| MRT_{(0-∞)} | hr | 74.27±8.76 | 72.94±10.91 |

The pharmacokinetics study on cynomolgus monkeys by subcutaneously injecting the compounds according to the present invention shows that: the half-life of P014, P016 and P020 were equivalent to that of Tirzepatide, and the Tₘₐₓ of P016 and P017 were delayed as compared with that of Tirzepatide. As compared with Tirzepatide, a commercially available dual-target product, the compounds according to the present invention modified by the modifying chain can provide a comparable half-life in plasma, and the Tₘₐₓ of P016 and P017 were 2 times that of Tirzepatide. The time to peak drug concentration was significantly delayed as compared to Tirzepatide, which may result in less/few gastrointestinal response or shorter titration time.

### (IV) Pharmacokinetics in Beagle Dogs

After subcutaneous administration to beagle dogs, plasma was collected and the drug concentration in plasma was determined to illustrate the in vivo pharmacokinetic properties of the compound according to the present invention. The compound of the present invention was administered to a beagle dog (7-9 kg) at a dose of 0.05mg/kg subcutaneously (s.c.). About 500 µL of whole blood was collected from the saphenous vein into an EDTA-K₂ anticoagulant tube at predose, 0.5 hours, 1 hour, 2 hours, 6 hours, 10 hours, 24 hours, 48 hours, 72 hours and 96 hours, respectively. The blood samples were centrifuged at 3000 g and at 2°C to 8°C for 10 minutes to obtain plasma, which was stored at -90°C to -60°C for further analysis and assay.

### Sample Treatment

### (1) Method for Treating the Samples for P016

20.0 µL of thawed plasma sample was taken. 20 µL of 100 ng/ml P016 was added, and mixed uniformly for 2 minutes. Then 120 µL of 0.1% formic acid in methanol was added to precipitate the protein. The mixture was vortexed for 5 minutes, and centrifuged at 4815g for 10 minutes. 100.0 µL of the supernatant was taken, into which 100 µL of 0.5% aqueous formic acid solution was added. The mixture was vortexed for 5 minutes. 10 µL of the mixture solution was sampled and injected into the LC-MS/MS to determine the drug concentration in plasma.

Based on the drug concentration in plasma of the compound in the beagle dogs, the pharmacokinetic parameters were calculated by applying the software Winnolin 8.3 in a non-compartmental model. The results were shown in Table 11 below.

**Table 11 Average pharmacokinetic parameters after single subcutaneous administration in beagle dogs**

| PK parameters | Unit | P016 |
|---|---|---|
| Tₘₐₓ | hr | 19.33±8.08 |
| Cₘₐₓ | ng/mL | 407.33±45.83 |
| T_{1/2} | hr | 69.32±13.97 |
| AUC₀₋ₜ | hr*ng/mL | 29924.99±5656.65 |
| AUC_{0-inf} | hr*ng/mL | 52127.67±14728.99 |
| MRT₀₋ₜ | hr | 44.77±4.17 |
| MRT_{0-inf} | hr | 107.9±23.22 |

The pharmacokinetics study on beagle dogs by subcutaneously injecting the compound P016 according to the present invention shows that: the half-life of P016 was 69.32 hours, the time to peak was 19.33 hours, the peak concentration was 407.33 ng/mL, and the AUC₀₋ₜ was 29925 hr*ng/mL. There was a certain exposure after subcutaneous administration, and it can support subcutaneous administration and a dosage frequency of once per week.

### (V) Single dose Pharmacokinetics in Cats

After subcutaneous administration to cats, plasma was collected and the drug concentration in plasma was determined to illustrate the in vivo pharmacokinetic properties of the compound according to the present invention. The compound was dissolved in citrate buffer to obtain a 0.125 mg/mL compound solution. The compound of the present invention was administered to female and male cats (2.5-4.5 kg, n=3/gender) at a dose of 0.025mg/kg subcutaneously (s.c.). About 0.5 mL of whole blood was collected from the jugular vein into an EDTA-K2 anticoagulant tube at predose, 0.5, 2, 4, 8, 24, 48, 72, 96, 120, 168, and 240 hours, respectively. The blood samples were stored on wet ice and centrifuged (1500-1600 g, 2-8°C, for 10 minutes) within 0.5 hours to obtain plasma, with at least 100 µL of plasma required for each sample. The plasma was then frozen and stored at -90°C to -60°C for subsequent analysis and detection.

### Sample treatment

### (1) Method for Treating the Samples for P016

30.0 µL of thawed plasma sample were taken, and 300 µL of acetonitrile solution containing an internal standard was added to precipitate the proteins. The mixture was vortexed for 1 minute and then centrifuged at 13,000 rpm for 5 minutes. 165 µL of the supernatant were transferred to a 96-well plate for LC-MS/MS analysis to determine the drug concentration in the plasma.

Based on the drug concentration in plasma of the compound in the cats, the pharmacokinetic parameters were calculated by applying the software Winnolin 8.3 in a non-compartmental model. The results were shown in Table 12 below.

**Table 12 Average pharmacokinetic parameters after single subcutaneous administration in cats**

| PK parameters | Unit | P016 |
|---|---|---|
| Tₘₐₓ | hr | 24.0 |
| Cₘₐₓ | ng/mL | 123 |
| T_{1/2} | hr | 37.4 |
| AUC₀₋ₜ | hr*ng/mL | 9260 |
| AUC_{0-∞} | hr*ng/mL | 11200 |
| MRT₀₋ₜ | hr | 54.4 |
| MRT_{0-∞} | hr | 61.6 |

The pharmacokinetics study on cats by subcutaneously injecting the compound P016 according to the present invention shows that: the half-life of P016 was 37.4 hours, the time to peak was 24.0 hours, the peak concentration was 123 ng/mL, and the AUC₀₋ₜ was 9260 hr*ng/mL. There was a certain exposure after subcutaneous administration, and it can support subcutaneous administration and a dosage frequency of once per week.

### III. Pharmacodynamics Effects

### (I) Pharmacodynamics Effects in db/db Mice was intended to studying the effects of the compounds according to the present invention on blood glucose in diabetic model mice (db/db mice).

In this study, db/db mice were subcutaneously single administered, and the changes in the blood glucose, the food intake, and the body weight of the mice were measured to illustrate the hypoglycemic effects of the compounds according to the present invention as well as the duration of the drug effect, and compared them with those of the positive controls of Tirzepatide and P001. In this study, 8-9 week old male db/db mice were used. The db/db mice were placed in an individual ventilated cage (IVC) facility with a controlled temperature (20-26°C) and humidity (40-70%) and a controlled 12h:12 h light-dark cycle. The mice can receive food and water ad libitum. Blood was sampled at the tail tip, and the random base blood glucose was measured by a Roche blood glucose meter. The mice were randomly divided into groups according to the random initial fasting blood glucose and initial body weight (n=6/group), and each group had similar body weight and blood glucose.

The compound according to the present invention (10 nmol/kg), or the positive controls of Tirzepatide (10 nmol/kg) and P001 (10 nmol/kg), were dissolved in a vehicle (PBS containing 0.1% Tween 20, pH 7.2-7.4). After a single subcutaneous administration, the random base blood glucoses were recorded at the set time point (0 to 120 hours, the recording of the random base blood glucose was stopped until there was no difference between the random base blood glucose of the compound and that of the vehicle group), and the daily body weight and food intake were also recorded. The data results were statistically analyzed using GraphPad Prism8, and the statistical differences among the groups were analyzed following a T-Test. Significant differences were identified at *p*<0.05.

Hypoglycemic Duration: the longest time during which the random base blood glucose of the compound has a significant difference (*p*<0.05) vs. the vehicle group.

**Table 13. Hypoglycemic Duration of the Compounds**

| Group | Compound | Hypoglycemic Duration (h) |
|---|---|---|
| Group 1 | P001 | 32 |
| | Tirzepatide | 56 |
| | P007 | 56 |
| | P008 | 72 |
| | P014 | 104 |
| | P019 | 56 |
| Group 2 | Tirzepatide | 48 |
| | P013 | 72 |
| | P015 | 32 |
| | P016 | 104 |
| | P017 | 72 |
| | P018 | 48 |
| | P020 | 104 |

As compared with P001, the hypoglycemic duration of the compounds according to the present invention was more than 24 hours (P007, P008, P014, P019), even more than 40 hours (P008, P014), and more than 72 hours (P014) (see Table 13, Figure 1A, and Figure 1B) longer than that of P001.

As compared with Tirzepatide, the hypoglycemic duration of the compounds according to the present invention was equivalent or longer (P007, P008, P013, P014, P016, P017, P018, P019, and P020), even more than 16 hours (P008, P013, P014, P016, P017, P020), more than 24 hours (P013, P014, P016, P017, P020), more than 48 hours (P014, P016, P020), more than 56 hours (P016, P020) longer than that of Tirzepatide (see Table 13).

As compared with P001, based on the experiments according to the method described above, during the test period, the hypoglycemic effects of P008 (*p*<0.01), P014 (*p*<0.001) and P019 (*p*<0.05) were significantly better than that of P001 (blood glucose AUC, P<0.05, as shown in Figure 2A). The inhibition of the blood glucose AUC was 2.3, 2.6 and 2.2 times of that of P001 respectively; the duration of hypoglycemic effect was 40 hours, 72 hours and 24 hours longer than that of P001 respectively (Table 13). The hypoglycemic effect of P007 was comparable to that of P001, but the duration of hypoglycemic effect was 24 hours longer than that of P001 (as shown in Figure 2A, and Table 13).

As compared with Tirzepatide: the hypoglycemic effects of P014 (*p*<0.05, Figure 2B), P016 (*p*<0.05, Figure 2C) and P020 (*p*<0.05, Figure 2C) were stronger than that of Tirzepatide (blood glucose AUC, p< 0.05). The inhibitions of the blood glucose AUC were 1.7, 2.2 and 2.0 times of that of Tirzepatide respectively. Moreover, they also show significantly longer duration of drug effects (48 hours, 56 hours and 56 hours longer than that of Tirzepatide respectively, Table 13). The hypoglycemic effects of P013 and P017 were comparable to that of Tirzepatide (Figure 2C), but the duration of drug effects was 24 hours longer than that of Tirzepatide (Table 13).

Surprisingly, as compared with P001, the GLP-1/GIP dual agonists according to the present invention significantly improve the hypoglycemic effect while maintaining the long-acting effect. The compounds according to the present invention with modifying chains in different forms can realize stronger and longer-lasting hypoglycemic effects than P001. Moreover, some compounds according to the present invention exhibit stronger and longer-lasting hypoglycemic effects than Tirzepatide.

Furthermore, in conjunction with performance of the binding and agonistic activity of the compounds according to the present invention to the targeted human GLP-1R and GIPR, it was surprisingly found that, although the compounds according to the present invention show a significantly reduced binding and agonistic activity at both the targeted human GLP-1R and the targeted human GIPR as compared with P001, the hypoglycemic effects of the compounds according to the present invention were significantly better than that of P001.

### (II) Multiple Administration Experiment on db/db Mice

In this study, db/db mice were subcutaneously administrated multiple times, and the changes in the blood glucose in the mice were determined to further illustrate the hypoglycemic effects of the compounds according to the present invention, and compared them with that of the positive control Tirzepatide. In this study, 7-8 week old male db/db mice were used. The db/db mice were placed in an individual ventilated cage (IVC) facility with a controlled temperature (20-26°C) and humidity (40-70%) and a controlled 12:12 h light-dark cycle. The mice received food and water ad libitum. Blood were sampled at the tail tip, and the base blood glucose was measured by a Roche blood glucose meter. The mice were randomly divided into groups according to the initial blood glucose and initial body weight (n=6/group), and each group had similar body weight and blood glucose.

The compounds according to the present invention (3, 10, 30 nmol/kg) or the positive control Tirzepatide (3, 10, 30 nmol/kg) was dissolved in a vehicle (PBS containing 0.1% Tween 20, pH 7.2-7.4). The mice were subcutaneously administrated every 3 days for 4 weeks. Subcutaneous administration was conducted on days 1, 4, 7, 10, 13, 16, 19, 22 and 25. Random blood glucose was recorded every 3 days throughout the study. On day 28, the fasting blood glucose was measured. Then the blood was collected by orbital blood sampling. After the orbital blood sampling, the animals were sacrificed, and the liver was taken and weighed. After 1 to 2 hours, the collected blood was centrifuged at 3000 rpm for 10 min to separate the serum. Biochemical indicators such as triglyceride (TG), alanine aminotransferase (ALT), and total bilirubin (TBIL) were determined with a biochemical analyzer. The data results were statistically analyzed using GraphPad Prism8, and the statistical differences among the groups were analyzed following T-Test. Significant differences were identified at *p*<0.05.

In the experiment performed as described in the above method, the compounds according to the present invention, such as P014, P016, P017 and P020, all show better blood glucose inhibition as compared with Tirzepatide at a dose of 3 nmol/kg. At a dose of 30 nmol/kg, the drug efficacy of P016 was superior to that of Tirzepatide (see Figures 3A-3D, and Table 14). P016, P017 and P020 significantly reduced liver weight in db/db mice at several doses, while Tirzepatide did not reduce liver weight at the three tested doses. The compounds according to the present invention, such as P014, P016, P017, P020 and Tirzepatide, can effectively reduce TG in the serum of the db/db mice (except for P017 and Tirzepatide groups at 3 nmol/kg), and the P016 and P020 groups at some doses show better drug efficacy than Tirzepatide (see Table 15). In addition, P014 and P016 can also reduce TBIL and/or ALT in serum (see Table 16).

**Table 14. Fasting Blood Glucose and Blood Glucose AUC of db/db Mice**

| Group | D28 FBG(Fasting Blood Glucose) (mmol/L) | AUC _{4-25 day} Glu (mmol/L·day) |
|---|---|---|
| Vehicle | 18.34±3.45 | 493.71±40.3 |
| P014 3 nmol/kg | 10.22±1.58*^{##} | 294±40.43**^{#} |
| P014 10 nmol/kg | 10.72±1.75 | 214.4±33.73***# |
| P014 30 nmol/kg | 13.38±3.38 | 190.62±26.47*** |
| P016 3 nmol/kg | 10.42±1.46*^{##} | 256.38±22***^{###} |
| P016 10 nmol/kg | 12.5±2.19 | 249.25±41.42** |
| P016 30 nmol/kg | 6.05±0.41**^{##} | 163.98±11.57****^{#} |
| P017 3 nmol/kg | 11.68±1.7^{#} | 303.78±33.24**^{#} |
| P017 10 nmol/kg | 11.95±2.51 | 249.65±47.65** |
| P017 30 nmol/kg | 10.08±0.79* | 294.96±11.96** |
| P020 3 nmol/kg | 9.6±1.32*^{##} | 268.28±24.64***^{#} |
| P020 10 nmol/kg | 10.7±1.62 | 290.78±27.25** |
| P020 30 nmol/kg | 8.22±1.07* | 184.18±22.44**** |
| TZP 3 nmol/kg | 17.35±1.21 | 419.85±26.42 |
| TZP 10 nmol/kg | 13.82±1.99 | 355.68±30.8* |
| TZP 30 nmol/kg | 10.88±1.2 | 252.7±32.81** |

| | | |
|---|---|---|
| T-Test, **p*<0.05, ***p*<0.01, ****p*<0.001, *****p*<0.0001 vs. vehicle group;#*p*<0.05, ##*p*< 0.01, ###*p*<0.001 vs. Tirzepatide(TZP) group at the same dose. Results were expressed as Mean±SEM for 6 mice. | | |

**Table 15. Liver Weight and Serum TG in db/db Mice**

| Group | Liver Weight (g) | Serum TG(mmol/L) |
|---|---|---|
| Vehicle | 2.61±0.19 | 1.87±0.13 |
| P014 3 nmol/kg | 2.26±0.18 | 1.39±0.07* |
| P014 10 nmol/kg | 2.1±0.14 | 1.29±0.03** |
| P014 30 nmol/kg | 1.89±0.31 | 1.14±0.18* |
| P016 3 nmol/kg | 2.24±0.1^{#} | 1.21±0.04***^{#} |
| P016 10 nmol/kg | 1.98±0.12* | 1.22±0.24* |
| P016 30 nmol/kg | 1.78±0.08** | 1.2±0.05*** |
| P017 3 nmol/kg | 2.14±0.08*^{##} | 1.58±0.17 |
| P017 10 nmol/kg | 2.11±0.09* | 1.19±0.05*** |
| P017 30 nmol/kg | 2.35±0.09 | 1.09±0.06*** |
| P020 3 nmol/kg | 2.03±0.09*^{##} | 1.37±0.12* |
| P020 10 nmol/kg | 2.09±0.1* | 1.07±0.03***^{#} |
| P020 30 nmol/kg | 1.83±0.16* | 1.2±0.07** |
| TZP 3 nmol/kg | 2.49±0.05 | 1.52±0.11 |
| TZP 10 nmol/kg | 2.31±0.17 | 1.21±0.04** |
| TZP 30 nmol/kg | 2.07±0.15 | 1.18±0.12** |

| | | |
|---|---|---|
| T-Test, **p*<0.05, ***p*<0.01, ****p*<0.001 vs. vehicle group; #*p*<0.05, ##*p*<0.01 vs. Tirzepatide(TZP) group at the same dose. Results were expressed as Mean±SEM for 6 mice. | | |

**Table 16. Serum ALT and TBIL in db/db Mice**

| Group | Serum ALT (U/L) | Serum TBIL (µmol/L) |
|---|---|---|
| Vehicle | 189.55±40.92 | 1.43±0.3 |
| P014 3 nmol/kg | 111.24±13.23 | 0.58±0.11*^{##} |
| P014 10 nmol/kg | 89.9±8.66* | 0.58±0.15* |
| P014 30 nmol/kg | 106.47±14.3 | 0.36±0.15*^{##} |
| P016 3 nmol/kg | 137.93±34.59 | 0.82±0.13 |
| P016 10 nmol/kg | 128.94±16.93 | 1.22±0.28 |
| P016 30 nmol/kg | 97.7±12.36* | 0.73±0.16 |
| P017 3 nmol/kg | 105.16±7.1 | 0.88±0.26 |
| P017 10 nmol/kg | 141.65±12.72 | 1.59±0.07 |
| P017 30 nmol/kg | 176.48±51.6 | 1.11±0.1 |
| P020 3 nmol/kg | 214.56±43.83 | 1.54±0.25 |
| P020 10 nmol/kg | 113.48±24.18 | 0.91±0.05 |
| P020 30 nmol/kg | 112.47±16.69 | 0.95±0.28 |
| TZP 3 nmol/kg | 146.03±19.67 | 1.54±0.17 |
| TZP 10 nmol/kg | 138.66±31.26 | 1.2±0.3 |
| TZP 30 nmol/kg | 112.48±14.55 | 0.91±0.04 |

| | | |
|---|---|---|
| T-Test, **p*<0.05 vs. vehicle group; #*p*<0.05, ##*p*<0.01 vs. Tirzepatide(TZP)group at the same dose. Results were expressed as Mean±SEM for 6 mice. | | |

To sum up, under this experimental system, the compounds according to the present invention can effectively reduce blood glucose in db/db mice by subcutaneous injection for 4 weeks. The minimum effective dose (≤3 nmol/kg) of the compounds according to the present invention was one third of that of Tirzepatide (10 nmol/kg), and the maximum drug efficacy (30 nmol/kg) of some compounds according to the present invention such as P016 was superior to that of Tirzepatide. In addition, some compounds according to the present invention exhibit reduced liver weight and protective effects to the livers in db/db mice, while Tirzepatide had no similar effect.

### (III) Weight Loss Effect in DIO Mice Obesity Models

In this study, DIO mice were subcutaneously administrated multiple times with the compound according to the present invention, and the changes in the body weight and blood glucose of the mice were determined to illustrate the weight reduction effect of the compound according to the present invention, and were compared with those of the positive control Tirzepatide. In this study, 26-week-old male DIO mice were used. DIO mice were housed in appropriate cages. DIO mice were housed in an environment-monitored and well-ventilated SPF-grade animal room, with the temperature maintained between 20 and 26°C and relative humidity between 40% and 70%. A 12-hour light/dark cycle was provided for illumination. The mice received food and water ad libitum. In the experiment, DIO mice with similar random blood glucoses (RBG) (D1) and body weights (D1) were selected and randomly grouped (n=10/group). The compound P016 of the present invention (0.3, 1, 3, 30 nmol/kg) or the positive control Tirzepatide (1, 30 nmol/kg) was dissolved in a vehicle (PBS containing 0.1% Tween20, pH 7.2-7.4). The mice were subcutaneously administrated once every 3 days for 4 consecutive weeks. Subcutaneous injection administration was conducted on Day 1, 4, 7, 10, 13, 16, 19, 22, 25, and 28.

The animals were weighed before administration on the day of first administration (D1), and at a fixed time once every 3 days after administration. Food intake was measured once daily after administration. RBG was measured before grouping and at 48 h after each administration. After fasted for 5 h on D30 (no water deprivation was required), FBG and glycated hemoglobin A1c (HbA1c) were detected, and blood was collected intravenously to separate the serum. A mouse insulin ELISA kit was used to detect the insulin content in the serum, and a biochemistry analyser was used to detect total cholesterol (TC), TG, low-density lipoprotein cholesterol (LDL), high-density lipoprotein cholesterol (HDL), free fatty acid (FFA), ALT, and aspartate aminotransferase (AST) content. The liver was collected, the perirenal and paratesticular fat tissues were separated and weighed, the organ-body ratio was calculated. The liver was stained with HE and Oil Red O for pathological scoring. The data results were statistically analyzed using one-way analysis of variance (ANOVA). A Levene's test was performed to test for variance homogeneity. If the result was homogeneous (P>0.05), a one-way analysis of variance (ANOVA) would be performed. If ANOVA showed significance (P≤0.05), a Tukey test of variancpair-wise comparisons would be performed. In the case of heterogeneity of variance (P ≤0.05 ), a Dunnett's T3 test would be performed. Results were expressed as mean and standard deviation (Mean ± SEM). The test level was 0.05. Both statistical significance and biological significance were considered when analyzing the results.

In the experiment conducted as described in the above method, the compound according to the present invention significantly reduced the cumulative food intake and body weight of obese mice in a dose-dependent manner. The minimum effective dose was 1 nmol/kg, and the weight loss effect at this dose was significantly better than the positive control Tirzepatide at the same dose. At a dose of 30 nmol/kg, the weight loss degree of the compound according to the present invention was greater than that of Tirzepatide (percentage of weight loss, 39.97% vs 34.47%) (see Figure 4, Figure 5, and Table 17). The compound according to the present invention significantly reduced the total abdominal fat (sum of perirenal fat and paratesticular fat) and serum TC in DIO mice (Table 17, Table 19), and had significant lipid-lowering effect. 1, 3, and 30 nmol/kg of the compound according to the present invention significantly reduced FBG and insulin level on D30, and improved insulin resistance (Table 18). The compound according to the present invention significantly reduced liver weight and serum ALT level and improved liver function (Table 19).

**Table 17. Cumulative food intake, body weight, fat and liver weights of DIO mice**

| | Cumulative food intake | Body weight (g) | Total abdominal fat (g) | Liver weight (g) |
|---|---|---|---|---|
| Vehicle | 78.83±0.73 | 48.0±0.5 | 3.071±0.115 | 1.758±0.072 |
| P016 0.3 nmol/kg | 75.75±2.81 | 45.4±1.3 | 2.631±0.069 | 1.552±0.106 |
| P016 1 nmol/kg | 61.40±1.58****^{####} | 38.3±1.1^{****##} | 1.849±0.181*** | 1.191±0.075^{***} |
| P016 3 nmol/kg | 55.47±2.72**** | 34.1±0.7**** | 0.972±0.076**** | 1.069±0.029**** |
| P016 30 nmol/kg | 48.38±2.31**** | 29±0.7**** | 0.461±0.034**** | 0.995±0.029**** |
| Tirzepatide 1 nmol/kg | 76.51±1.67 | 44.7±0.87 | 2.318±0.123** | 1.305±0.073** |
| Tirzepatide 30 nmol/kg | 47.95±2.14**** | 31.8±0.7**** | 0.781±0.093**** | 0.951±0.031**** |

| | | | | |
|---|---|---|---|---|
| Note: ***p*≤0.01, ****p*≤0.001, *****p*≤0.0001 were compared with the Vehicle group, ##*p*≤0.01, ####*p*≤0.0001 were compared with the same dose of positive control Tirzepatide group. Results were expressed as Mean±SEM for 10 mice. | | | | |

**Table 18. Fasting blood glucose, insulin and insulin resistance index in DIO mice**

| | Fasting blood glucose | Insulin | Insulin resistance index |
|---|---|---|---|
| Vehicle | 14.84±0.67 | 2.911±0.294 | 41.698±4.071 |
| P016 0.3 nmol/kg | 13.78±0.39 | 3.285±0.481 | 43.94±6.288 |
| P016 1 nmol/kg | 11.23±0.40****^{#} | 1.911±0.29 | 21.532±3.705* |
| P016 3 nmol/kg | 10.36±0.67**** | 1.196±0.185* | 11.991±1.907*** |
| P016 30 nmol/kg | 8.39±0.30**** | 0.624±0.130** | 5.179±1.178**** |
| Tirzepatide 1 nmol/kg | 9.19±0.35**** | 2.317±0.511 | 20.983±4.926 |
| Tirzepatide 30 nmol/kg | 7.96±0.33**** | 1.418±0.535 | 11.023±4.147* |

| | | | |
|---|---|---|---|
| Note: **p*≤0.05, ****p≤0.01, *****p≤0.001, *****p*≤0.0001 were compared with the Vehicle group, #P≤0.05 was compared with the same dose of positive control Tirzepatide group. Results were expressed as Mean±SEM for 10 mice. | | | |

**Table 19. Serum TC and ALT of DIO mice**

| | Serum TC | ALT |
|---|---|---|
| Vehicle | 4.48±0.24 | 120.90±16.22 |
| P016 0.3 nmol/kg | 4.07±0.26 | 95.60±14.09 |
| P016 1 nmol/kg | 3.26±0.20* | 43.90±3.86* |
| P016 3 nmol/kg | 2.61±0.13*** | 52.80±9.62* |
| P016 30 nmol/kg | 2.50±0.18**** | 49.60±5.67* |
| Tirzepatide 1 nmol/kg | 3.54±0.15 | 56.20±4.66* |
| Tirzepatide 30 nmol/kg | 2.65±0.08*** | 41.50±5.31* |

| | | |
|---|---|---|
| Note: **p*≤0.05, ****p*≤0.001, *****p*≤0.0001 were compared with the Vehicle group. Results were expressed as Mean±SEM for 10 mice. | | |

In summary, in this experimental system, the compound of the present invention could effectively reduce the weight of DIO mice after 4 weeks of subcutaneous injection. The minimum effective dose was 1 nmol/kg. The weight loss effect at this dose was significantly better than the positive control Tirzepatide at the same dose, and the maximum weight loss of the compound according to the present invention (30 nmol/kg) was greater than Tirzepatide at the same dose (percentage of weight loss, 39.97% vs 34.47%). In addition, the compound of the present invention exhibited effects on lowering blood glucose, improving insulin resistance, and improving liver function.

### (IV) Weight Loss Efficacy in Obese Dogs

In this study, the compound of the present invention was administered subcutaneously to obese dogs multiple times to detect changes in their body weights and elucidate the weight loss effect of the compound. Obese dogs aged 8 months or older with a body condition score (BCS) ≥6 were used in this study. The dogs were housed in appropriately sized cages under controlled environmental conditions with a room temperature of 20-26°C and a relative humidity of 40%-70%, and had free access to food and water. Experimental dogs with similar body weights and BCSs (body weight: 15~24 kg, BCS: 6~9) were randomly divided into four groups (n=6 per group): G1, G2, G3, and G4. Group G1 served as the vehicle control group. As shown in Table 20, for the obese dogs in Groups G2 and G3, the compound P016 of the present invention was administered continuously subcutaneously once a week (QW) with escalating doses for a total of six administrations. For Group G2 obese dogs, the first dose (4 nmol/kg) was administered on Day 1 (D1) and Day 8 (D8), adjusted to the second dose (6 nmol/kg) on Day 15 (D15), and further adjusted to the third dose (96 nmol/kg) on Day 22 (D22), which third dose (96 nmol/kg) was maintained for the subsequent administrations on Day 29 (D29) and Day 36 (D36). For Group G3 obese dogs, the first dose (12 nmol/kg) was administered on D1 and D8, adjusted to the second dose (19 nmol/kg) on D15, and further adjusted to the third dose (385 nmol/kg) on D22, which third dose (385 nmol/kg) was also maintained for the subsequent administrations on D29 and D36. For Group G4 dogs, the positive control Dirlotapide was administered orally at a dose of 0.5 mg/kg once per day (QD) for 42 consecutive days.

After administration, the body weights of dogs in Group G4 receiving Dirlotapide were measured at a fixed time once daily, while the body weights of dogs in the other groups (G1, G2, and G3) were measured at a fixed time once every 7 days. Food intake was measured once per day after administration. Blood tests for routine and biochemical parameters were conducted before grouping and on D43. Imaging examinations of abdominal fat volume were performed before grouping and from D43 to D45 to measure total abdominal fat volume (TFV) and visceral abdominal fat volume (VFV), and to calculate subcutaneous abdominal fat (SFV). Body condition score (BCS), body mass index (BMI), and abdominal circumference (AC) were measured once a week. The data were statistically analyzed using ANOVA. If ANOVA was statistically significant (*p* < 0.05) and variances were homogeneous, Tukey's test was used for intergroup comparison. If variances were heterogeneous, Dunnet's T3 test was used. Results were expressed as mean ± standard error of the mean (Mean ± SEM). The significance level was set at 0.05, and both statistical and biological significance were considered in the analysis.

In the experiment conducted as described above, the compound P016 of the present invention, administered subcutaneously with escalating doses QW for six consecutive administrations, significantly reduced body weight, BMI, SFV, and TFV in obese dogs (Table 20) and decreased food intake. The effective dose of P016 for weight loss was 96 nmol/kg (equivalent to 0.5 mg/kg), with a maximum weight loss of 9.51%. The weight-loss effect of P016 was superior to that of the marketed drug Dirlotapide (weight loss: 4.72%), as shown in Figures 6 and 7.

**Table 20. Dosing regimen for administering Compound P016 to obese dogs in Group G2 and Group G3.**

| Animal | Group | First administration, second administration D1, D8, first dose | Third administration, D15, second dose | Fourth, fifth, and sixth administration D22, D29, D36, third dose |
|---|---|---|---|---|
| Dog | G2 | 4 nmol/kg (0.02 mg/kg) | 6 nmol/kg (0.03 mg/kg) | 96 nmol/kg (0.50 mg/kg) |
| Dog | G3 | 12 nmol/kg (0.06 mg/kg) | 19 nmol/kg (0.10 mg/kg) | 385 nmol/kg (2.00 mg/kg) |

**Table 21. Comparison of the change rate in abdominal fat indicators among dogs in different groups. (D43) (Mean±SEM)**

| | Group | TFV | VFV | SFV | rVFV | rSFV |
|---|---|---|---|---|---|---|
| G1 | Vehicle | 8.64±7.78 | 4.24±11.51 | 12.42±6.89 | 4.07±2.47 | -5.30±4.73 |
| G2 | P016 4/6/96 nmol/kg | -16.16±5.88^{*} | -10.81±10.19 | -18.11±4.49^{**} | -1.51±3.56 | 4.80±6.52 |
| G3 | P016 12/19/385 nmol/kg | -13.00±4.47^{*} | -9.42±5.39 | -15.14±4.56^{**} | -2.41±2.12 | 4.19±4.19 |
| G4 | Dirlotapide 0.5 mg/kg | -20.50±6.83^{*} | -17.93±6.39 | -21.02±8.14^{**} | -1.35±2.62 | 4.19±4.62 |

| | | | | | | |
|---|---|---|---|---|---|---|
| For Groups G2 and G3, the dose was adjusted from the first dose (4, 12 nmol/kg) to the second dose (6, 19 nmol/kg) on D15, and then adjusted to the third dose (96, 385 nmol/kg) on D22, D29, and D36; **p* <0.05, ***p* <0.05, versus Vehicle. | | | | | | |

### (V) Weight Loss Efficacy in Obese Cats

This study evaluated the weight loss efficacy of the compound of this invention through multiple subcutaneous administrations to obese cats, with changes in cat body weights being monitored. The study involved cats aged 8 months or older with a body condition score (BCS) of ≥6. The obese cats were housed in appropriately sized cat cages under controlled environmental conditions, with a room temperature of 20-26°C and a relative humidity of 40%-70%, and had free access to food and water. Experimental cats with similar body weights and BCS (body weight: 3.3~7.5 kg, BCS: 6~9) were randomly divided into four groups (n=6/group): G11 (control group), G12, G13, and G14. . Group G11 served as the vehicle control group. As shown in Table 22, the compound P016 of this invention was administered subcutaneously to cats in G12, G13, and G14 once a week (QW) with escalating doses over five consecutive administrations. For Group G12 obese cats, the first dose (4 nmol/kg) was administered on Day 1 (D1) and Day 8 (D8), adjusted to the second dose (48 nmol/kg) on Day 15 (D15), and further adjusted to the third dose (96 nmol/kg) on Day 22 (D22), which third dose (96 nmol/kg) was also maintained for the subsequent administrations on D29. For Group G13 obese cats, the first dose (14 nmol/kg) was administered on Day 1 (D1) and Day 8 (D8), adjusted to the second dose (96 nmol/kg) on Day 15 (D15), and further adjusted to the third dose (192 nmol/kg) on Day 22 (D22), which third dose (192 nmol/kg) was also maintained for the subsequent administrations on D29. For Group G14 obese cats, the first dose (58 nmol/kg) was administered on Day 1 (D1) and Day 8 (D8), adjusted to the second dose (192 nmol/kg) on Day 15 (D15), and further adjusted to the third dose (385 nmol/kg) on Day 22 (D22), which third dose (385 nmol/kg) was also maintained for the subsequent administrations on D29.

The animals' body weights were measured at a fixed time every 7 days after administration, and food intake was measured once per day. Blood tests for routine and biochemical parameters were conducted before grouping and on D36. Body condition score (BCS), feline body mass index (FBMI), and abdominal circumference (AC) were measured once a week. The data were statistically analyzed using ANOVA. If ANOVA yielded statistical significance (*p* < 0.05) and the variances were homogeneous, Tukey's test was used for intergroup comparisons. If the variances were heterogeneous, Dunnett's T3 test was used. Results are presented as means and standard errors of the mean (Mean ± SEM). The significance level was set at 0.05, and both statistical and biological significance were considered in the analysis.

In the experiments conducted as described above, subcutaneous administration of the escalating doses of the compound P016 of this invention, administered once a week for five consecutive times, significantly reduced body weight, cumulative food intake, AC, and BCS in obese cats. The effective dose for weight loss was 96 nmol/kg (equivalent to 0.5 mg/kg), with a maximum weight loss of 19.45%, as shown in Figures 8 and 9.

**Table 22. Dosing regimen for administering Compound P016 to obese cats in Groups G12, G13 and G14**

| Animal | Group | First administration, second administration, D1, D8, first dose | Third administration, D15, second dose | Fourth, fifth, and sixth Administration D22, D29, third dose |
|---|---|---|---|---|
| Cat | G12 | 4 nmol/kg (0.019 mg/kg) | 48 nmol/kg (0.25 mg/kg) | 96 nmol/kg (0.50mg/kg) |
| Cat | G13 | 14 nmol/kg (0.075 mg/kg) | 96 nmol/kg (0.50 mg/kg) | 192 nmol/kg (1.00 mg/kg) |
| Cat | G14 | 58 nmol/kg (0.30 mg/kg) | 192 nmol/kg (1.00 mg/kg) | 385 nmol/kg (2.00 mg/kg) |

### (VI) Repeated Administration on Male Rats for 2 Weeks

### 1. Experimental Schedule

77 SD male rats (SPF grade) were selected and randomly divided into 10 groups based on body weight. Group 1 was the vehicle control group, including 5 animals. Group 2 to Group 4 were the control groups administrated with commercially available Tirzepatide, each group including 8 animals, wherein 5 animals for primary test, and 3 animals for toxicokinetics test, and the administration dose was 1, 3, and 10 mg/kg respectively. Group 5 to Group 7 were the groups administrated with the test substance 1; and Group 8 to Group 10 were the groups administrated with the test substance 2, each group including 8 animals, wherein 5 animals for primary test, and 3 animals for toxicokinetics test, and the administration dose was 1, 3 and 10 mg/kg respectively. Each of the above groups was continuously administered twice a week for 2 weeks (administrating at Day 1, Day 5, Day 8, Day 12 and Day 15).

During the experiment, the cage condition monitoring, detailed clinical observations, determination of the food consumption, determination of body weight, measurement of the blood biochemical and hematology indicator, investigation of the gross anatomy, weighing of the organ, studying on toxicokinetic were performed on all the groups of the animals.

### 2. Data Statistics

The tables below illustrates the combinations used for statistical comparison in detail. The tables were as follows:

### Method for Statistical Analysis

| Group as control | Groups used for comparison |
|---|---|
| 1 | 2, 3, 4, 5, 6, 7, 8, 9, 10 |

Original data during each time period was tabulated. The average values and standard deviations and/or group change were calculated for each assay endpoint based on the group and sex. For each endpoint, the administrated group will be compared with the control group in the following aspect. A logarithmic conversion was performed to the endpoint data as desired prior to the start of the specific analyses.

### Statistical Analysis

| Item | Analytical Method | |
|---|---|---|
| Food Consumption | comparison between groups | |
| Weight | | |
| Hematology (excluding white blood cell count) | | |
| Coagulation | | |
| Serum Biochemistry | | |
| Organ Weight | | |
| | Absolute Weight | |
| | Organ Coefficients relative to body weight and brain | |
| White Blood Cell Count | | logarithmic conversion/group-group comparison |
| | Total White Blood Cells | |
| | White Blood Cell Differential Count | |

### 3. Experimental Results

Body Weight/Weight Gain: After the first administration, the decrease of the body weight was observed in each of the following groups: Tirzepatide at a dose ≥3 mg/kg; P014 and P016 at a dose ≥1 mg/kg. With the continuation of the administration, the body weight was recovered, and by the end of the administration, all groups had a positive increase in body weight, but the weight gain was lower than that of the control group, showing an obvious dose-dependent relationship.

Food Intake: throughout the test procedure, a dose-dependent decrease in food intake was observed in each administration group (Tirzepatide, P014 and P016). Blood Chemistry: Amylase and triglyceride levels decrease. Hematology: reticulocyte level decreases slightly. Gross Anatomy: no abnormalities were found in each administration group (Tirzepatide, P014 and P016). Organ Weight: the weight of heart, liver and spleen and the like in each administration group (Tirzepatide, P014 and P016) decreased, which was not significantly different from that in the vehicle control group. This may be associated to the weight loss caused by pharmacological effects. Toxicokinetic: Cₘₐₓ and AUC in each administration group (Tirzepatide, P014 and P016) increase nearly proportionally to the dose. The administration groups (Tirzepatide, P014 and P016) show no substantial accumulation when continuously administered twice a week for 2 weeks.

Each administration group (Tirzepatide, P014 and P016) had a MTD≥10 mg/kg when continuously administered twice a week for 2 weeks. Cₘₐₓ, AUCₗₐₛₜ after the last dose and the safety window of the test were as follows:

**Table 23 Safety Window for Each Administration Group**

| Test Article | Tirzepatide | P014 | P016 |
|---|---|---|---|
| MTD | 2077 nmol/kg (MTD for 2 weeks) | 1980 nmol/kg (MTD for 2 weeks) | 1924 nmol/kg (MTD for 2 weeks) |
| Efficacy Dose | 10 nmol/kg | 3 nmol/kg | 3 nmol/kg |
| Safety Window | 416 | 1319 | 1282 |
| (based on dosage) | | | |
| Safety Window (based on exposure AUCₗₐₛₜ) | 130 | 596 | 920 |

To sum up, P016 and P014 have similar toxicity properties to Tirzepatide, but have a wider safety window (based on exposure, the safety window of P014 was more than 4 times of that of Tirzepatide, and the safety window of P016 was more than 7 times of that of Tirzepatide).

In addition, the safety windows of the compounds according to the present invention, in particular P014 and P016, was 10 times larger, or 20 times larger, or 30 times larger, or 40 times larger, or 50 times larger, or 60 times larger, or 70 times larger, or 80 times larger, or 90 times larger than that of P001.

Meanwhile, under the same exposure, the compounds according to the present invention have a lower impact on heart rate (HR) than Tirepatide. The increase in HR for P016 was lower than that for Tirepatide. No sustained HR increase exceeding 30% was observed. At the same exposure, heart rate recovery for P016 was faster than TZP (the recover time for P016 was between 72 and 120 hours, and the recovery time for TZP >120 hours).

The peptide compounds provided by the present invention and the use thereof have been introduced in detail above.

Specific examples were applied herein to illustrate the principle and embodiments of the present invention. The illustration of the above examples were merely used to help understand the method and the central idea of the present invention. It should be indicated that those skilled in the art can make several improvements and modifications to the present invention without departing from the principles of the present invention, and these improvements and modifications also fall within the protection scope of the claims of the present invention.

### Amino Acid Sequence:

| Name | No. | Sequence |
|---|---|---|
| P001 | SEQ ID NO:1 | Y-X1-E-G-T-X2-T-S-D-Y-X3-I-X4-L-D-K-Q-A-Q-A-E-F-V-K-W-L-L-K-G-G-P-S- S-G-A-P-P-P-S-K; |
| | | wherein X₁ is Aib; X₂ is αMePhe; X₃ is Aib; X₄ is Aib; and the C-terminal amino acid is amidated as a C-terminal primary amide (SEQ ID NO:1) |
| P007 | SEQ ID NO:7 | Y-X1-E-G-T-X2-T-S-D-Y-X3-I-X4-L-D-K-Q-A-Q-A-E-F-V-K-W-L-L-K-G-G-P-S- S-G-A-P-P-P-S-K; |
| | | wherein X₁ is Aib; X₂ is αMePhe; X₃ is Aib; X₄ is Aib; K at position 24 is chemically modified through conjugation to the ε-amino of the K side-chain with ([2-(2-amino-ethoxy)-ethoxy]-acetyl)₂-(γ-Glu)₁-CO-(CH₂)₁₆-CO₂H; and the C-terminal amino acid is amidated as a C-terminal primary amide (SEQ ID NO:7). |
| P008 | SEQ ID NO:8 | Y-X1-E-G-T-X2-T-S-D-Y-X3-I-X4-L-D-K-Q-A-Q-A-E-F-V-K-W-L-L-K-G-G-P-S- S-G-A-P-P-P-S-K; |
| | | wherein X₁ is Aib; X₂ is αMePhe; X₃ is Aib; X₄ is Aib; K at position 28 is chemically modified through conjugation to the ε-amino of the K side-chain with ([2-(2-amino-ethoxy)-ethoxy]-acetyl)₂-(γ-Glu)₁-CO-(CH₂)₁₆-CO₂H; and the C-terminal amino acid is amidated as a C-terminal primary amide (SEQ ID NO:8). |
| P013 | SEQ ID NO:13 | Y-X1-E-G-T-X2-T-S-D-Y-X3-I-X4-L-D-K-Q-A-Q-A-E-F-V-K-W-L-L-K-G-G-P-S- S-G-A-P-P-P-S-K; |
| | | wherein X₁ is Aib; X₂ is αMePhe; X₃ is Aib; X₄ is Aib; |
| | | K at position 24 is chemically modified through conjugation to the ε-amino of the K side-chain with ([2-(2-amino-ethoxy)-ethoxy]-acetyl)₂-(γ-Glu)₁-CO-(CH₂)₁₈-CO₂H; and the C-terminal amino acid is amidated as a C-terminal primary amide (SEQ ID NO:13). |
| P014 | SEQ ID NO:14 | Y-X1-E-G-T-X2-T-S-D-Y-X3-I-X4-L-D-K-Q-A-Q-A-E-F-V-K-W-L-L-K-G-G-P-S- S-G-A-P-P-P-S-K; |
| | | wherein X₁ is Aib; X₂ is αMePhe; X₃ is Aib; X₄ is Aib; |
| | | K at position 24 is chemically modified through conjugation to the ε-amino of the K side-chain with ([2-(2-amino-ethoxy)-ethoxy]-acetyl)₂-(γ-Glu)₁-CO-(CH₂)₂₀-CO₂H; and the C-terminal amino acid is amidated as a C-terminal primary amide (SEQ ID NO:14). |
| P015 | SEQ ID NO:15 | Y-X1-E-G-T-X2-T-S-D-Y-X3-I-X4-L-D-K-Q-A-Q-A-E-F-V-K-W-L-L-K-G-G-P-S- S-G-A-P-P-P-S-K; |
| | | wherein X₁ is Aib; X₂ is αMePhe; X₃ is Aib; X₄ is Aib; |
| | | K at position 24 is chemically modified through conjugation to the ε-amino of the K side-chain with ([2-(2-amino-ethoxy)-ethoxy]-acetyl)₂-(γ-Glu)₃-CO-(CH₂)₁₆-CO₂H; and the C-terminal amino acid is amidated as a C-terminal primary amide (SEQ ID NO:15). |
| P016 | SEQ ID NO:16 | Y-X1-E-G-T-X2-T-S-D-Y-X3-I-X4-L-D-K-Q-A-Q-A-E-F-V-K-W-L-L-K-G-G-P-S- S-G-A-P-P-P-S-K; |
| | | wherein X₁ is Aib; X₂ is αMePhe; X₃ is Aib; X₄ is Aib; |
| | | K at position 24 is chemically modified through conjugation to the ε-amino of the K side-chain with ([2-(2-amino-ethoxy)-ethoxy]-acetyl)₃-(γ-Glu)₁-CO-(CH₂)₂₀-CO₂H; and the C-terminal amino acid is amidated as a C-terminal primary amide (SEQ ID NO:16). |
| P017 | SEQ ID NO:17 | Y-X1-E-G-T-X2-T-S-D-Y-X3-I-X4-L-D-K-Q-A-Q-A-E-F-V-K-W-L-L-K-G-G-P-S- S-G-A-P-P-P-S-K; |
| | | wherein X₁ is Aib; X₂ is αMePhe; X₃ is Aib; X₄ is Aib; |
| | | K at position 28 is chemically modified through conjugation to the ε-amino of the K side-chain with ([2-(2-amino-ethoxy)-ethoxy]-acetyl)₂-(γ-Glu)₁-CO-(CH₂)₂₀-CO₂H; |
| | | and the C-terminal amino acid is amidated as a C-terminal primary amide (SEQ ID NO:17). |
| P018 | SEQ ID NO:18 | Y-X1-E-G-T-X2-T-S-D-Y-X3-I-X4-L-D-K-Q-A-Q-A-E-F-V-K-W-L-L-K-G-G-P-S- S-G-A-P-P-P-S-K; |
| | | wherein X₁ is Aib; X₂ is αMePhe; X₃ is Aib; X₄ is Aib; |
| | | K at position 28 is chemically modified through conjugation to the ε-amino of the K side-chain with ([2-(2-amino-ethoxy)-ethoxy]-acetyl)₂-(γ-Glu)₃-CO-(CH₂)₁₆-CO₂H; |
| | | and the C-terminal amino acid is amidated as a C-terminal primary amide (SEQ ID NO:18). |
| P019 | SEQ ID NO:19 | Y-X1-E-G-T-X2-T-S-D-Y-X3-I-X4-L-D-K-Q-A-Q-A-E-F-V-K-W-L-L-K-G-G-P-S- S-G-A-P-P-P-S-K; |
| | | wherein X₁ is Aib; X₂ is αMePhe; X₃ is Aib; X₄ is Aib; |
| | | K at position 28 is chemically modified through conjugation to the ε-amino of the K side-chain with ([2-(2-amino-ethoxy)-ethoxy]-acetyl)₃-(γ-Glu)₁-CO-(CH₂)₁₆-CO₂H; |
| | | and the C-terminal amino acid is amidated as a C-terminal primary amide (SEQ ID NO:19). |
| P020 | SEQ ID NO:20 | Y-X1-E-G-T-X2-T-S-D-Y-X3-I-X4-L-D-K-Q-A-Q-A-E-F-V-K-W-L-L-K-G-G-P-S- S-G-A-P-P-P-S-K; |
| | | wherein X₁ is Aib; X₂ is αMePhe; X₃ is Aib; X₄ is Aib; |
| | | K at position 28 is chemically modified through conjugation to the ε-amino of the K side-chain with ([2-(2-amino-ethoxy)-ethoxy]-acetyl)₃-(γ-Glu)₁-CO-(CH₂)₂₀-CO₂H; |
| | | and the C-terminal amino acid is amidated as a C-terminal primary amide (SEQ ID NO:20). |

## Claims

1. Use of a compound of the following Formula (AI) or a pharmaceutically acceptable salt thereof in the manufacture of a medicament for preventing or treating a disease in a non-primate and non-rodent animal:
Y-X₁-E-G-T-X₂-T-S-D-Y-A11-A12-A13-L-D-K-A17-A-Q-A20-E-F-V-K-W-L-L-K-A29-G-P-S- S-G-A-P-P-P-S-K Formula (AI);
wherein X₁ is Aib; X₂ is αMePhe;
A11 is Aib or Ala;
A12 is Ala, Ile, Lys, Phe or Pya(4);
A13 is Aib, Cha, Leu, αMePhe or αMeTyr;
A17 is Gln or Ile;
A20 is Ala or Ser;
A29 is Gln or Gly,
1, 2, 3 or 4 Ks, which are selected from the group consisting of K at position 16, K at position 24, K at position 28 and K at position 40, are chemically modified through conjugation to the ε-aminos of the K side-chains with ([2-(2-amino-ethoxy)-ethoxy]-acetyl)ₐ-(γ-Glu)_{b}-CO-(CH₂)_{c}-Z, wherein each a is independently an integer from 0 to 5, each b is independently an integer from 0 to 5, and each c is independently an integer from 10 to 24, wherein Z is independently selected from the group consisting of -CH₃, carboxylic acids or carboxylic acid bioisosteres, phosphonates or sulfonates.

2. The use according to claim 1, wherein
A11 is Aib; or/and
A12 is Ile; or/and
A13 is Aib; or/and
A17 is Gln; or/and
A20 is Ala; or/and
A29 is Gly; or/and
a is independently selected from an integer of 1, 2, 3, 4 or 5, b is independently selected from an integer of 1, 2, 3, 4 or 5, and c is independently selected from an integer of 12 to 22; preferably, c is independently selected from 14, 16, 18 or 20.

3. Use of a compound of the following Formula (I) or a pharmaceutically acceptable salt thereof in the manufacture of a medicament for preventing or treating a disease in a non-primate and non-rodent animal:
Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K Formula (I);
wherein X₁ is Aib; X₂ is αMePhe; X₃ is Aib; X₄ is Aib; 1, 2, 3 or 4 Ks, which are selected from the group consisting of K at position 16, K at position 24, K at position 28 and K at position 40, are chemically modified through conjugation to the ε-aminos of the K side-chains with ([2-(2-amino-ethoxy)-ethoxy]-acetyl)ₐ-(γ-Glu)_{b}-CO-(CH₂)_{c}-Z, wherein each a is independently an integer from 0 to 5, each b is independently an integer from 0 to 5, and each c is independently an integer from 10 to 24, wherein Z is independently selected from the group consisting of -CH₃, carboxylic acids or carboxylic acid bioisosteres, phosphonates or sulfonates; and the C-terminal amino acid is amidated as a C-terminal primary amide.

4. The use according to claim 3, wherein the compound is:
Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K Formula (I),
or a pharmaceutically acceptable salt thereof, wherein X₁ is Aib; X₂ is αMePhe; X₃ is Aib; X₄ is Aib; 1 or 2 Ks, which are selected from the group consisting of K at position 16, K at position 24, K at position 28 and K at position 40, are chemically modified through conjugation to the ε-aminos of the K side-chains with ([2-(2-amino-ethoxy)-ethoxy]-acetyl)ₐ-(γ-Glu)_{b}-CO-(CH₂)_{c}-Z, wherein Z is independently selected from the group consisting of -CH₃, carboxylic acids or carboxylic acid bioisosteres, phosphonates or sulfonates, preferably -CO₂H, each a is independently an integer from 0 to 5, each b is independently an integer from 0 to 5, and each c is independently an integer from 10 to 22; and the C-terminal amino acid is amidated as a C-terminal primary amide.

5. The use according to claim 4, wherein
K at position 16 is chemically modified through conjugation to the ε-amino of the K side-chain with ([2-(2-amino-ethoxy)-ethoxy]-acetyl)ₐ-(γ-Glu)_{b}-CO-(CH₂)_{c}-CO₂H; or
K at position 24 is chemically modified through conjugation to the ε-amino of the K side-chain with ([2-(2-amino-ethoxy)-ethoxy]-acetyl)ₐ-(γ-Glu)_{b}-CO-(CH₂)_{c}-CO₂H; or
K at position 28 is chemically modified through conjugation to the ε-amino of the K side-chain with ([2-(2-amino-ethoxy)-ethoxy]-acetyl)ₐ-(γ-Glu)_{b}-CO-(CH₂)_{c}-CO₂H; or
K at position 40 is chemically modified through conjugation to the ε-amino of the K side-chain with ([2-(2-amino-ethoxy)-ethoxy]-acetyl)ₐ-(γ-Glu)_{b}-CO-(CH₂)_{c}-CO₂H.

6. The use according to claim 4, wherein
K at position 24 and K at position 28 are chemically modified with two modifying chains through conjugation to the ε-aminos of the K side-chains with([2-(2-amino-ethoxy)-ethoxy]-acetyl)ₐ-(γ-Glu)_{b}-CO-(CH₂)_{c}-CO₂H; or
K at position 16 and K at position 24 are chemically modified with two modifying chains through conjugation to the ε-aminos of the K side-chains with ([2-(2-amino-ethoxy)-ethoxy]-acetyl)ₐ-(γ-Glu)_{b}-CO-(CH₂)_{c}-CO₂H; or
K at position 16 and K at position 28 are chemically modified with two modifying chains through conjugation to the ε-aminos of the K side-chains with ([2-(2-amino-ethoxy)-ethoxy]-acetyl)ₐ-(γ-Glu)_{b}-CO-(CH₂)_{c}-CO₂H; or
K at position 16 and K at position 40 are chemically modified with two modifying chains through conjugation to the ε-aminos of the K side-chains with ([2-(2-amino-ethoxy)-ethoxy]-acetyl)ₐ-(γ-Glu)_{b}-CO-(CH₂)_{c}-CO₂H; or
K at position 24 and K at position 40 are chemically modified with two modifying chains through conjugation to the ε-aminos of the K side-chains with ([2-(2-amino-ethoxy)-ethoxy]-acetyl)ₐ-(γ-Glu)_{b}-CO-(CH₂)_{c}-CO₂H; or
K at position 28 and K at position 40 are chemically modified with two modifying chains through conjugation to the ε-aminos of the K side-chains with ([2-(2-amino-ethoxy)-ethoxy]-acetyl)ₐ-(γ-Glu)_{b}-CO-(CH₂)_{c}-CO₂H.

7. The use according to any one of claims 3-6, wherein
a is independently selected from an integer of 1, 2, 3, 4 or 5, b is independently selected from an integer of 1, 2, 3, 4 or 5, and c is independently selected from an integer of 12 to 22; preferably, c is independently selected from 14, 16, 18 or 20; and/or
Z is -CO₂H.

8. The use according to claim 4, wherein the compound is
Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K Formula (I);
wherein X₁ is Aib; X₂ is αMePhe; X₃ is Aib; X₄ is Aib; K at position 24 is chemically modified through conjugation to the ε-amino of the K side-chain with ([2-(2-amino-ethoxy)-ethoxy]-acetyl)ₐ-(γ-Glu)_{b}-CO-(CH₂)_{c}-CO₂H, wherein a is independently selected from an integer from 0 to 5, b is independently selected from an integer from 0 to 5, and c is independently selected from an integer from 10 to 24; and the C-terminal amino acid is amidated as a C-terminal primary amide.

9. The use according to claim 4, the compound is
Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K Formula (I);
wherein X₁ is Aib; X₂ is αMePhe; X₃ is Aib; X₄ is Aib; K at position 28 is chemically modified through conjugation to the ε-amino of the K side-chain with ([2-(2-amino-ethoxy)-ethoxy]-acetyl)ₐ-(γ-Glu)_{b}-CO-(CH₂)_{c}-CO₂H, wherein a is independently selected from an integer from 0 to 5, b is independently selected from an integer from 0 to 5, and c is independently selected from an integer from 10 to 24; and the C-terminal amino acid is amidated as a C-terminal primary amide.

10. The use according to any one of claims 8-9, wherein
a is independently selected from an integer of 1, 2, 3, 4 or 5, b is independently selected from an integer of 1, 2, 3, 4 or 5, and c is independently selected from an integer of 12 to 22; preferably, c is independently selected from 14, 16, 18 or 20.

11. The use according to claim 4, the compound is
Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K;
wherein X₁ is Aib; X₂ is αMePhe; X₃ is Aib; X₄ is Aib; K at position 24 is chemically modified through conjugation to the ε-amino of the K side-chain with ([2-(2-amino-ethoxy)-ethoxy]-acetyl)₂-(γ-Glu)₁-CO-(CH₂)₁₆-CO₂H; and the C-terminal amino acid is amidated as a C-terminal primary amide (SEQ ID NO:7); or
Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K;
wherein X₁ is Aib; X₂ is αMePhe; X₃ is Aib; X₄ is Aib; K at position 24 is chemically modified through conjugation to the ε-amino of the K side-chain with ([2-(2-amino-ethoxy)-ethoxy]-acetyl)₂-(γ-Glu)₁-CO-(CH₂)₁₈-CO₂H; and the C-terminal amino acid is amidated as a C-terminal primary amide (SEQ ID NO:13); or
Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K;
wherein X₁ is Aib; X₂ is αMePhe; X₃ is Aib; X₄ is Aib; K at position 24 is chemically modified through conjugation to the ε-amino of the K side-chain with ([2-(2-amino-ethoxy)-ethoxy]-acetyl)₂-(γ-Glu)₁-CO-(CH₂)₂₀-CO₂H; and the C-terminal amino acid is amidated as a C-terminal primary amide (SEQ ID NO:14); or
Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K;
wherein X₁ is Aib; X₂ is αMePhe; X₃ is Aib; X₄ is Aib; K at position 24 is chemically modified through conjugation to the ε-amino of the K side-chain with ([2-(2-amino-ethoxy)-ethoxy]-acetyl)₂-(γ-Glu)₃-CO-(CH₂)₁₆-CO₂H; and the C-terminal amino acid is amidated as a C-terminal primary amide (SEQ ID NO:15); or
Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K;
wherein X₁ is Aib; X₂ is αMePhe; X₃ is Aib; X₄ is Aib; K at position 24 is chemically modified through conjugation to the ε-amino of the K side-chain with ([2-(2-amino-ethoxy)-ethoxy]-acetyl)₃-(γ-Glu)₁-CO-(CH₂)₂₀-CO₂H; and the C-terminal amino acid is amidated as a C-terminal primary amide (SEQ ID NO:16); or
Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K;
wherein X₁ is Aib; X₂ is αMePhe; X₃ is Aib; X₄ is Aib; K at position 28 is chemically modified through conjugation to the ε-amino of the K side-chain with ([2-(2-amino-ethoxy)-ethoxy]-acetyl)₂-(γ-Glu)₁-CO-(CH₂)₁₆-CO₂H; and the C-terminal amino acid is amidated as a C-terminal primary amide (SEQ ID NO:8); or
Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K;
wherein X₁ is Aib; X₂ is αMePhe; X₃ is Aib; X₄ is Aib; K at position 28 is chemically modified through conjugation to the ε-amino of the K side-chain with ([2-(2-amino-ethoxy)-ethoxy]-acetyl)₂-(γ-Glu)₁-CO-(CH₂)₂₀-CO₂H; and the C-terminal amino acid is amidated as a C-terminal primary amide (SEQ ID NO:17); or
Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K;
wherein X₁ is Aib; X₂ is αMePhe; X₃ is Aib; X₄ is Aib; K at position 28 is chemically modified through conjugation to the ε-amino of the K side-chain with ([2-(2-amino-ethoxy)-ethoxy]-acetyl)₂-(γ-Glu)₃-CO-(CH₂)₁₆-CO₂H; and the C-terminal amino acid is amidated as a C-terminal primary amide (SEQ ID NO:18); or
Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K;
wherein X₁ is Aib; X₂ is αMePhe; X₃ is Aib; X₄ is Aib; K at position 28 is chemically modified through conjugation to the ε-amino of the K side-chain with ([2-(2-amino-ethoxy)-ethoxy]-acetyl)₃-(γ-Glu)₁-CO-(CH₂)₁₆-CO₂H; and the C-terminal amino acid is amidated as a C-terminal primary amide (SEQ ID NO:19); or
Y-X₁-E-G-T-X₂-T-S-D-Y-X₃-I-X₄-L-D-K-Q-A-Q-A-E-F-V-K-W-L-L-K-G-G-P-S-S-G-A-P-P-P- S-K;
wherein X₁ is Aib; X₂ is αMePhe; X₃ is Aib; X₄ is Aib; K at position 28 is chemically modified through conjugation to the ε-amino of the K side-chain with ([2-(2-amino-ethoxy)-ethoxy]-acetyl)₃-(γ-Glu)₁-CO-(CH₂)₂₀-CO₂H; and the C-terminal amino acid is amidated as a C-terminal primary amide (SEQ ID NO:20).

12. The use according to claim 4, the compound is 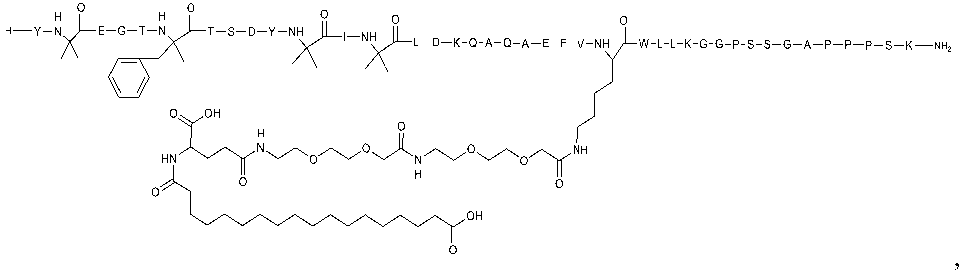 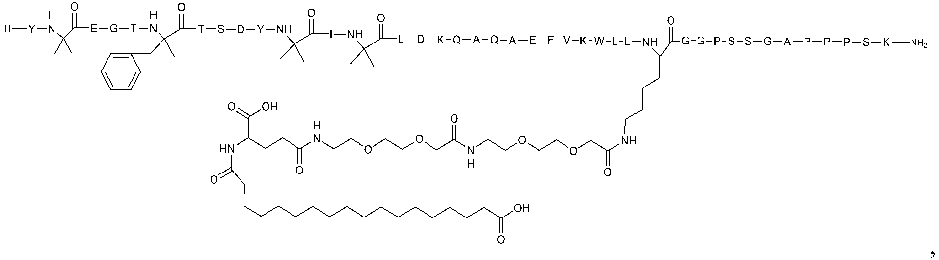 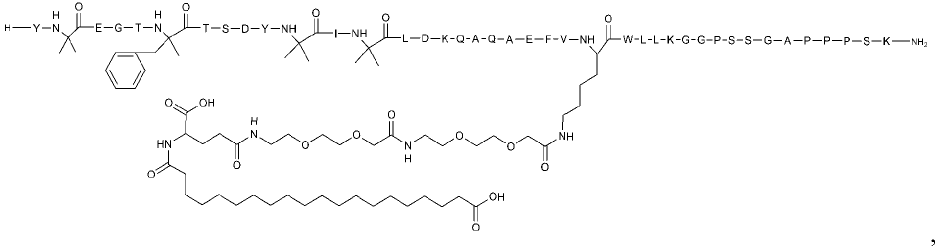 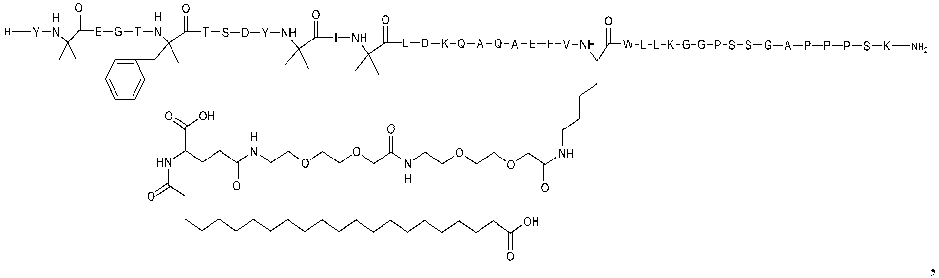 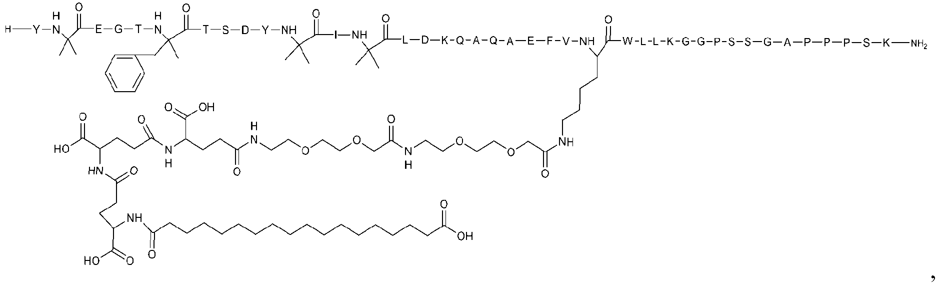 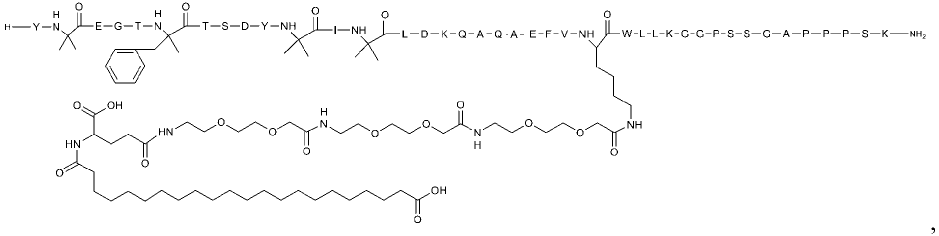 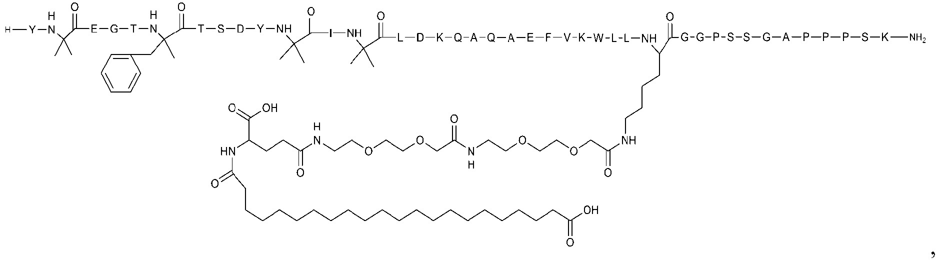 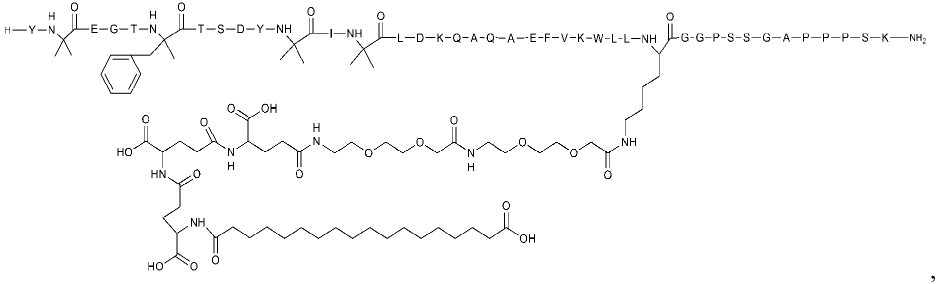 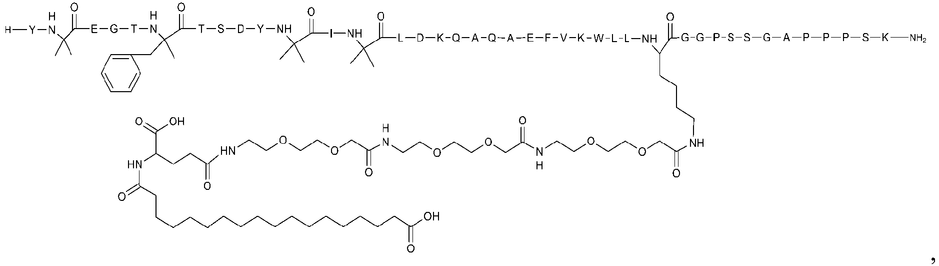 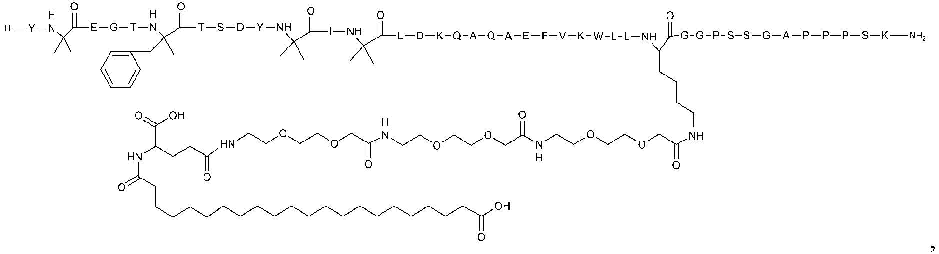 or a pharmaceutically acceptable salt thereof.

13. The use according to any one of claims 1-12, wherein the disease includes hyperglycemia, impaired glucose tolerance, diabetes mellitus (including Type I diabetes mellitus, Type II diabetes mellitus), obesity, hypertension, dyslipidemia, cognitive impairment, atherosclerosis, myocardial infarction, coronary heart disease and other cardiovascular diseases, stroke, inflammatory bowel syndrome, dyspepsia and gastric ulcers.

14. The use according to any one of claims 1-12, wherein the medicament is a medicament for preventing, delaying or treating Type II diabetes mellitus.

15. The use according to any one of claims 1-12, wherein the compound or a pharmaceutically acceptable salt thereof is used to manufacture a medicament for reducing animal food intake, reducing β-cell apoptosis, increasing β-cell function and β-cell volume and/or restoring glucose sensitivity of β-cells.

16. The use according to any one of claims 1-12, wherein the disease includes metabolic disorders, dyslipidaemia, obesity and/or hepatic steatosis associated with insulin resistance and diabetes mellitus.

17. The use according to any one of claims 1-12, wherein the disease includes osteopenia and bone/joint diseases, such as knee osteoarthritis, hip osteoarthritis, and/or spondylitis deformans.

18. The use according to any one of claims 1-12, wherein the compound or a pharmaceutically acceptable salt thereof is used for chronic weight management as an adjunct to a reduced-calorie diet and increased physical activity in an overweight or obese animal.

19. The use according to any one of claims 1-12, wherein the disease includes: symptomatic obesity, obesity based on simple obesity, obesity-related conditions or diseases, eating disorders, diabetes mellitus (for example, Type I diabetes mellitus, Type II diabetes mellitus, gestational diabetes mellitus, obesity diabetes mellitus), hyperlipidemia (e.g., hypertriglyceridemia, hypercholesterolemia, hyper-LDL-cholesterolemia, low HDL-cholesterolemia, postprandial hyperlipidemia), hypertension, heart failure, complications of diabetes mellitus (for example, neuropathy, nephropathy, retinopathy, diabetic cardiomyopathy, cataract, macroangiopathy, osteopenia, hyperosmolar diabetic coma), infectious disease (for example, respiratory tract infection, urinary tract infection, gastrointestinal infection, superficial soft tissue infection, lower extremity infection), diabetic gangrene, xerostomia, hypacusia, cerebrovascular disorder, peripheral blood circulation disorder, metabolic syndrome (with three or more conditions selected from the group consisting of hypertriglyceridemia (TG), low HDL-cholesterolemia , hypertension, abdominal obesity, and impaired glucose tolerance), and/or sarcopenia.

20. The use according to any one of claims 1-19, wherein the animal is a pet.

21. The use according to claim 20, wherein the pet is selected from dogs, cats, rabbits, pigs, alpacas, horses, sheep and bovines.

22. The use according to any one of claims 1-21, wherein the animal is a dog or a cat.
